# EUROPEAN PATENT SPECIFICATION

(11) **EP 4 039 683 B1**
(45) Date of publication and mention of the grant of the patent: **04.11.2026**
(21) Application number: 20890898.8
(22) Date of filing: 18.11.2020
(51) Int. Cl.: C07D 405/12, C07D 405/14, C07D 409/12, C07D 409/14, C07D 413/14

(54) **COMPOUND FOR ORGANIC ELECTRICAL ELEMENT, ORGANIC ELECTRICAL ELEMENT USING SAME AND ELECTRONIC DEVICE THEREOF**
VERBINDUNG FÜR ORGANISCHES ELEKTRISCHES ELEMENT, ORGANISCHES ELEKTRISCHES ELEMENT MIT VERWENDUNG DAVON UND ELEKTRONISCHE VORRICHTUNG DAVON
COMPOSÉ POUR ÉLÉMENT ÉLECTRIQUE ORGANIQUE, ÉLÉMENT ÉLECTRIQUE ORGANIQUE CONTENANT LEDIT COMPOSÉ ET DISPOSITIF ÉLECTRONIQUE ASSOCIÉ

(30) Priority: 19.11.2019 KR 20190148780; 14.08.2020 KR 20200102536
(43) Date of publication of application: 10.08.2022
(60) Divisional application: 23160806.8 / 4 269 406; 23160809.2 / 4 215 528
(73) Proprietor: Duk San Neolux Co., Ltd., Chungcheongnam-do 31027 (KR)
(72) Inventor: KIM, Won Sam, Cheonan-si, Chungcheongnam-do 31027 (KR); OH, Hyun Ji, Cheonan-si, Chungcheongnam-do 31027 (KR); OH, Dae Hwan, Cheonan-si, Chungcheongnam-do 31027 (KR); LEE, Sun Hee, Cheonan-si, Chungcheongnam-do 31027 (KR); MUN, Soung Yun, Cheonan-si, Chungcheongnam-do 31027 (KR); KIM, Jeong Seok, Cheonan-si, Chungcheongnam-do 31027 (KR)
(74) Representative: Gassner, Birgitta
(86) International application number: PCT/KR2020/016272
(87) International publication number: WO 2021/101247

(56) References cited:
- EP-A1- 3 348 552
- EP-A1- 3 764 416
- EP-A1- 3 919 482
- WO-A1-2020/159019
- CN-A- 105 778 891
- CN-A- 105 906 640
- CN-A- 109 867 652
- KR-A- 20150 083 917
- KR-A- 20170 138 799
- KR-A- 20180 112 962
- KR-A- 20190 015 211
- KR-A- 20190 058 640
- KR-A- 20190 061 314

## Description

### BACKGROUND

### [Technical Field]

The present invention relates to a compound for organic electronic element, organic electronic element using the same, and an electronic device thereof.

### [Background Art]

In general, organic light emitting phenomenon refers to a phenomenon that converts electric energy into light energy by using an organic material. An organic electronic element using an organic light emitting phenomenon usually has a structure including an anode, a cathode, and an organic material layer interposed therebetween. Here, in order to increase the efficiency and stability of the organic electronic element, the organic material layer is often composed of a multi-layered structure composed of different materials, and for example, may include a hole injection layer, a hole transport layer, an emitting layer, an electron transport layer, an electron injection layer and the like.

A material used as an organic material layer in an organic electronic element may be classified into a light emitting material and a charge transport material, such as a hole injection material, a hole transport material, an electron transport material, an electron injection material and the like depending on its function.

In the organic light emitting diode, the most problematic is the lifetime and the efficiency. As the display becomes larger, the efficiency and the lifetime problem must be solved. Efficiency, life span, driving voltage and the like are related to each other. As the efficiency is increased, the driving voltage is relatively decreased, and as the driving voltage drops, the crystallization of the organic material due to joule heating generated during driving is reduced, and as a result, the life span tends to increase.

However, simply improving the organic material layer cannot maximize the efficiency. This is because, when the optimal combination of the energy level and T1 value between each organic material layer and the intrinsic properties (mobility, interface characteristics, etc.) of the material are achieved, long life and high efficiency can be achieved at the same time.

Further, recently, in organic electroluminescent devices, in order to solve the emission problem in the hole transport layer, an emitting-auxiliary layer must be present between the hole transport layer and an emitting layer, and it is necessary to develop different emitting-auxiliary layers according to the respective emitting layers (R, G, B).

In general, electrons are transferred from the electron transport layer to the emitting layer, and holes are transferred from the hole transport layer to the emitting layer to generate excitons by recombination.

However, the material used for the hole transport layer has a low HOMO value and therefore has mostly low T1 value. As a result, the exciton generated in the emitting layer is transferred to the hole transport layer, resulting in a charge imbalance in the emitting layer, and light is emitted at the interface of the hole transport layer.

When light is emitted at the interface of the hole transport layer, the color purity and efficiency of the organic electronic device are lowered and the life span is shortened. Therefore, it is urgently required to develop an emitting-auxiliary layer having a high T1 value and a HOMO level between the HOMO energy level of the hole transport layer and the HOMO energy level of the emitting layer.

Meanwhile, it is necessary to develop a hole injection layer material having stable characteristics, that is, a high glass transition temperature against joule heating generated when the device is driven while delaying penetration of the metal oxide from the anode electrode (ITO), which is one of the causes of shortening the lifetime of the organic electronic device, into the organic layer. The low glass transition temperature of the hole transport layer material has a characteristic that when the device is driven, the uniformity of the surface of the thin film is lowered, which has been reported to have a great influence on the lifetime of the device. In addition, OLED devices are mainly formed by a deposition method, and it is necessary to develop a material that can withstand long time in deposition, that is, a material having high heat resistance characteristics.

That is, in order to sufficiently exhibit the excellent characteristics of the organic electronic element, a material for forming an organic material layer in an element such as a hole injection material, a hole transport material, a light emitting material, an electron transport material, an electron injection material, an emitting-auxiliary layer material should be supported by stable and efficient materials. However, such stable and efficient organic material layer material for an organic electronic element have not been sufficiently developed yet. KR20190058640 relates to specific xanthene compounds as such and to the use thereof in an organic electroluminescent device. Therefore, development of new materials is continuously required, and development of materials for the hole transport layer or the emitting-auxiliary layer is urgently required.

As a reference prior art document, KR1020130076842 A was used.

### DETAILED DESCRIPTION OF THE INVENTION

### [Summary]

In order to solve the problems of the background art described above, the present invention has revealed a compound having a novel structure, and that when the compound is applied to an organic electronic element, the luminous efficiency, stability and lifetime of the element are greatly improved. Accordingly, it is an object of the present invention to provide a novel compound, an organic electronic element using the same, and an electronic device.

The invention is set out in the appended set of claims.

### [Technical Solution]

Described herein is a compound represented by the following Formula (1).

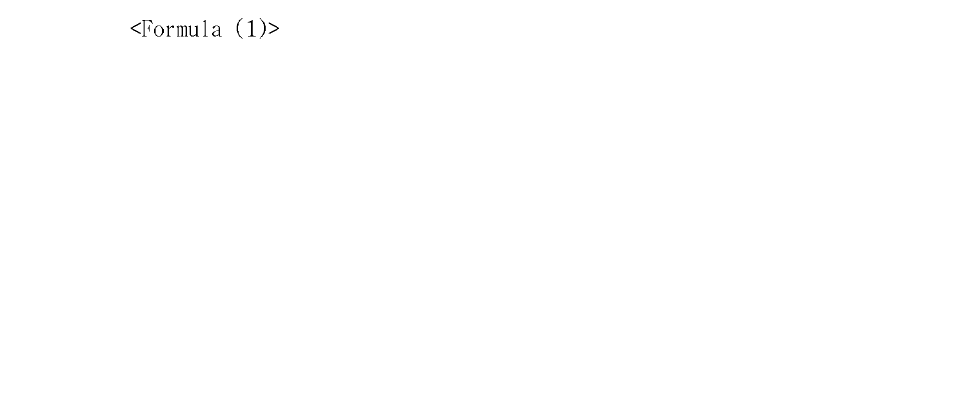

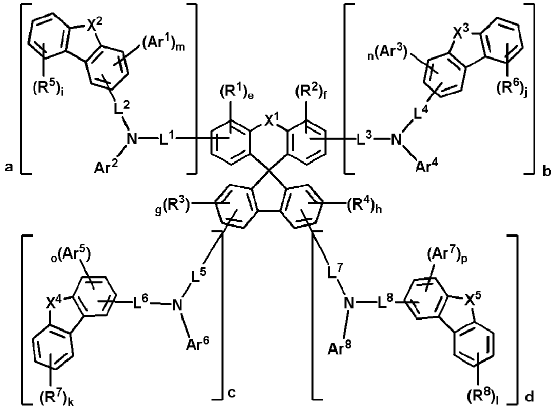

The present invention provides a compound represented by the following Formula (4-2).

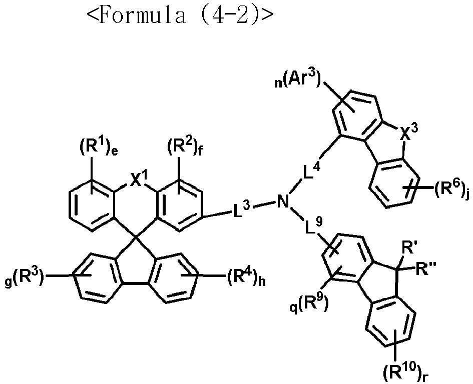

In another aspect, the present invention provides an organic electric device including the compound represented by Formula (4-2) and an electronic device thereof.

### [Effects of the Invention]

By using the compound according to the present invention, it is possible to achieve a high luminous efficiency, a low driving voltage, and a high heat resistance of the element, and can greatly improve the color purity and lifetime of the element.

### [BRIEF DESCRIPTION OF THE DRAWINGS]

FIG. 1 to FIG. 3 illustrate an example of an organic electronic element according to the present invention.
FIG. 4 shows the formula according to one aspect of the present invention.

| | | | |
|---|---|---|---|
| 100, 200, 300: | organic electronic element | 110 : | the first electrode |
| 120 : | hole injection layer | 130 : | hole transport layer |
| 140 : | emitting layer | 150 : | electron transport layer |
| 160 : | electron injection layer | 170 : | second electrode |
| 180 : | light efficiency enhancing Layer | 210 : | buffer layer |
| 220 : | emitting-auxiliary layer | 320 : | first hole injection layer |
| 330 : | first hole transport layer | 340 : | first emitting layer |
| 350 : | first electron transport layer | 360 : | first charge generation layer |
| 361 : | second charge generation layer | 420 : | second hole injection layer |
| 430 : | second hole transport layer | 440 : | second emitting layer |
| 450 : | second electron transport layer | CGL : | charge generation layer |
| ST1 : | first stack | ST2 : | second stack |

### [DETAILED DESCRIPTION]

Hereinafter, some embodiments of the present invention will be described in detail. Further, in the following description of the present invention, a detailed description of known functions and configurations incorporated herein will be omitted when it may make the subject matter of the present invention rather unclear.

In addition, terms, such as first, second, A, B, (a), (b) or the like may be used herein when describing components of the present invention. Each of these terminologies is not used to define an essence, order or sequence of a corresponding component but used merely to distinguish the corresponding component from other component(s). It should be noted that if a component is described as being "connected", "coupled", or "connected" to another component, the component may be directly connected or connected to the other component, but another component may be "connected ", "coupled" or "connected" between each component.

As used in the specification and the accompanying claims, unless otherwise stated, the following is the meaning of the term as follows.

Unless otherwise stated, the term "halo" or "halogen" , as used herein, includes fluorine, bromine, chlorine, or iodine.

Unless otherwise stated, the term "alkyl" or "alkyl group" , as used herein, has a single bond of 1 to 60 carbon atoms, and means saturated aliphatic functional radicals including a linear alkyl group, a branched chain alkyl group, a cycloalkyl group (alicyclic), a cycloalkyl group substituted with a alkyl or an alkyl group substituted with a cycloalkyl.

Unless otherwise stated, the term "alkenyl" or "alkynyl" , as used herein, has double or triple bonds of 2 to 60 carbon atoms, but is not limited thereto, and includes a linear or a branched chain group.

Unless otherwise stated, the term "cycloalkyl" , as used herein, means alkyl forming a ring having 3 to 60 carbon atoms, but is not limited thereto.

Unless otherwise stated, the term "alkoxyl group" , "alkoxy group" or "alkyloxy group" , as used herein, means an oxygen radical attached to an alkyl group, but is not limited thereto, and has 1 to 60 carbon atoms.

Unless otherwise stated, the term "aryloxyl group" or "aryloxy group" , as used herein, means an oxygen radical attached to an aryl group, but is not limited thereto, and has 6 to 60 carbon atoms.

Unless otherwise stated, the term "aryl group" or "arylene group" , as used herein, has 6 to 60 carbon atoms, but is not limited thereto. Herein, the aryl group or arylene group means a monocyclic and polycyclic aromatic group, and may also be formed in conjunction with an adjacent group. Examples of "aryl group" may include a phenyl group, a biphenyl group, a fluorene group, or a spirofluorene group.

The prefix "aryl" or "ar" means a radical substituted with an aryl group. For example, an arylalkyl may be an alkyl substituted with an aryl, and an arylalkenyl may be an alkenyl substituted with aryl, and a radical substituted with an aryl has the number of carbon atoms as defined herein.

Also, when prefixes are named subsequently, it means that substituents are listed in the order described first. For example, an arylalkoxy means an alkoxy substituted with an aryl, an alkoxylcarbonyl means a carbonyl substituted with an alkoxyl, and an arylcarbonylalkenyl also means an alkenyl substituted with an arylcarbonyl, wherein the arylcarbonyl may be a carbonyl substituted with an aryl.

Unless otherwise stated, the term "heterocyclic group" , as used herein, contains one or more heteroatoms, but is not limited thereto, has 2 to 60 carbon atoms, includes any one of monocyclic and polycyclic rings, and may include heteroaliphatic ring and/or heteroaromatic ring. Also, the heterocyclic group may also be formed in conjunction with an adjacent group.

Unless otherwise stated, the term "heteroatom" , as used herein, represents at least one of N, O, S, P, or Si.

Also, the term "heterocyclic group" may include a ring including SO₂ instead of carbon consisting of cycle. For example, "heterocyclic group" includes a following compound.

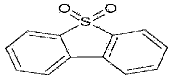

Unless otherwise stated, the term "fluorenyl group" or "fluorenylene group" , as used herein, means a monovalent or divalent functional group, in which R, R' and R" are all hydrogen in the following structures, and the term "substituted fluorenyl group" or "substituted fluorenylene group" means that at least one of the substituents R, R' , R" is a substituent other than hydrogen, and include those in which R and R' are bonded to each other to form a spiro compound together with the carbon to which they are bonded.

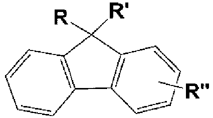

The term "spiro compound" , as used herein, has a 'spiro union' , and a spiro union means a connection in which two rings share only one atom. At this time, atoms shared in the two rings are called 'spiro atoms' , and these compounds are called 'monospiro-' , 'di-spiro-' and 'tri-spiro-', respectively, depending on the number of atoms in a compound.

Unless otherwise stated, the term "aliphatic" , as used herein, means an aliphatic hydrocarbon having 1 to 60 carbon atoms, and the term "aliphatic ring" , as used herein, means an aliphatic hydrocarbon ring having 3 to 60 carbon atoms.

Unless otherwise stated, the term "ring" , as used herein, means an aliphatic ring having 3 to 60 carbon atoms, or an aromatic ring having 6 to 60 carbon atoms, or a hetero ring having 2 to 60 carbon atoms, or a fused ring formed by the combination of them, and includes a saturated or unsaturated ring.

Other hetero compounds or hetero radicals other than the above-mentioned hetero compounds include, but are not limited thereto, one or more heteroatoms.

Unless otherwise stated, the term "substituted or unsubstituted" , as used herein, means that substitution is substituted by at least one substituent selected from the group consisting of, but is not limited thereto, deuterium, halogen, an amino group, a nitrile group, a nitro group, a C₁-C₂₀ alkyl group, a C₁-C₂₀ alkoxyl group, a C₁-C₂₀ alkylamine group, a C₁-C₂₀ alkylthiophene group, a C₆-C₂₀ arylthiophene group, a C₂-C₂₀ alkenyl group, a C₂-C₂₀ alkynyl group, a C₃-C₂₀ cycloalkyl group, a C₆-C₂₀ aryl group, a C₆-C₂₀ aryl group substituted by deuterium, a C₈-C₂₀ arylalkenyl group, a silane group, a boron group, a germanium group, and a C₂-C₂₀ heterocyclic group.

Unless otherwise expressly stated, the Formula used in the present invention, as used herein, is applied in the same manner as the substituent definition according to the definition of the exponent of the following Formula. 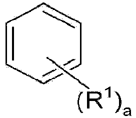 wherein, when a is an integer of zero, the substituent R¹ is absent; when a is an integer of 1, the sole substituent R¹ is linked to any one of the carbon constituting the benzene ring; when a is an integer of 2 or 3, each substituent R¹s may be the same and different; when a is an integer of 4 to 6, and is linked to the benzene ring in a similar manner, whereas the indication of hydrogen bound to the carbon forming the benzene ring is omitted.

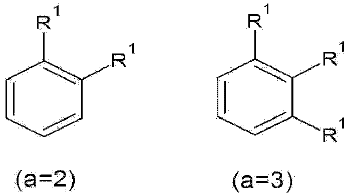

Hereinafter, a compound according to an aspect of the present invention and an organic electronic element comprising the same will be described.

Described herein is a compound represented by Formula (1).

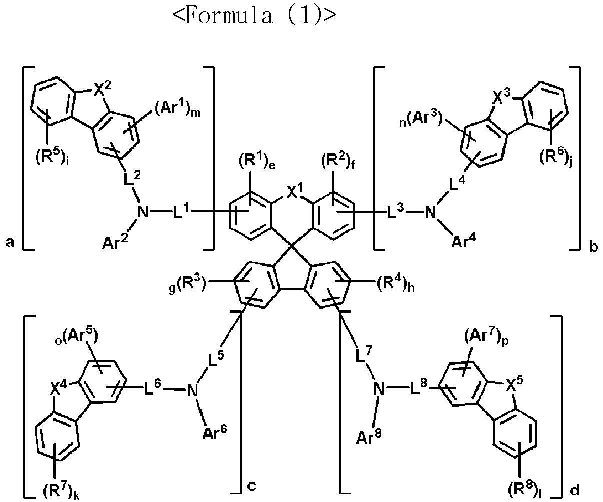

In Formula (1), each symbol may be defined as follows:
1) X¹, X², X³, X⁴ and X⁵ are each independently O or S,
2) a, b, c and d are each independently an integer of 0 to 4, a+b+c+d is 1 or more,
3) e, f, g, h, i, j, k and l are each independently an integer of 0 to 4, wherein in case e, f, g, h, i, j, k and l are 2 or more, R¹, R², R³, R⁴, R⁵, R⁶, R⁷ and R⁸ are each in plural being the same or different, and an adjacent plurality of R' or a plurality of R² or a plurality of R³ or a plurality of R⁴ or a plurality of R⁵ or a plurality of R⁶ or a plurality of R⁷ or a plurality of R⁸ may be bonded to each other to form a ring,
4) m, n, o and p are each independently an integer of 0 to 3, m+n+o+p is 1 or more,
5) L¹, L², L³, L⁴, L⁵, L⁶, L⁷ and L⁸ are the same or different from each other, and are each independently selected from the group consisting of single bond;
   a C₆-C₆₀ arylene group; a fluorenylene group; a fused ring group of a C₃-C₆₀ aliphatic ring and a C₆-C₆₀ aromatic ring; a C₂-C₆₀ heterocyclic group;
   wherein in case L¹, L², L³, L⁴, L⁵, L⁶, L⁷ and L⁸ are an arylene group, it is preferably an C₆-C₃₆ arylene group, more preferably an C₆-C₂₄ arylene group, when L¹, L², L³, L⁴, L⁵, L⁶, L⁷ and L⁸ are a fused ring group, it is preferably a fused ring group of an C₆-C₃₆ aliphatic ring and an C₆-C₃₆ aromatic ring, and more preferably a fused ring group of an C₆-C₂₄ aliphatic ring and an C₆-C₂₄ aromatic ring, when L¹, L², L³, L⁴, L⁵, L⁶, L⁷ and L⁸ are a heterocyclic group, it is preferably a C₂-C₃₆ heterocyclic group, and more preferably a C₂-C₂₄ heterocyclic group,
6) Ar¹, Ar³, Ar⁵ and Ar⁷ are the same or different from each other, and are each independently selected from the group consisting of hydrogen; deuterium; tritium; halogen; cyano group; nitro group; C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; C₁-C₅₀ alkyl group; C₂-C₂₀ alkenyl group; C₂-C₂₀ alkynyl group; C₁-C₃₀ alkoxyl group; C₆-C₃₀ aryloxy group; and -L'-N(Rₐ)(R_{b});
   wherein in case Ar¹, Ar³, Ar⁵ and Ar⁷ are an aryl group, it is preferably an C₆-C₃₆ aryl group, more preferably an C₆-C₂₅ aryl group, in case Ar¹, Ar³, Ar⁵ and Ar⁷ are an heterocyclic group, it is preferably a C₂-C₃₆ heterocyclic group, and more preferably a C₂-C₂₄ heterocyclic group, in case Ar¹, Ar³, Ar⁵ and Ar⁷ are a fused ring group, it is preferably a fused ring group of an C₆-C₃₆ aliphatic ring and an C₆-C₃₆ aromatic ring, more preferably a fused ring group of an C₆-C₂₄ aliphatic ring and an C₆-C₂₄ aromatic ring, in case Ar¹, Ar³, Ar⁵ and Ar⁷ are alkyl groups, it is preferably an C₁-C₃₆ alkyl group, more preferably an C₁-C₂₄ alkyl group,
7) Ar², Ar⁴, Ar⁶ and Ar⁸ are each independently selected from the group consisting of a C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; -L⁹-Ar' ;
   wherein in case Ar², Ar⁴, Ar⁶ and Ar⁸ are an aryl group, it is preferably an C₆-C₃₆ aryl group, more preferably an C₆-C₂₅ aryl group, in case Ar², Ar⁴, Ar⁶ and Ar⁸ are heterocyclic groups, it is preferably a C₂-C₃₆ heterocyclic group, and more preferably a C₂-C₂₄ heterocyclic group, in case Ar², Ar⁴, Ar⁶ and Ar⁸ are a fused ring group, it is preferably a fused ring group of an C₆-C₃₆ aliphatic ring and an C₆-C₃₆ aromatic ring, more preferably a fused ring group of an C₆-C₂₄ aliphatic ring and an C₆-C₂₄ aromatic ring,
8) wherein L⁹ is selected from the group consisting of single bond; a C₆-C₆₀ arylene group; a fluorenylene group; a fused ring group of a C₃-C₆₀ aliphatic ring and a C₆-C₆₀ aromatic ring; a C₂-C₆₀ heterocyclic group; Ar' is selected from a C₆-C₆₀ aryl group; a fluorenylene group; a C₂-C₆₀ heterocyclic group including at least one heteroatom of O, N, S, Si or P; a fused ring group of a C₃-C₆₀ aliphatic ring and a C₆-C₆₀ aromatic ring;
   wherein in case L⁹ is arylene group, it is preferably an C₆-C₃₆ arylene group, more preferably an C₆-C₂₄ arylene group, when L⁹ is a fused ring group, it is preferably a fused ring group of an C₆-C₃₆ aliphatic ring and an C₆-C₃₆ aromatic ring, and more preferably a fused ring group of an C₆-C₂₄ aliphatic ring and an C₆-C₂₄ aromatic ring, when L⁹ is a heterocyclic group, it is preferably a C₂-C₃₆ heterocyclic group, and more preferably a C₂-C₂₄ heterocyclic group;
   wherein in case Ar' is an aryl group, it is preferably an C₆-C₃₆ aryl group, more preferably an C₆-C₂₅ aryl group, in case Ar' is a heterocyclic group, it is preferably C₂-C₃₆ heterocyclic groups, and more preferably C₂-C₂₄ heterocyclic groups, in case Ar' is a fused ring group, it is preferably a fused ring group of an C₆-C₃₆ aliphatic ring and an C₆-C₃₆ aromatic ring, more preferably a fused ring group of an C₆-C₂₄ aliphatic ring and an C₆-C₂₄ aromatic ring,
9) R¹, R², R³, R⁴, R⁵, R⁶, R⁷ and R⁸ are the same or different from each other, and are each independently selected from the group consisting of hydrogen; deuterium; tritium; halogen; cyano group; nitro group; C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; C₁-C₅₀ alkyl group; C₂-C₂₀ alkenyl group; C₂-C₂₀ alkynyl group; C₁-C₃₀ alkoxyl group; C₆-C₃₀ aryloxy group; and -L'-N(Rₐ)(R_{b});
   wherein in case R¹, R², R³, R⁴, R⁵, R⁶, R⁷ and R⁸ are an aryl group, it is preferably an C₆-C₃₆ aryl group, more preferably an C₆-C₂₅ aryl group, in case R¹, R², R³, R⁴, R⁵, R⁶, R⁷ and R⁸ are an heterocyclic group, it is preferably a C₂-C₃₆ heterocyclic group including at least one heteroatom of O, N, S, Si or P; in case R¹, R², R³, R⁴, R⁵, R⁶, R⁷ and R⁸ are a fused ring group, it is preferably a fused ring group of an C₆-C₃₆ aliphatic ring and an C₆-C₃₆ aromatic ring, more preferably a fused ring group of an C₆-C₂₄ aliphatic ring and an C₆-C₂₄ aromatic ring,
10) wherein L' is selected from the group consisting of a single bond; a C₆-C₆₀ arylene group; a fluorenylene group; C₃-C₆₀ aliphatic ring; a C₂-C₆₀ heterocyclic group including at least one heteroatom of O, N, S, Si or P; a combination thereof; wherein Rₐ and R_{b} are each independently selected from the group consisting of a C₆-C₆₀ aryl group; a fluorenyl group; C₃-C₆₀ aliphatic ring; a C₂-C₆₀ heterocyclic group including at least one heteroatom of O, N, S, Si or P;
   wherein in case L' is an arylene group, it is preferably an C₆-C₃₆ arylene group, more preferably an C₆-C₂₄ arylene group, in case L' is an aliphatic ring, it is preferably a C₃-C₃₆ aliphatic ring, more preferably C₃-C₂₄ aliphatic ring, when L' is a heterocyclic group, it is preferably a C₂-C₃₆ heterocyclic group including at least one heteroatom of O, N, S, Si or P; and more preferably a C₂-C₂₄ heterocyclic group including at least one heteroatom of O, N, S, Si or P;
   wherein in case Rₐ and R_{b} are an aryl group, it is preferably an C₆-C₃₆ aryl group, more preferably an C₆-C₂₅ aryl group, in case Rₐ and R_{b} are an aliphatic ring, it is preferably a C₃-C₃₆ aliphatic ring, more preferably C₃-C₂₄ aliphatic ring, in case Rₐ and R_{b} are a heterocyclic group, it is preferably a C₂-C₃₆ heterocyclic group including at least one heteroatom of O, N, S, Si or P; and more preferably a C₂-C₂₄ heterocyclic group including at least one heteroatom of O, N, S, Si or P;
11) wherein, the aryl group, arylene group, heterocyclic group, fluorenyl group, fluorenylene group, fused ring group, alkyl group, alkenyl group, alkoxy group and aryloxy group may be substituted with one or more substituents selected from the group consisting of deuterium; halogen; silane group; siloxane group; boron group; germanium group; cyano group; nitro group; C₁-C₂₀ alkylthio group; C₁-C₂₀ alkoxyl group; C₁-C₂₀ alkyl group; C₂-C₂₀ alkenyl group; C₂-C₂₀ alkynyl group; C₆-C₂₅ aryl group; C₆-C₂₅ aryl group substituted with deuterium; a fluorenyl group; C₂-C₂₀ heterocyclic group; C₃-C₂₀ cycloalkyl group; C₇-C₂₀ arylalkyl group; C₈-C₂₀ arylalkenyl group; and - L'-N(Rₐ)(R_{b}); wherein the substituents may be bonded to each other to form a saturated or unsaturated ring, wherein the term 'ring' means a C₃-C₆₀ aliphatic ring, a C₆-C₆₀ aromatic ring, a C₂-C₆₀ heterocyclic group, a fused ring formed by combination thereof.

Also, Formula 1 includes a compound represented by any one of Formulas 1-1 to 1-9.

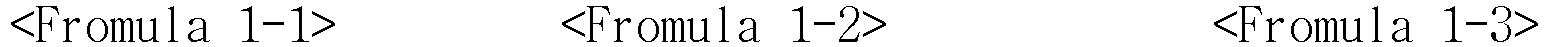

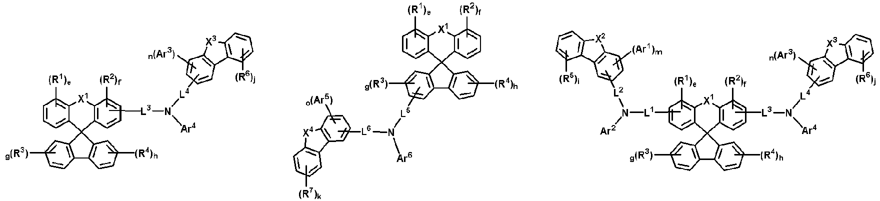

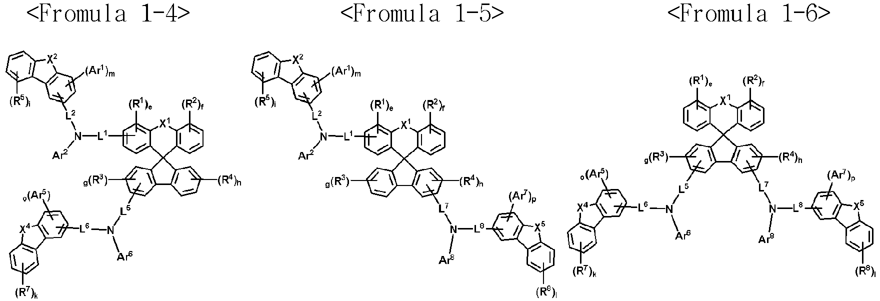

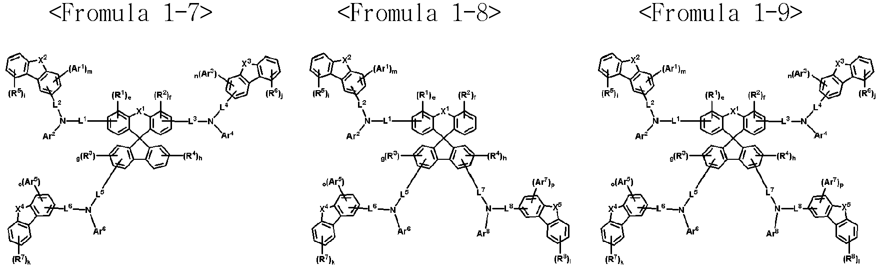

In Formulas 1-1 to 1-9,
X¹, X², X³, X⁴, X⁵ and R¹, R², R³, R⁴, R⁵, R⁶, R⁷, R⁸ and L¹, L², L³, L⁴, L⁵, L⁶, L⁷, L⁸ and, Ar¹, Ar², Ar³, Ar⁴, Ar⁵, Ar⁶, Ar⁷, Ar⁸ and e, f, g, h, i, j, k, l, m, n, o and p are the same as defined in Formula 1.

Also, Formula 1 includes a compound represented by Formula 2.

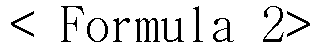

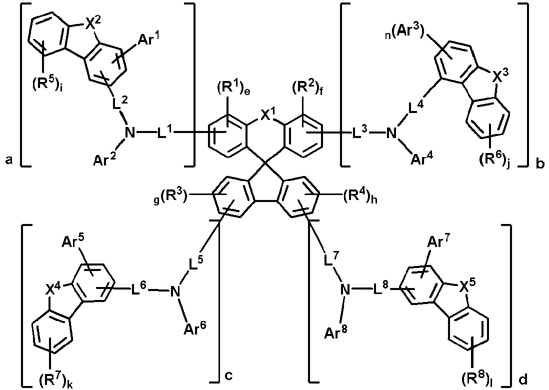

In Formula 2,
X¹, X², X³, X⁴, X⁵ and R¹, R², R³, R⁴, R⁵, R⁶, R⁷, R⁸ and L¹, L², L³, L⁴, L⁵, L⁶, L⁷, L⁸ and Ar¹, Ar², Ar³, Ar⁴, Ar⁵, Ar⁶, Ar⁷, and Ar⁸, and a, b, c, d, e, f, g, h, i, j, k, l and n are the same as defined in Formula 1.

Formula 1 includes a compound represented by any one of Formulas 2-1 to 2-4 .

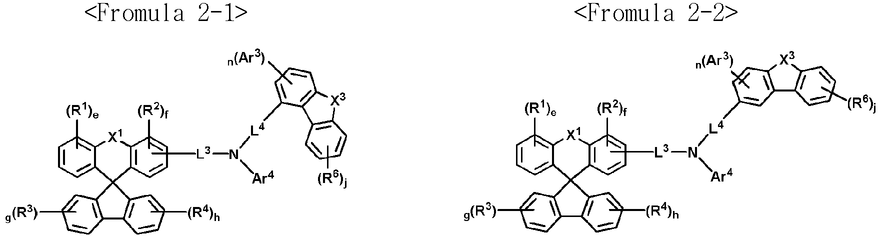

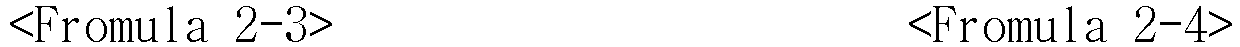

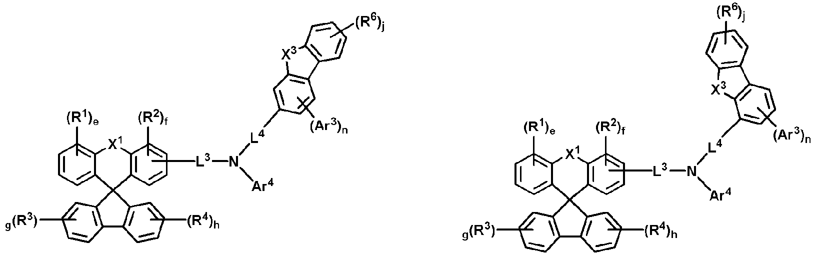

In Formulas 2-1 to 2-4,
X¹, X³, R¹, R², R³, R⁴, R⁶, L³, L⁴, Ar³, Ar⁴, e, f, g, h, j and n are the same as defined in Formula 1.

Formula 1 includes a compound represented by Formula 3.

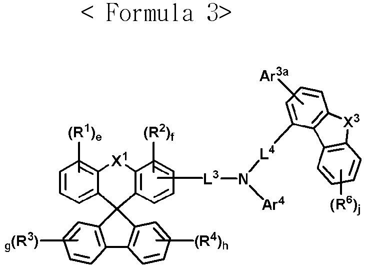

In Formula 3,
1) X¹, X³, R¹, R², R³, R⁴, R⁶, L³, L⁴, Ar⁴, e, f, g, h and j are the same as defined in Formula 1,
2) Ar^{3a} is selected from the group consisting of deuterium; tritium; halogen; cyano group; nitro group; C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; C₁-C₅₀ alkyl group; C₂-C₂₀ alkenyl group; C₂-C₂₀ alkynyl group; C₁-C₃₀ alkoxyl group; C₆-C₃₀ aryloxy group; and -L'-N(Rₐ)(R_{b});

When Ar^{3a} is an aryl group, it is preferably a C₆-C₃₆ aryl group, more preferably a C₆-C₂₅ aryl group, when Ar^{3a} is a heterocyclic group, it is preferably a C₂-C₃₆ heterocyclic group, more preferably a C₂-C₂₄ heterocyclic group, when Ar^{3a} is a fused ring group, it is preferably a fused ring group of a C₆-C₃₆ aliphatic ring and a C₆-C₃₆ aromatic ring, and more preferably a fused ring group of a C₆-C₂₄ aliphatic ring and a C₆-C₂₄ aromatic ring, when Ar^{3a} is an alkyl group, it is preferably a C₁-C₃₆ alkyl group, and more preferably a C₁-C₂₄ alkyl group.

Also, Formula 1 includes a compound represented by Formula 4.

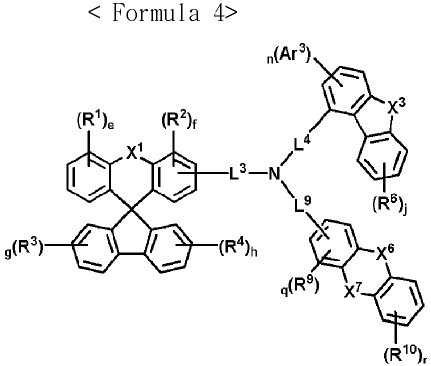

In Formula 4, each symbol may be defined as follows.
1) X¹, X³, R¹, R², R³, R⁴, R⁶, L³, L⁴, L⁹, Ar³, e, f, g, h, j and n are the same as defined in Formula 1,
2) X⁶ and X⁷ are each independently a single bond, CR'R", O or S, except when X⁶ and X⁷ are a single bond at the same time,
3) R' and R" are each independently a hydrogen; C₆-C₆₀ aryl group; a fluorenyl group; a C₃-C₆₀ heterocyclic group; C₁-C₅₀ alkyl group; C₂-C₆₀ alkenyl group; or C₆-C₆₀ aryloxy group; fluorenyl group; a C₂-C₆₀ heterocyclic group including at least one heteroatom of O, N, S, Si or P; C₃-C₆₀ aliphatic ring; or a fused ring group of a C₃-C₆₀ aliphatic ring and a C₆-C₆₀ aromatic ring; wherein in case R' and R" are an aryl group, it is preferably an C₆-C₃₆ aryl group, more preferably an C₆-C₂₅ aryl group, in case R' and R" are a heterocyclic group, it is preferably C₂-C₃₆ heterocyclic groups, and more preferably C₂-C₂₄ heterocyclic groups, in case R' and R" are an alkyl group, it is preferably C₁-C₃₆ alkyl group, and more preferably C₁-C₂₄ alkyl group, in case R' and R" are an alkenyl group, it is preferably a C₂-C₃₆ alkenyl group, and more preferably a C₂-C₂₄ alkenyl group, in case R' and R" are an aryloxy group, it is preferably C₆-C₃₆ aryloxy group, and more C₆-C₂₄ aryloxy group,
4) R⁹ and R¹⁰ are the same as the definition of R¹ in Formula 1,
5) q is an integer of 0 to 3, r is an integer of 0 to 4.

Formula 1 includes a compound represented by Formula 4-1.

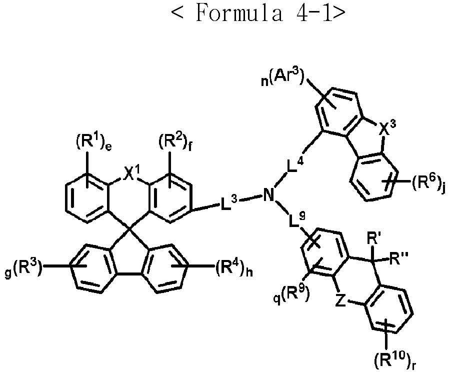

In Formula 4-1,
1) X¹, X³, R¹, R², R³, R⁴, R⁶ , L³, L⁴, L⁹, Ar³, e, f, g, h, j and n are the same as defined in Formula 1,
2) R⁹, R¹⁰, R', R", q and r are the same as defined in Formula 4,
3) Z is a single bond, O or S.

The present invention relates to a compound represented by Formula 4-2.

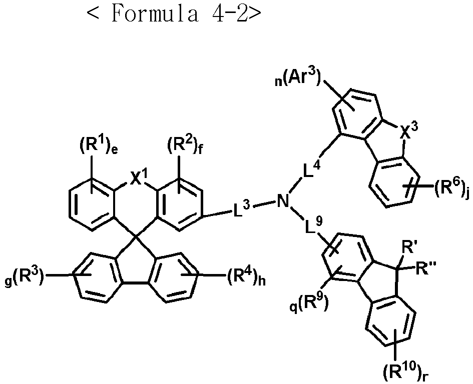

In Formula 4-2,
1) X¹, and X³ are each independently O or S,
2) e, g, h, j, and r, are each independently an integer of 0 to 4, and f and q are an integer of 0 to 3, wherein in case e, f, g, h, j, q and r are 2 or more, R¹, R², R³, R⁴, and R⁶ are each in plural being the same or different, and an adjacent plurality of R¹ or a plurality of R² or a plurality of R³ or a plurality of R⁴ or a plurality of R⁶ or a plurality of R⁷ or a plurality of R⁸ may be bonded to each other to form a ring,
3) n is an integer of 0 to 3,
4) L³, L⁴, and L⁹ are the same or different from each other, and are each independently selected from the group consisting of single bond; a C₆-C₆₀ arylene group; a fluorenylene group; a fused ring group of a C₃-C₆₀ aliphatic ring and a C₆-C₆₀ aromatic ring; a C₂-C₆₀ heterocyclic group;
5) Ar³ is selected from the group consisting of hydrogen; deuterium; tritium; halogen; cyano group; nitro group; C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; C₁-C₅₀ alkyl group; C₂-C₂₀ alkenyl group; C₂-C₂₀ alkynyl group; C₁-C₃₀ alkoxyl group; C₆-C₃₀ aryloxy group; and -L'-N(Rₐ)(R_{b});
6) R¹, R², R³, R⁴, R⁶, R⁹ and R¹⁰ are the same or different from each other, and are each independently selected from the group consisting of hydrogen; deuterium; tritium; halogen; cyano group; nitro group; C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; C₁-C₅₀ alkyl group; C₂-C₂₀ alkenyl group; C₂-C₂₀ alkynyl group; C₁-C₃₀ alkoxyl group; C₆-C₃₀ aryloxy group; and -L'-N(Rₐ)(R_{b});
7) wherein L' is selected from the group consisting of a single bond; a C₆-C₆₀ arylene group; a fluorenylene group; C₃-C₆₀ aliphatic ring; a C₂-C₆₀ heterocyclic group including at least one heteroatom of O, N, S, Si or P; a combination thereof; wherein Rₐ and R_{b} are each independently selected from the group consisting of a C₆-C₆₀ aryl group; a fluorenyl group; C₃-C₆₀ aliphatic ring; a C₂-C₆₀ heterocyclic group including at least one heteroatom of O, N, S, Si or P;
8) R' and R" are each independently a hydrogen; C₆-C₆₀ aryl group; a fluorenyl group; a C₃-C₆₀ heterocyclic group; C₁-C₅₀ alkyl group; C₂-C₆₀ alkenyl group; or C₆-C₆₀ aryloxy group; fluorenyl group; a C₂-C₆₀ heterocyclic group including at least one heteroatom of O, N, S, Si or P; C₃-C₆₀ aliphatic ring; or a fused ring group of a C₃-C₆₀ aliphatic ring and a C₆-C₆₀ aromatic ring;
9) wherein, the aryl group, arylene group, heterocyclic group, fluorenyl group, fluorenylene group, fused ring group, alkyl group, alkenyl group, alkoxy group and aryloxy group may be substituted with one or more substituents selected from the group consisting of deuterium; halogen; silane group; siloxane group; boron group; germanium group; cyano group; nitro group; C₁-C₂₀ alkylthio group; C₁-C₂₀ alkoxyl group; C₁-C₂₀ alkyl group; C₂-C₂₀ alkenyl group; C₂-C₂₀ alkynyl group; C₆-C₂₅ aryl group; C₆-C₂₅ aryl group substituted with deuterium; a fluorenyl group; C₂-C₂₀ heterocyclic group; C₃-C₂₀ cycloalkyl group; C₇-C₂₀ arylalkyl group; C₈-C₂₀ arylalkenyl group; and - L'-N(Rₐ)(R_{b}); wherein the substituents may be bonded to each other to form a saturated or unsaturated ring, wherein the term 'ring' means a C₃-C₆₀ aliphatic ring, a C₆-C₆₀ aromatic ring, a C₂-C₆₀ heterocyclic group, a fused ring formed by combination thereof..

Formula 4-2 includes a compound represented by any one of Formulas 5-1 to 5-4.

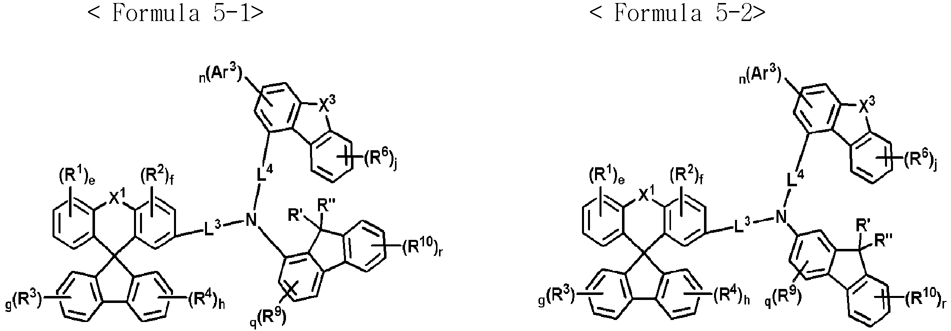

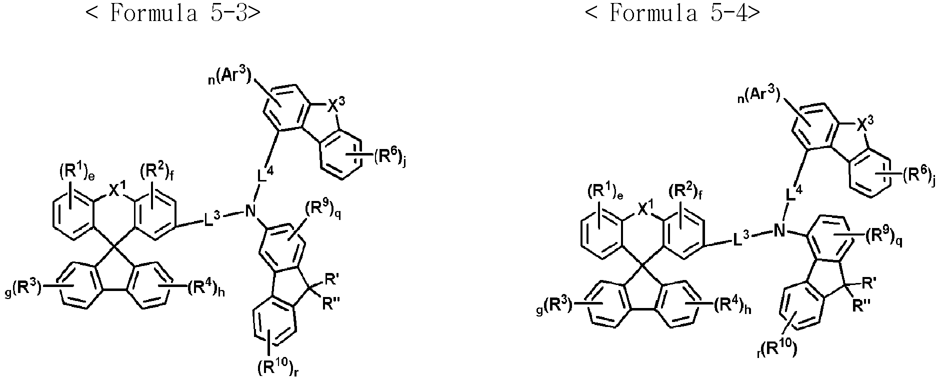

In Formulas 5-1 to 5-4,
1) X¹, X³, R¹, R², R³, R⁴, R⁶, L³, L⁴, Ar³, e, f, g, h, j, n, R⁹, R¹⁰, R', R", q and r are the same as defined Formula 4-2.

Formula 1 includes a compound represented by Formula 6-1.

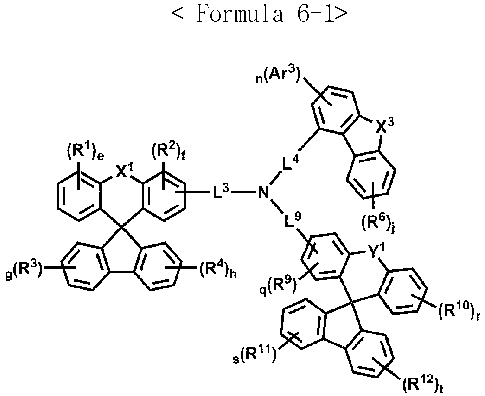

In Formula 6-1,
1) X¹, X³, R¹, R², R³, R⁴, R⁶, L³, L⁴, L⁹, Ar³, e, f, g, h, j and n are the same as defined in Formula 1,
2) R⁹, R¹⁰, q and r are the same as defined in Formula 4,
3) Y¹ is O or S,
4) R¹¹ and R¹² are the same as the definition of R¹ in Formula 1,
5) s and t are each independently an integer of 0 to 4.

Formula 1 includes a compound represented by Formula 6-2.

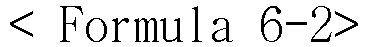

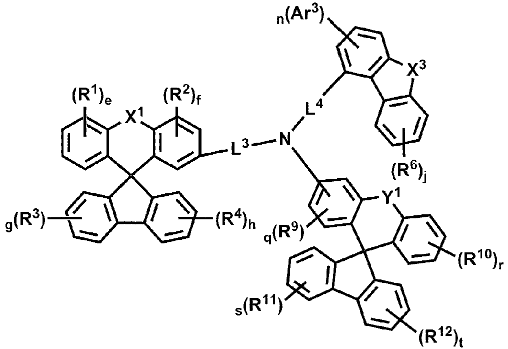

In Formula 6-2,
1) X¹, X³, R¹, R², R³, R⁴, R⁶, L³, L⁴, Ar³, e, f, g, h, j and n are the same as defined in Formula 1,
2) R⁹, R¹⁰, q and r are the same as defined in Formula 4,
3) Y¹, R¹¹, R¹², s and t are the same as defined in Formula 6-1.

Formula 1 includes a compound represented by Formula 6-3.

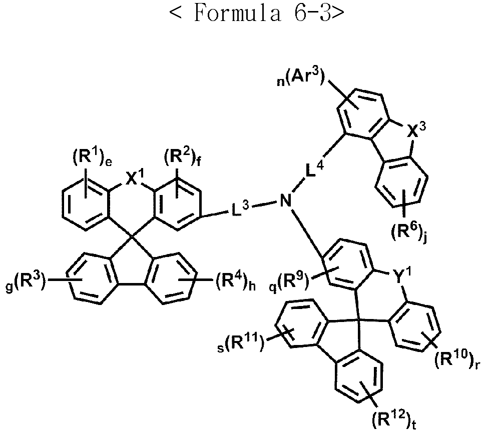

In Formula 6-3,
1) X¹, X³, R¹, R², R³, R⁴, R⁶, L³, L⁴, Ar³, e, f, g, h, j and n are the same as defined in Formula 1,
2) R⁹, R¹⁰, q and r are the same as defined in Formula 4,
3) Y¹, R¹¹, R¹², s and t are the same as defined in Formula 6-1.

Described herein are compounds represented by Formula 1 represented by any one of the following compounds P-1 to P-139.

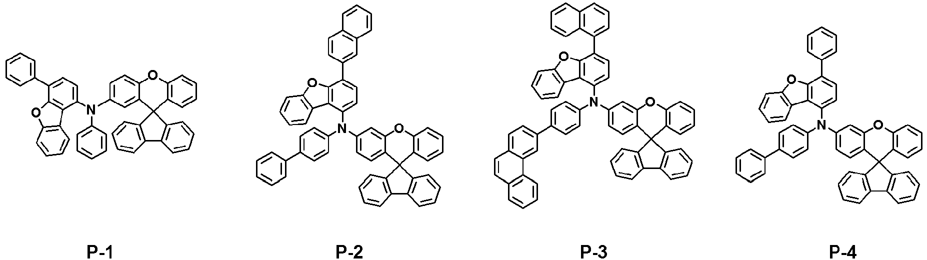

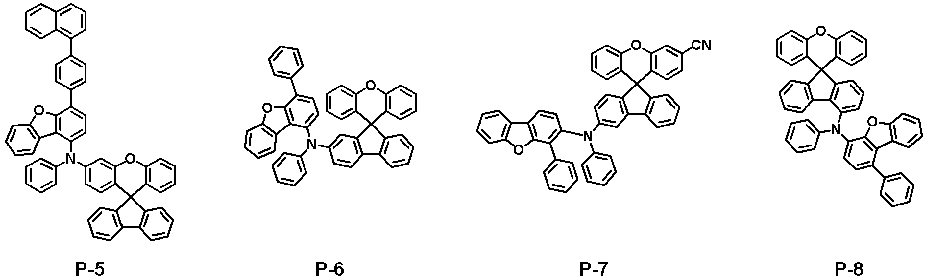

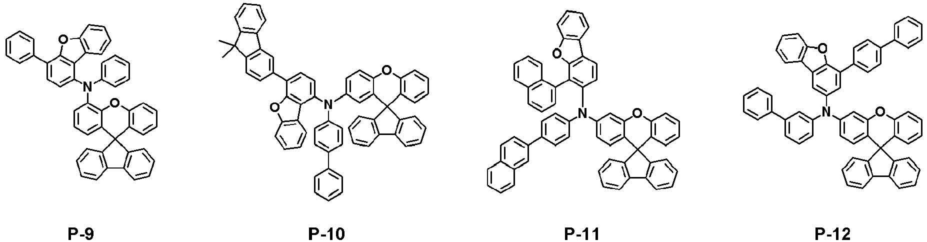

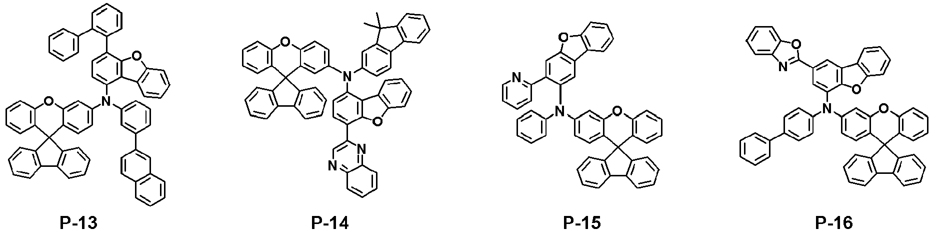

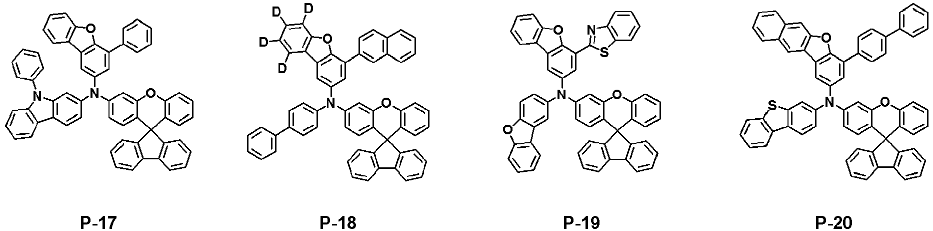

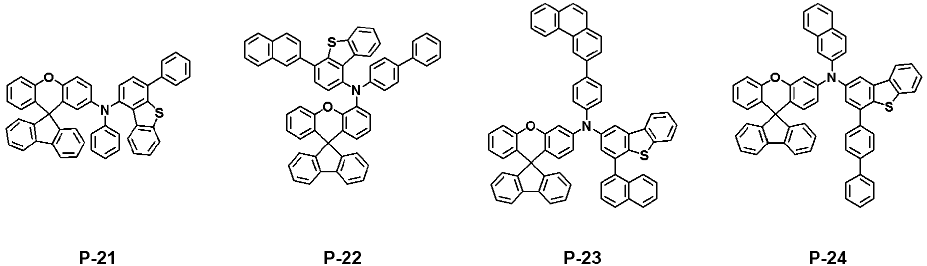

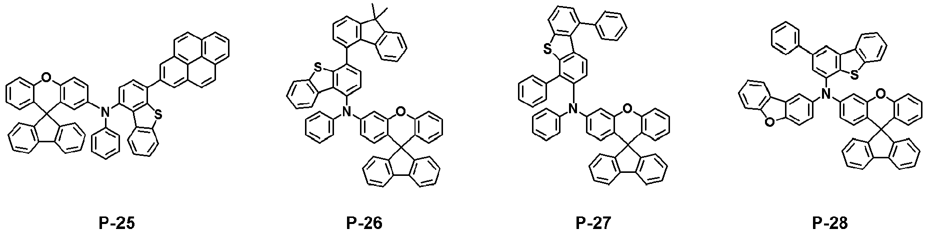

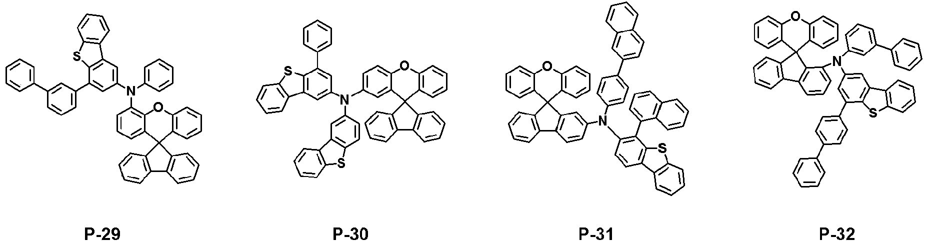

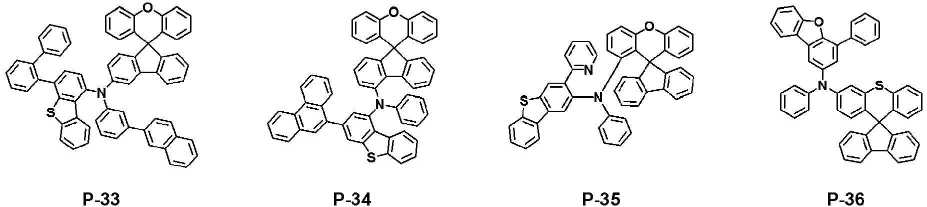

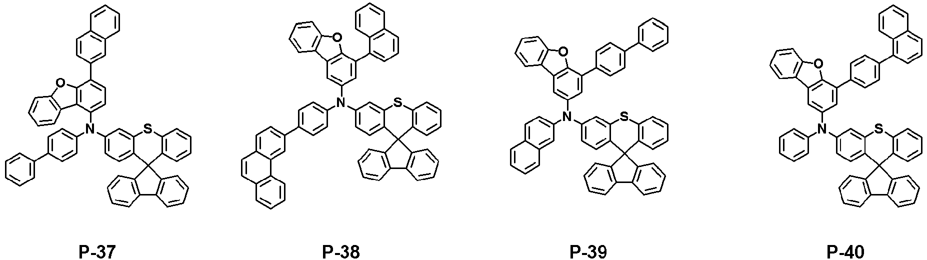

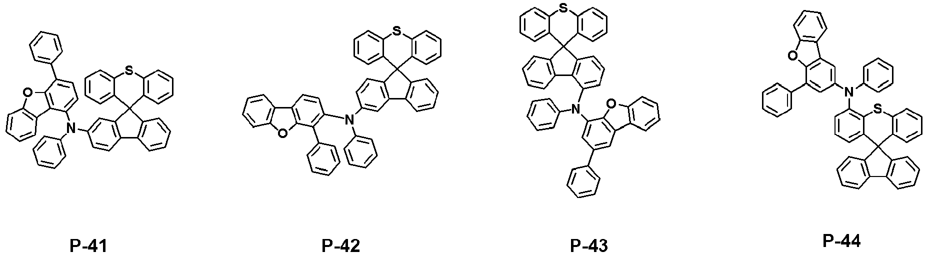

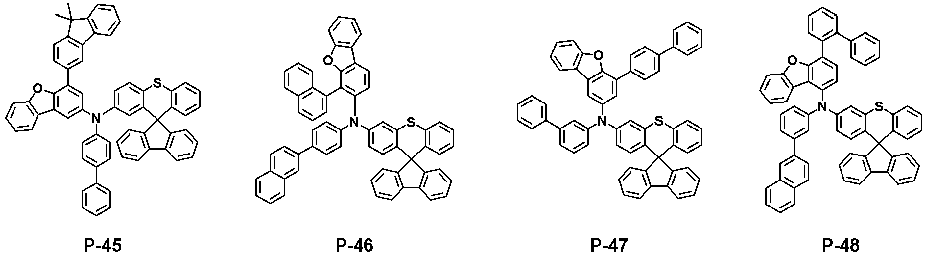

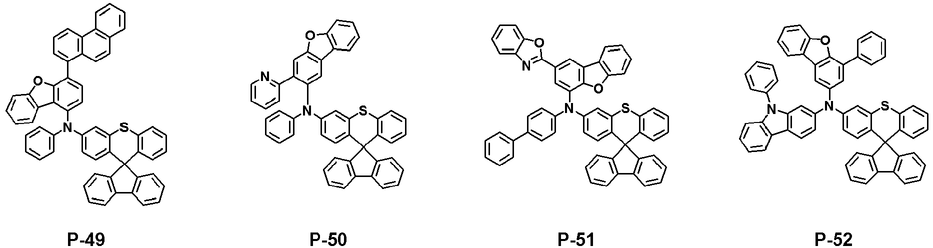

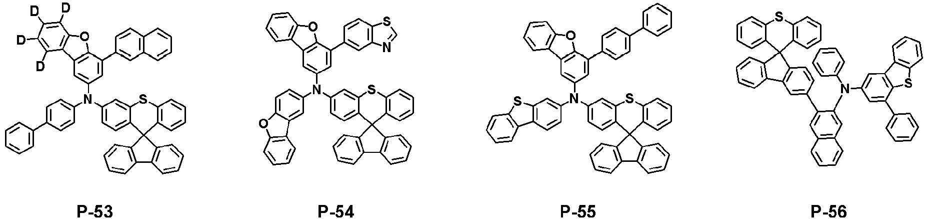

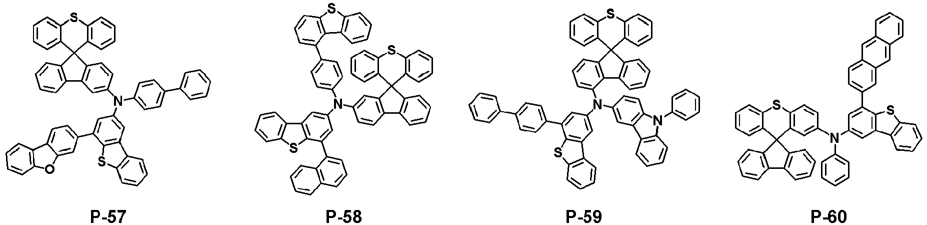

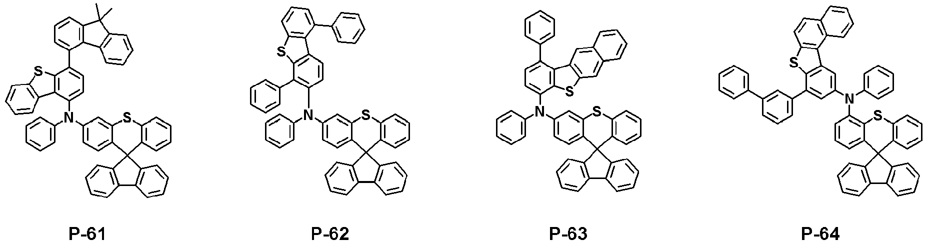

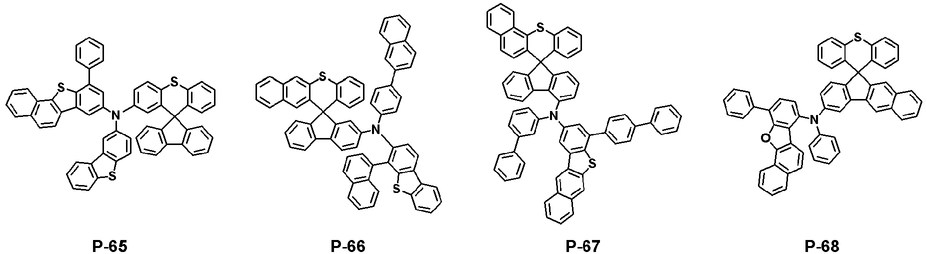

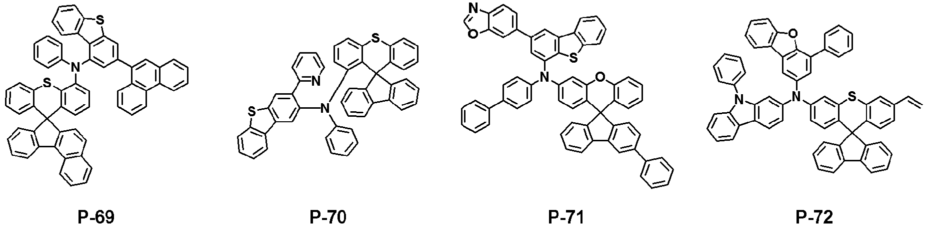

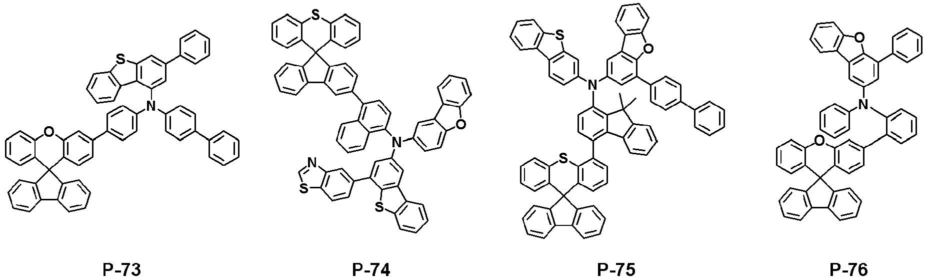

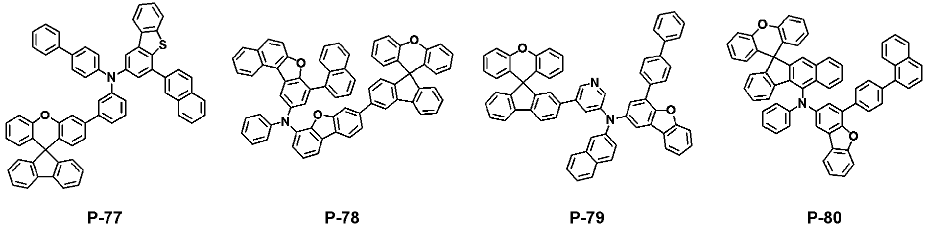

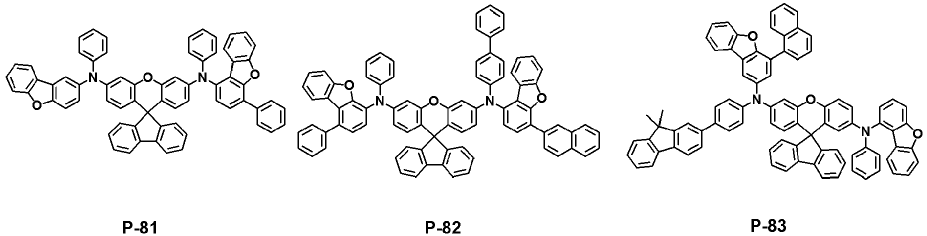

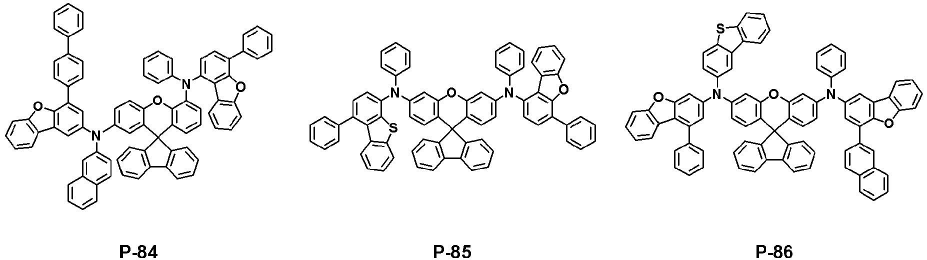

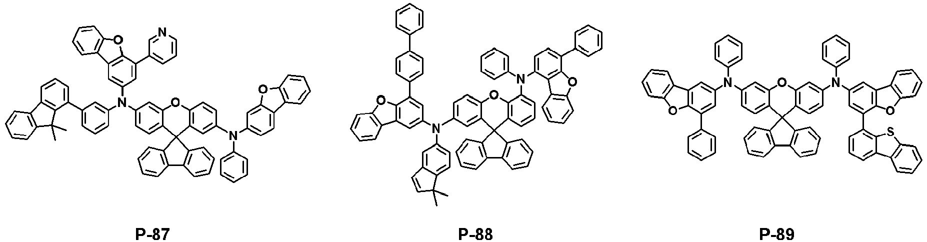

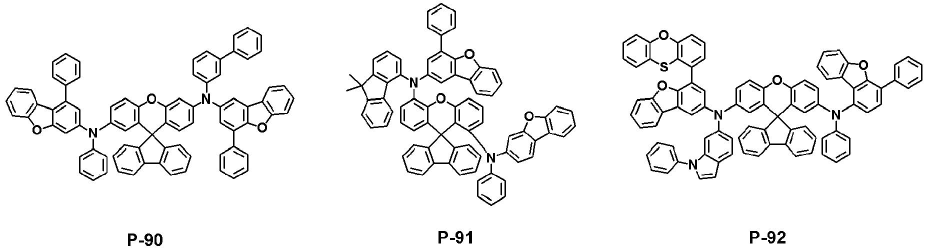

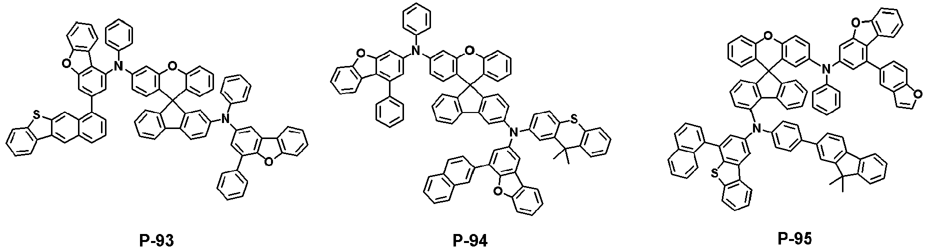

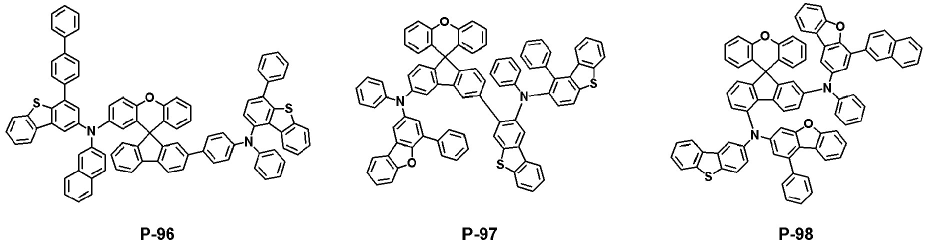

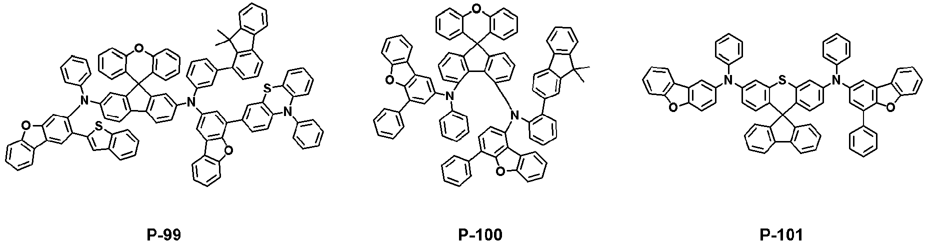

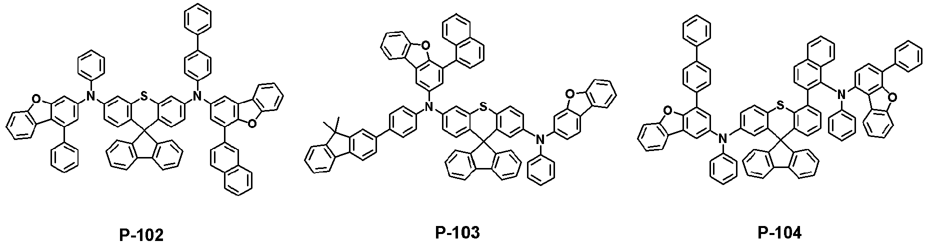

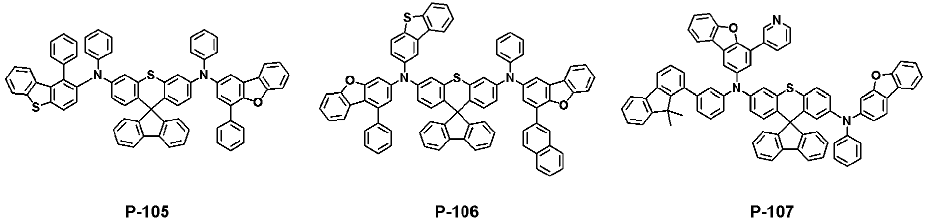

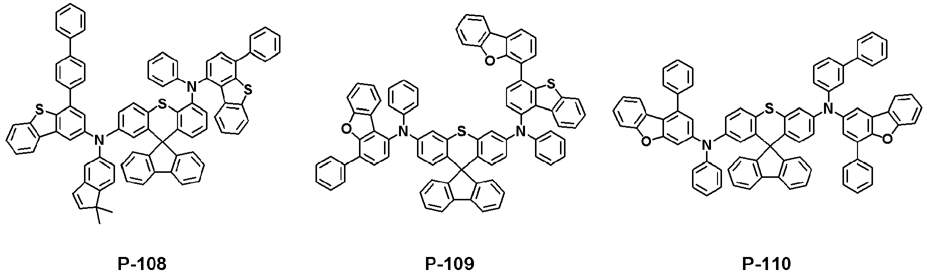

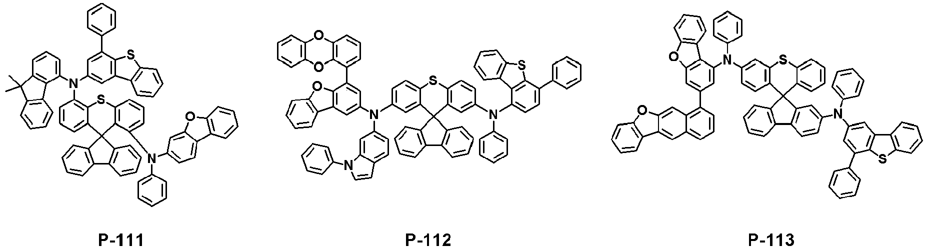

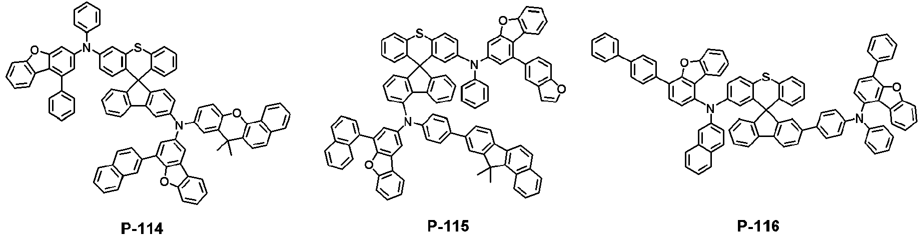

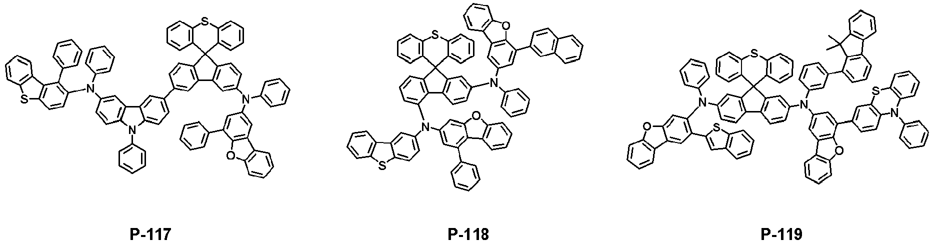

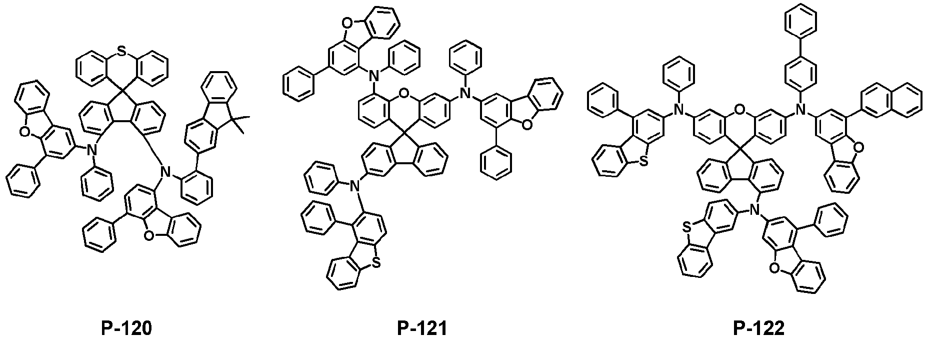

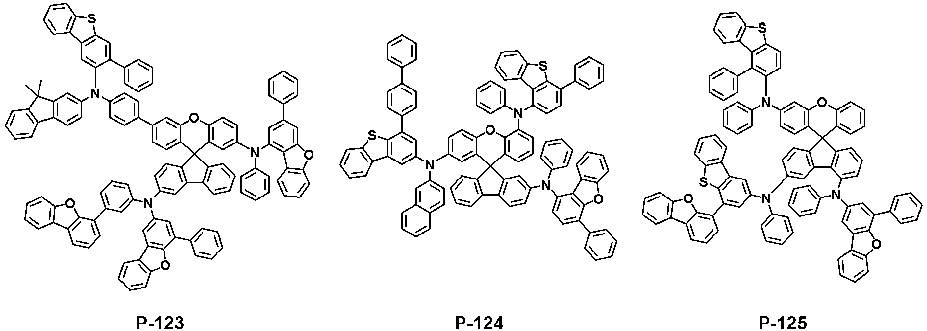

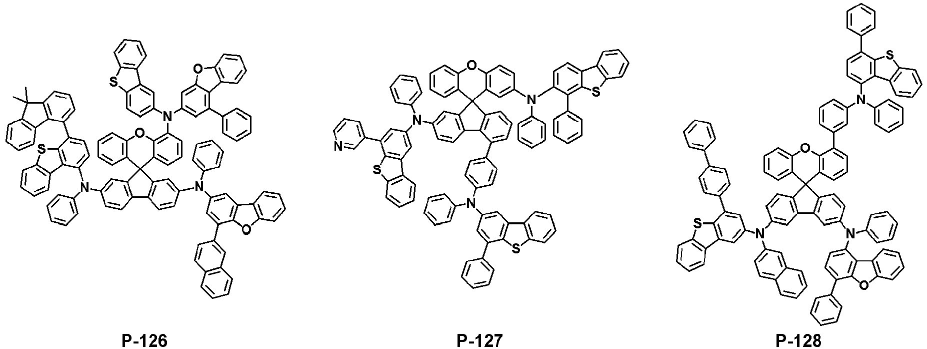

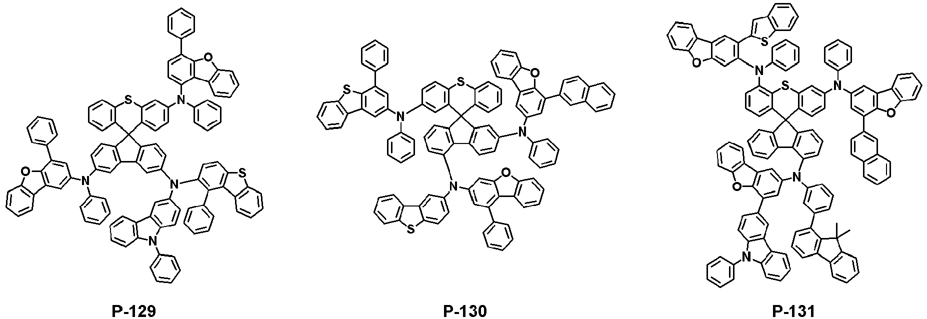

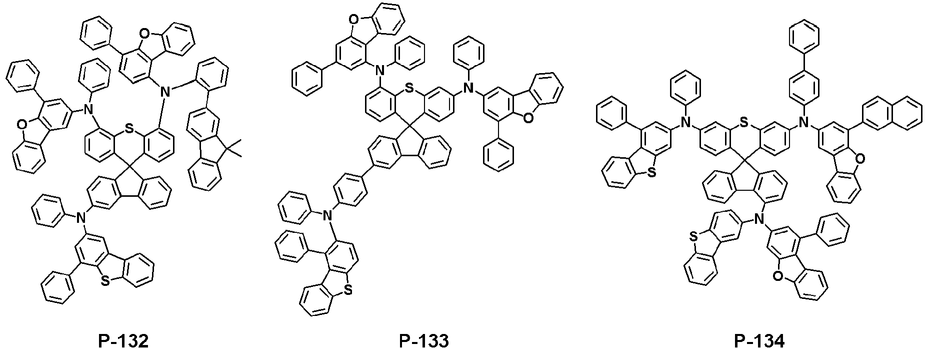

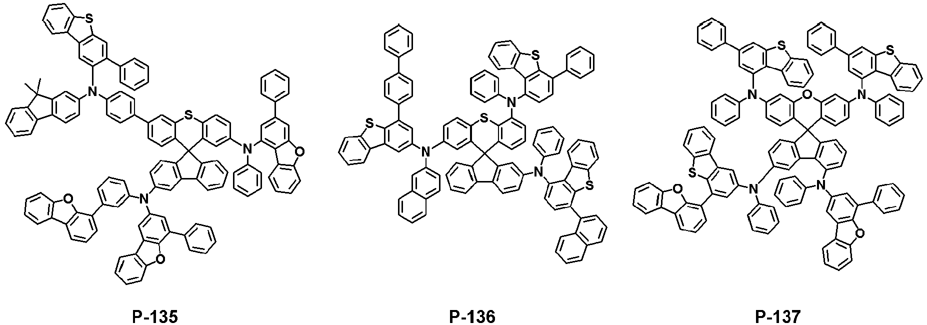

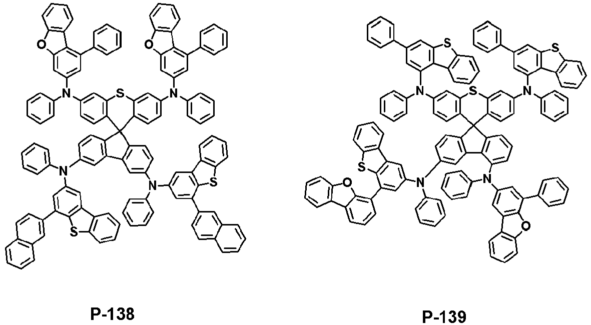

The compound according to the invention is represented by Formula 4-2 and is represented by any one of the following compounds P1-1 to P1-86.

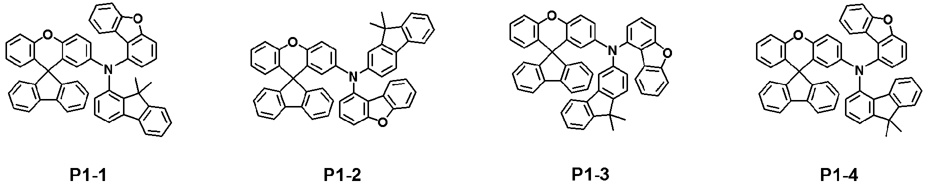

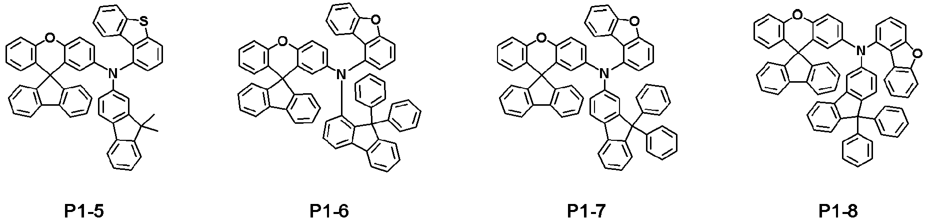

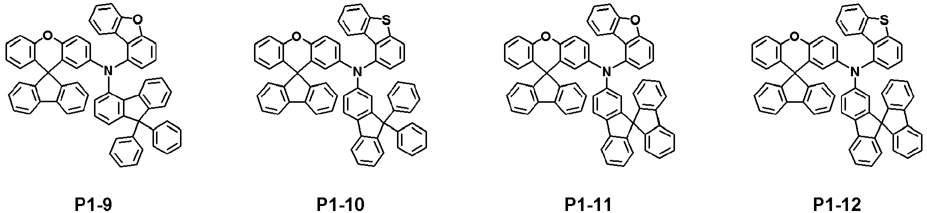

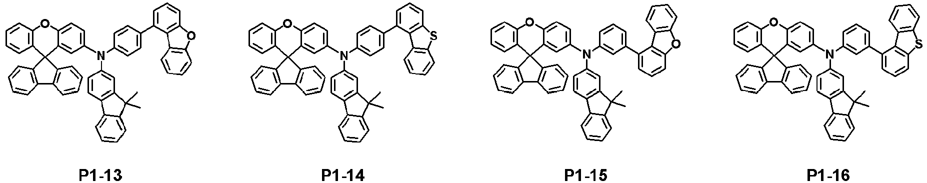

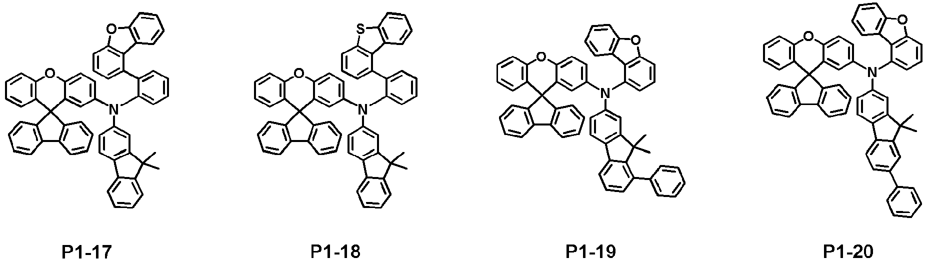

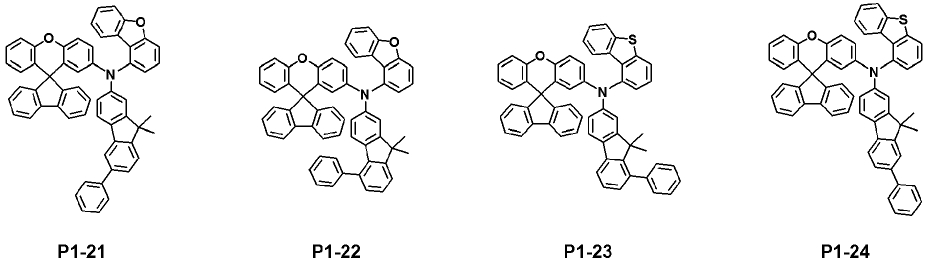

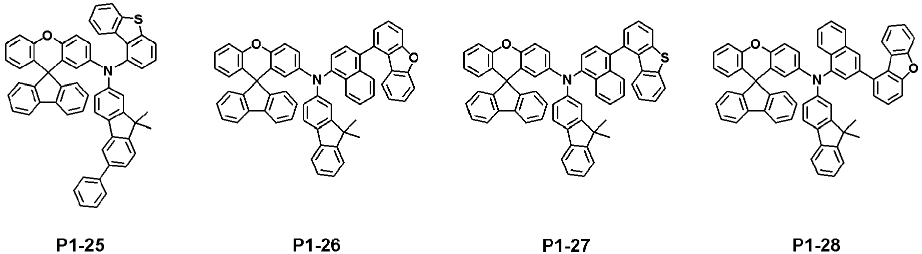

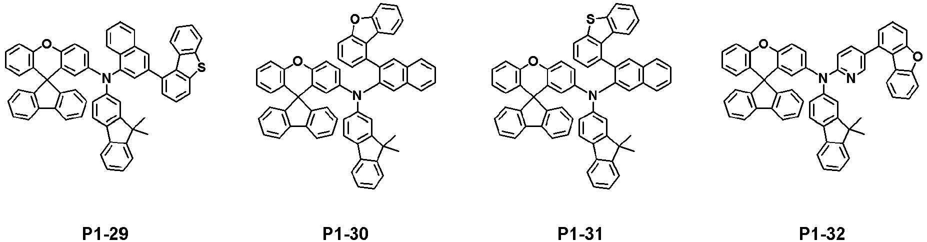

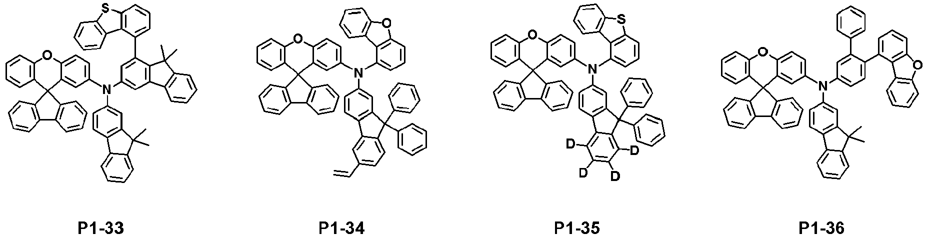

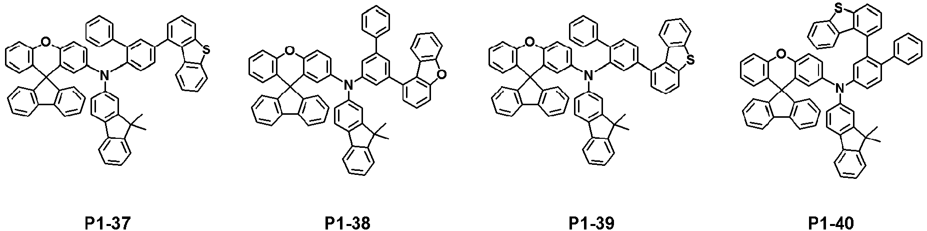

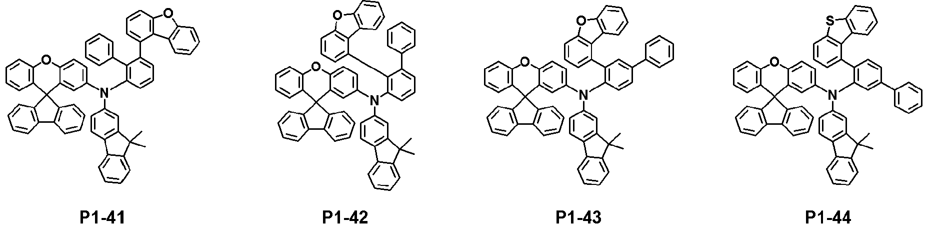

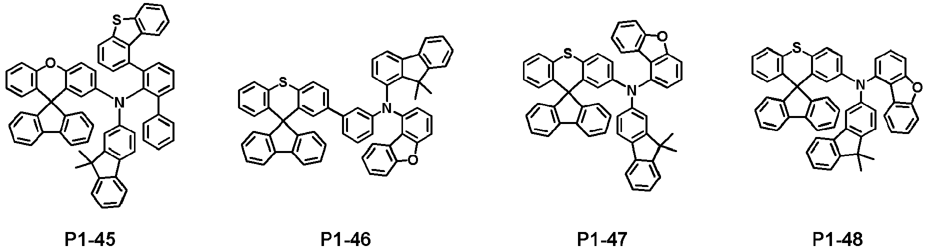

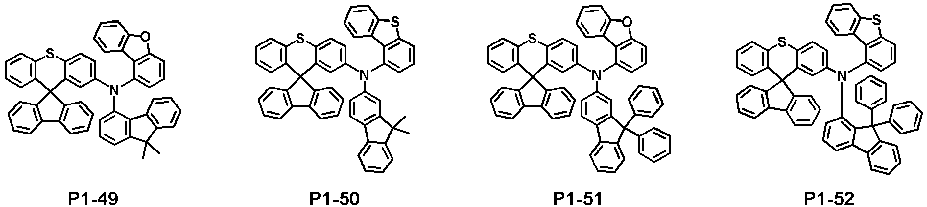

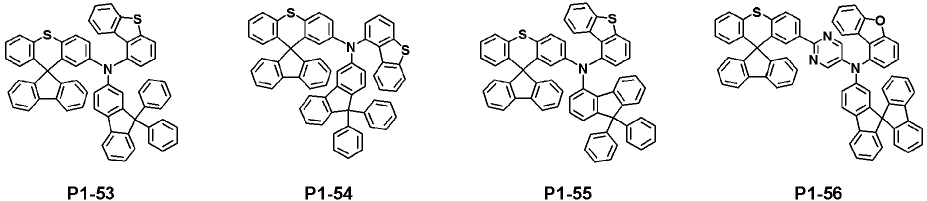

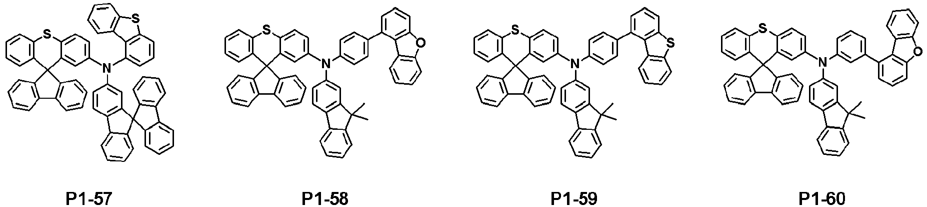

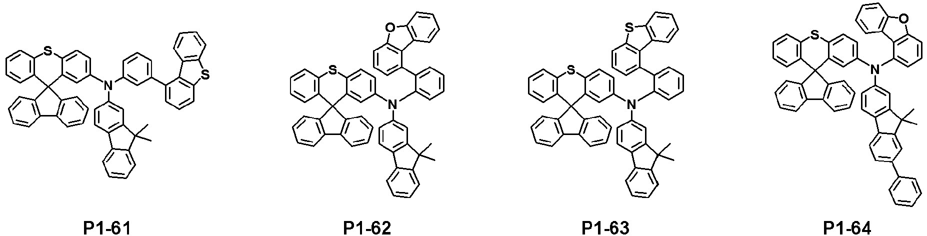

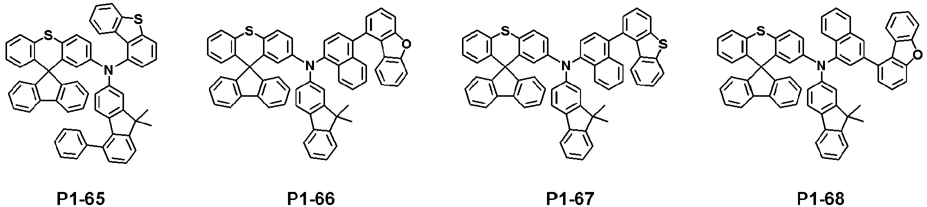

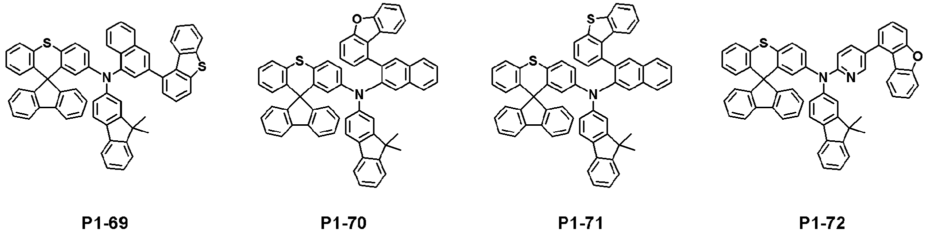

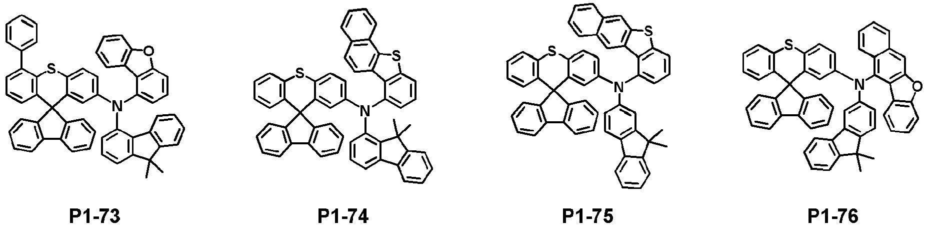

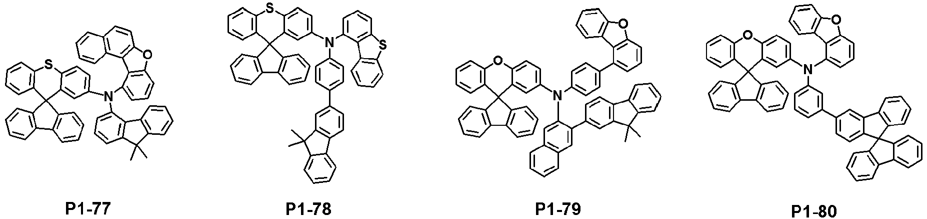

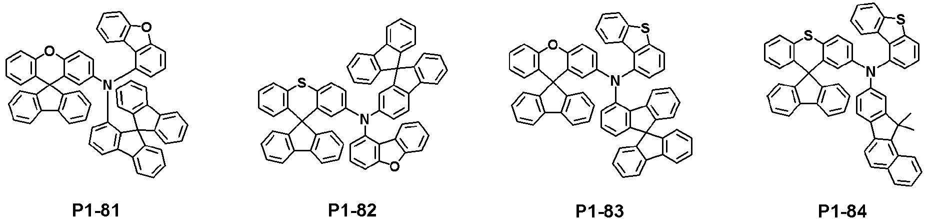

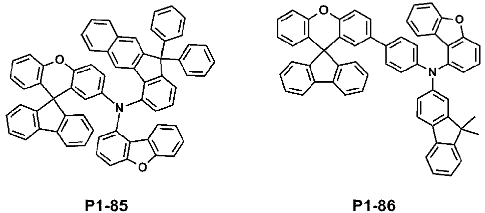

Described herein is a compound represented by Formula 1 represented by any one of the following compounds P2-1 to P2-55.

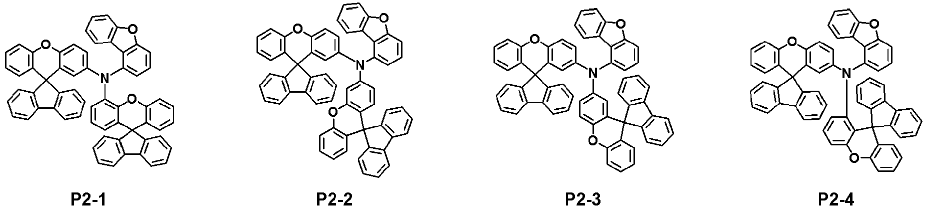

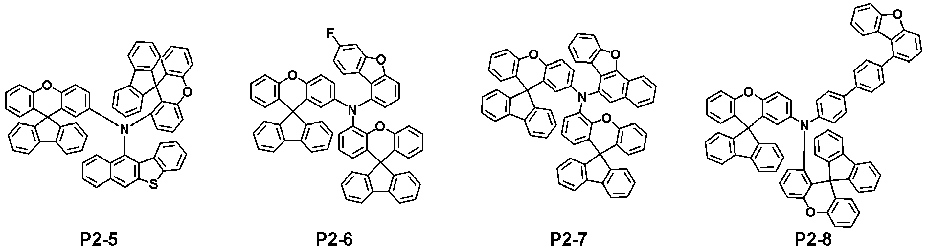

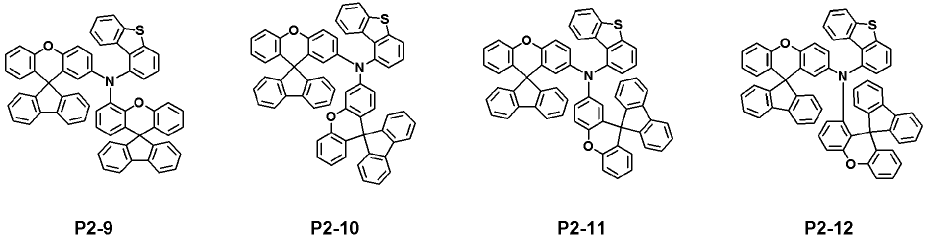

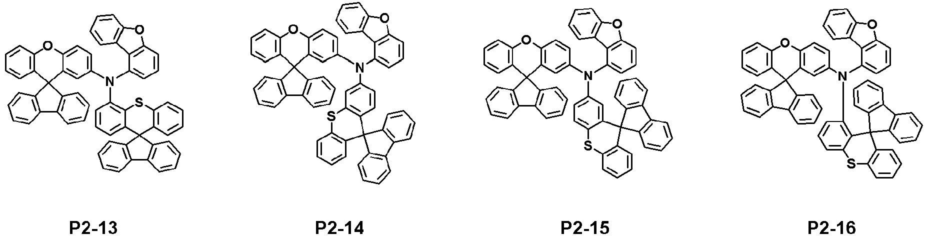

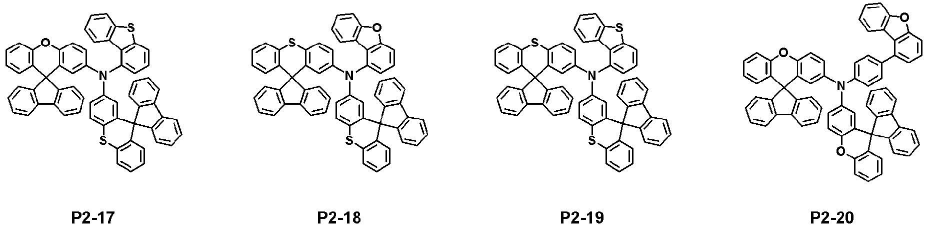

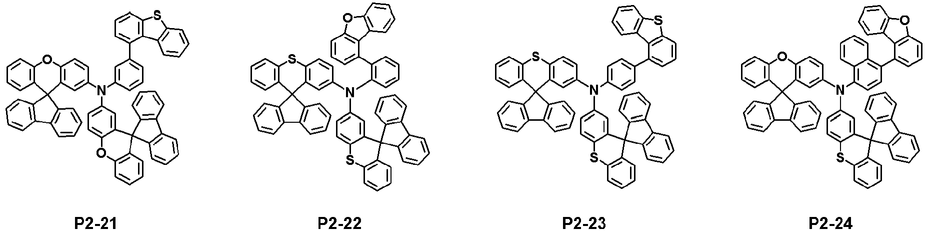

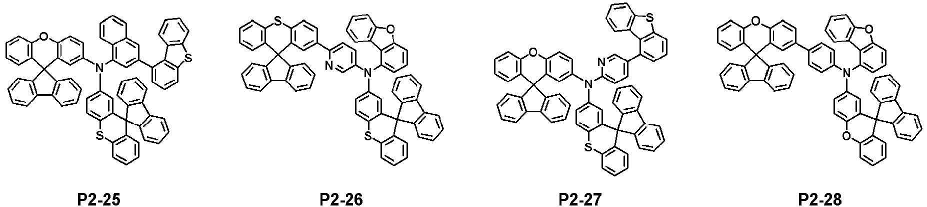

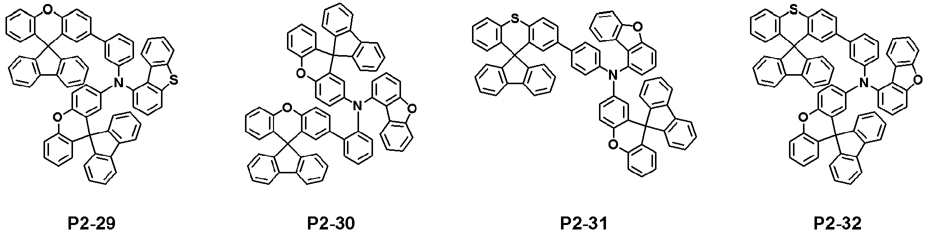

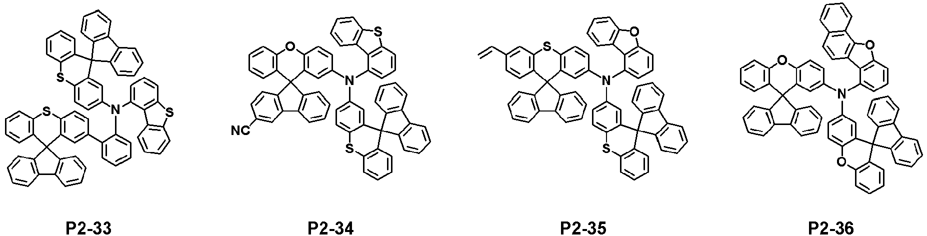

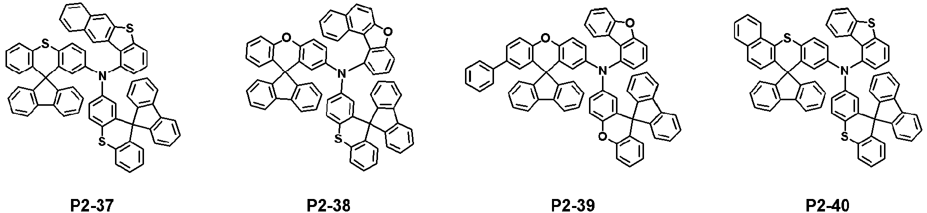

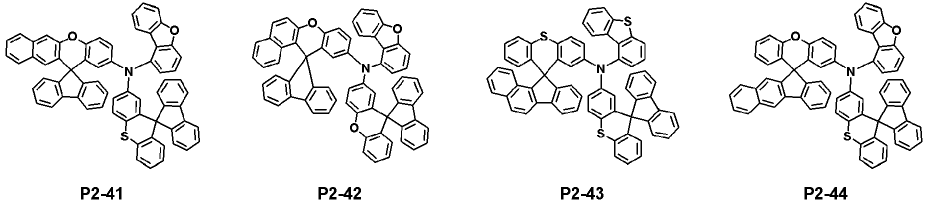

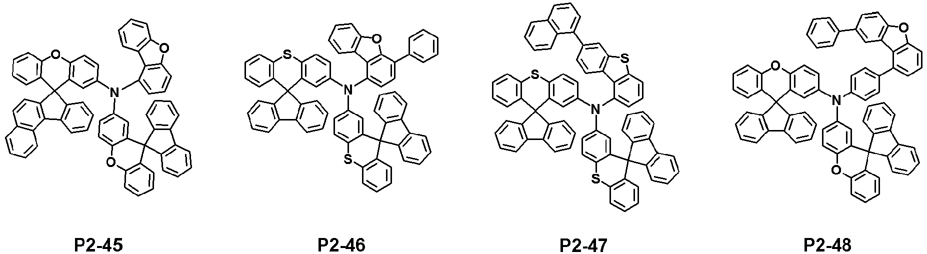

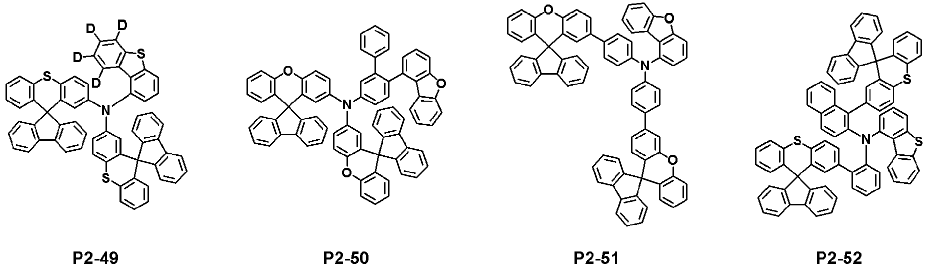

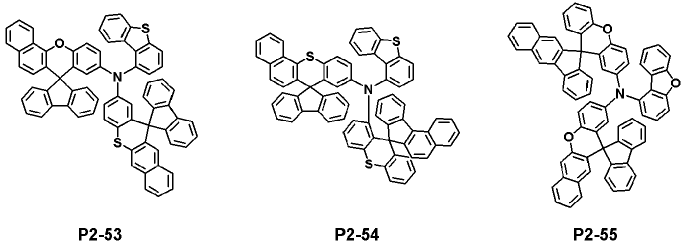

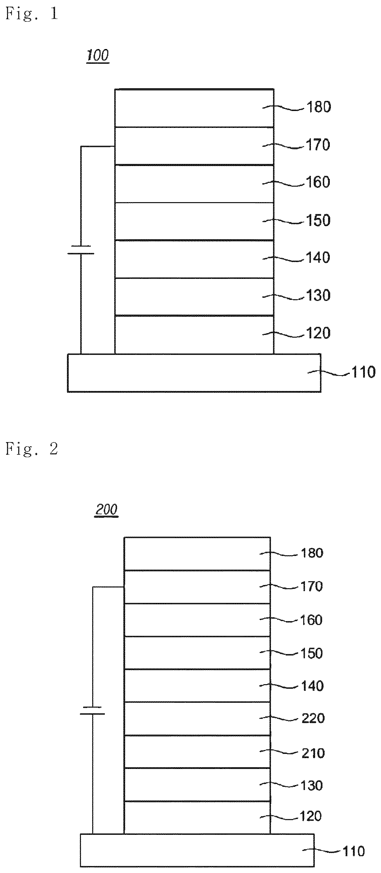

Referring to FIG. 1, the organic electronic element(100) according to the present invention includes a first electrode(110), a second electrode(170), an organic material layer comprising single compound or 2 or more compounds represented by Formula 4-2 between the first electrode(110) and the second electrode(170). Here, the first electrode(110) may be an anode or a positive electrode, and the second electrode(170) may be a cathode or a negative electrode. In the case of an inverted organic electronic element, the first electrode may be a cathode, and the second electrode may be an anode.

The organic material layer may sequentially include a hole injection layer(120), a hole transport layer (130), an emitting layer(140), an electron transport layer(150), and an electron injection layer(160) formed in sequence on the first electrode(110). Here, the remaining layers except the emitting layer(140) may not be formed. The organic material layer may further include a hole blocking layer, an electron blocking layer, an emitting-auxiliary layer(220), a buffer layer(210), etc., and the electron transport layer(150) and the like may serve as a hole blocking layer (see FIG. 2).

Also, the organic electronic element according to an embodiment of the present invention may further include a protective layer or a light efficiency enhancing layer(180). The light efficiency enhancing layer may be formed on a surface not in contact with the organic material layer among both surfaces of the first electrode or on a surface not in contact with the organic material layer among both surfaces of the second electrode. The compound according to an embodiment of the present invention applied to the organic material layer may be used as a material for a hole injection layer(120), a hole transport layer (130), an emitting-auxiliary layer(220), an electron transport auxiliary layer, an electron transport layer(150), an electron injection layer(160), a host or dopant of an emitting layer(140) or the light efficiency enhancing layer. Preferably, for example, the compound according to Formula 4-2 of the present invention may be used as a material for the emitting-auxiliary layer.

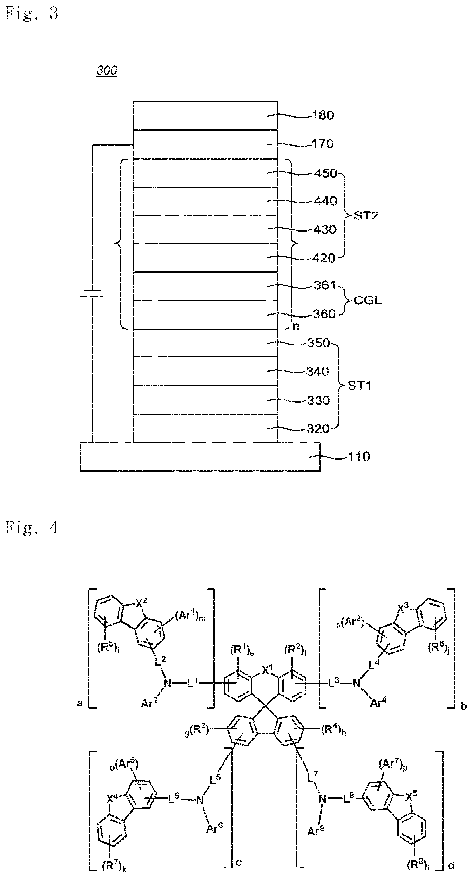

The organic material layer may include 2 or more stacks including a hole transport layer, an emitting layer, and an electron transport layer sequentially formed on the anode, and may further include a charge generation layer formed between the 2 or more stacks (see FIG. 3).

Otherwise, even if the same core is used, the band gap, the electrical characteristics, the interface characteristics, and the like may vary depending on which substituent is bonded at which position, therefore the choice of core and the combination of sub-substituents associated therewith is also very important, and in particular, when the optimal combination of energy levels and T1 values and unique properties of materials (mobility, interfacial characteristics, etc.) of each organic material layer is achieved, a long life span and high efficiency can be achieved at the same time.

The organic electroluminescent device according to an embodiment of the present invention may be manufactured using a PVD (physical vapor deposition) method. For example, a metal or a metal oxide having conductivity or an alloy thereof is deposited on a substrate to form a cathode, and the organic material layer including the hole injection layer(120), the hole transport layer(130), the emitting layer(140), the electron transport layer(150), and the electron injection layer(160) is formed thereon, and then depositing a material usable as a cathode thereon can manufacture an organic electroluminescent device according to an embodiment of the present invention.

Also, the present invention provides the organic electronic element wherein the organic material layer is formed by one of a spin coating process, a nozzle printing process, an inkjet printing process, a slot coating process, a dip coating process or a roll-to-roll process, and the organic material layer provides an organic electronic element comprising the compound as an electron transport material.

As another specific example, the present invention provides an organic electronic element that is used by mixing the same or different compounds of the compound represented by Formula 4-2 to the organic material layer.

In another aspect, the present invention provides an emitting-auxiliary layer composition comprising a compound represented by Formula 4-2, and provides an organic electronic element comprising the emitting-auxiliary layer.

The present invention also provides an electronic device comprising a display device including the organic electronic element, and a control unit for driving the display device.

According to another aspect, the present invention provides a display device, wherein the organic electronic element is at least one of an OLED, an organic solar cell, an organic photo conductor, an organic transistor organic TFT, and an element for monochromic or white illumination. Here, the electronic device may be a wired/wireless communication terminal which is currently used or will be used in the future, and covers all kinds of electronic devices including a mobile communication terminal such as a cellular phone, a personal digital assistant (PDA), an electronic dictionary, a point-to-multipoint (PMP), a remote controller, a navigation unit, a game player, various kinds of TVs, and various kinds of computers.

Hereinafter, synthesis examples of the compound represented by Formula 4-2 of the present invention, preparation examples of the organic electronic element of the present invention, and reference examples are described in detail.

### [Synthesis Example 1]

The compound (Final product) represented by Formula 1 as described herein may be prepared by reacting as in Scheme 1, but is not limited thereto.

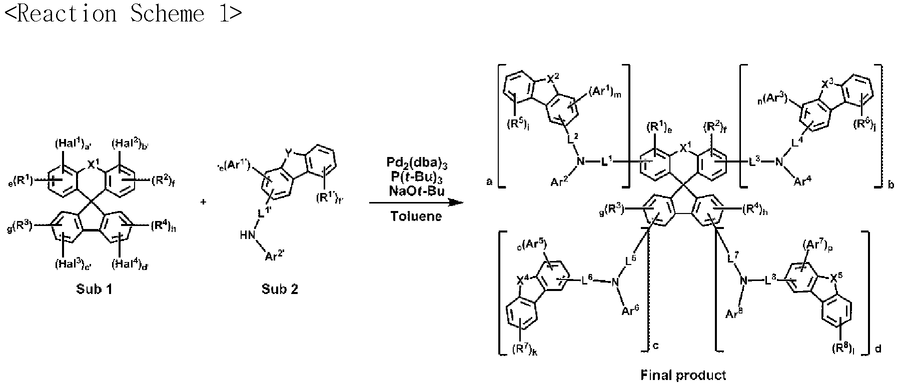

In Reaction Scheme 1,
1) X¹, X², X³, X⁴, X⁵, R¹, R², R³, R⁴, R⁵, R⁶, R⁷ and R⁸, L¹, L², L³, L⁴, L⁵, L⁶, L⁷ and L⁸, Ar¹, Ar², Ar³, Ar⁴, Ar⁵, Ar⁶, Ar⁷, and Ar⁸, and a to p are the same as defined above,
2) Hal¹ to Hal⁴ are each independently Br or Cl,
3) a' to d' are each independently 0 or 1, at least one of a' to d' is 1,
4) Y is the same as the definition of X¹, X², X³, X⁴, X⁵,
5) Ar¹' is the same as the definition of Ar¹, Ar³, Ar⁵ or Ar⁷,
6) Ar²' is the same as the definition of Ar², Ar⁴, Ar⁶ or Ar⁸,
7) R¹' is the same as the definition of R⁵, R⁶, R⁷, R⁸
8) L¹' is the same as the definition of L², L⁴, L⁶ or L⁸,
9) e' is an integer of 0 to 3, f' is an integer of 0 to 4.

### I. Synthesis of Sub 1

Sub 1 of Reaction Scheme 1 may be synthesized by the reaction path of Scheme 2, but is not limited thereto. When X¹ is -OH, the synthesis path of (1) of Reaction Scheme 2 is followed, and when X¹ is -SH, the synthesis path of (2) of Reaction Scheme 2 is followed.

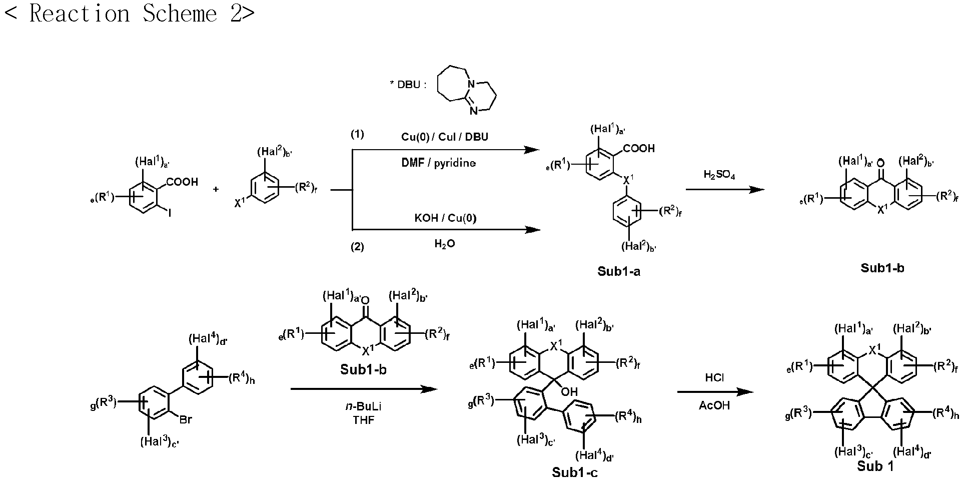

In Reaction Scheme 2,
X¹, R¹, R², R³, R⁴, e, f, g, h, Hal¹, Hal², Hal³, Hal⁴, and a', b', c', d' are the same as defined in Formula 1.

Synthesis examples of specific compounds belonging to Sub 1 are as follows.

### 1. Synthesis example of Sub 1-1

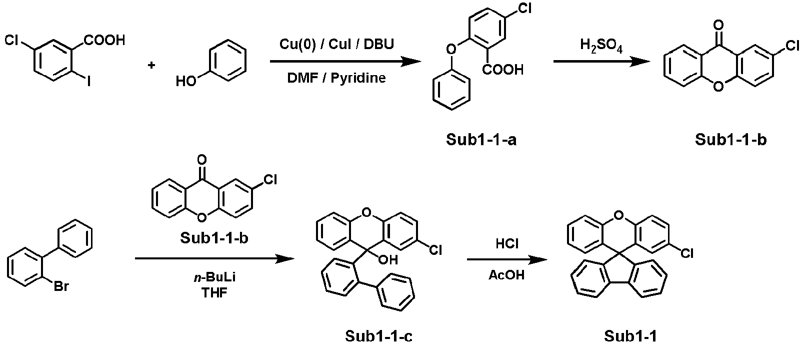

### (1) Synthesis example of Sub 1-1-a

5-Chloro-2-iodobenzoic acid (50.0 g, 177 mmol), phenol (33.3 g, 354 mmol), 1,8-diazabicyclo[5.4.0]undec-7-ene (DBU) (80.9 g, 531 mmol), pyridine (2.9 mL), copper powder (1.5 g, 23 mmol), CuI (1.5 g, 7.97 mmol) were placed in a round bottom flask, and DMF (1.2 L) was added, followed by refluxing for 3 hours. When the reaction is complete, cool to room temperature and add 3M HCl until precipitation is complete. Thereafter, the precipitate was washed with water and dried to obtain 38.3 g (yield 87%) of the product.

### (2) Synthesis example of Sub 1-1-b

Sub 1-1-a (38.3 g, 154 mmol) obtained in the above synthesis was added to a round bottom flask, and H₂SO₄ (1.1mL, 21.5 mmol) was added, followed by refluxing until all the starting materials were dissolved. When all the starting materials are dissolved, cool to room temperature and add ice water to precipitate. Thereafter, the precipitate was washed with water, dried, dissolved in CH₂Cl₂, and recrystallized with a Silica gel column to obtain 23.09 g (yield 65%) of the product.

### (3) Synthesis example of Sub 1-1-c

2-Bromo-1,1'-biphenyl (23.3 g, 99.7 mmol) was dissolved in THF (270 mL) in a round bottom flask under a nitrogen atmosphere, and then cooled to -78° C.

Then, *n*-BuLi (40 mL) was slowly titrated and the mixture was stirred for 30 minutes. Subsequently, Sub1-1-b (23 g, 99.7 mmol) obtained in the above synthesis was dissolved in THF (140 mL), and then slowly titrated into a reaction round bottom flask. After stirring for an additional 1 hour at - 78 °C, it is gradually raised to room temperature. When the reaction was completed, the product was extracted with Ethyl acetate and water, and the organic layer was dried over MgSO₄ and concentrated, and the resulting compound was recrystallized in a silicagel column to obtain 32.6 g (yield 85%) of the product.

### (4) Synthesis example of Sub 1-1

Sub 1-1-c (32 g, 84.7 mmol) obtained in the above synthesis, acetic acid (208 mL), and concentrated hydrochloric acid (34.6 mL) were added to a round bottom flask, followed by stirring at 60 to 80° C for 3 hours under a nitrogen atmosphere. When the reaction was completed, the product was extracted with CH₂Cl₂ and water, and the organic layer was dried over MgSO₄ and concentrated, and the resulting compound was recrystallized with a silica gel column to obtain 27.7 g (91% yield) of the product.

### 2. Synthesis example of Sub 1-11

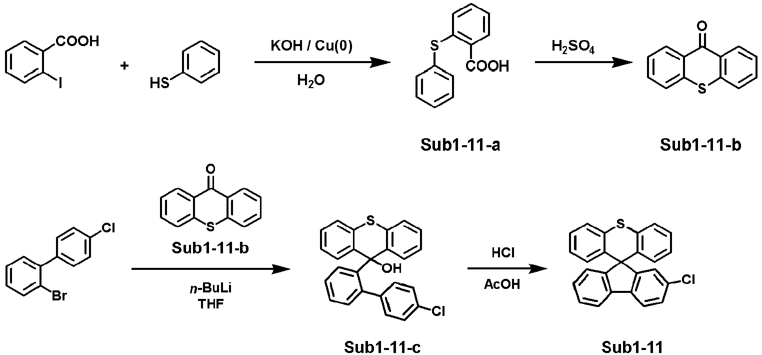

### (1) Synthesis example of Sub 1-11-a

2-Iodobenzoic acid (50.0 g, 202 mmol), thiophenol (22.2 g, 202 mmol), potassium hydroxide (56.6 g, 1008 mmol), copper powder (1.3 g, 20.2 mmol) were placed in a round bottom flask and water (1.3 L) was added, followed by refluxing for 12 hours. When the reaction is complete, cool to room temperature and add 3M HCl until precipitation is complete. Thereafter, the precipitate was washed with water and dried to obtain 41.3 g (yield 89%) of the product.

### (2) Synthesis example of Sub 1-11-b

Sub 1-11-a (41.3 g, 179 mmol) and H₂SO₄ (1.3 mL) obtained in the above synthesis was used for the synthesis of Sub 1-1-b to obtain 25.9 g (68% yield) of a product.

### (3) Synthesis example of Sub 1-11-c

2-Bromo-4'-chloro-1,1'-biphenyl (32.6 g, 122 mmol), *n*-BuLi (49 mL), Sub 1-11-b (25.9 g, 122 mmol) obtained in the above synthesis was used for the synthesis of Sub 1-1-c to obtain 40.1 g (82% yield) of a product.

### (4) Synthesis example of Sub 1-11

Sub 1-11-c (40.1 g, 100 mmol) obtained in the above synthesis, acetic acid (250 mL), and concentrated hydrochloric acid (40 mL) were obtained by using the synthesis method of Sub 1-1 above to obtain 31.8 g (yield 83%) of the product.

### 3. Synthesis example of Sub 1-20

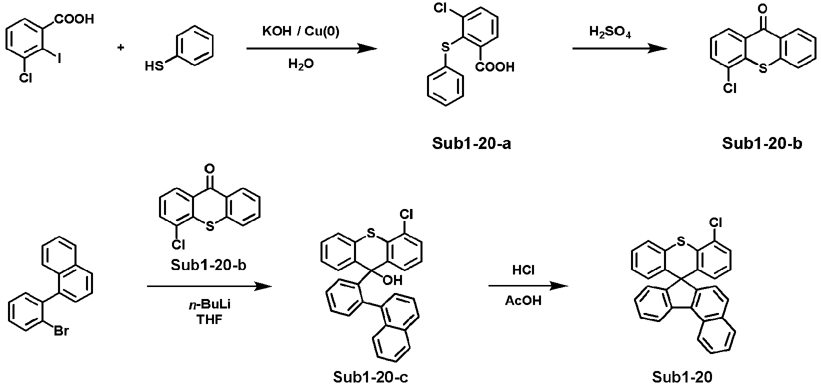

### (1) Synthesis example of Sub 1-20-a

3-Chloro-2-iodobenzoic acid (30.0 g, 106.2 mmol), thiophenol (11.7 g, 106.2 mmol), potassium hydroxide (29.8 g, 531 mmol), copper powder (0.67 g, 10.6 mmol) were used to obtain 23.6 g of a product (84% yield) using the synthesis method of Sub 1-11-a.

### (2) Synthesis example of Sub 1-20-b

Sub 1-20-a (23.6 g, 89.2 mmol) and H₂SO₄ (0.67 mL) obtained in the above synthesis was used for the synthesis of Sub 1-1-b to obtain 14.5 g (66% yield) of a product.

### (3) Synthesis example of Sub 1-20-c

1-(2-Bromophenyl)naphthalene (16.6 g, 58.8 mmol), *n*-BuLi (24 mL), Subl-20-b (14.5 g, 58.8 mmol) obtained in the above synthesis was used for the synthesis of Sub 1-1-c to obtain 21.5 g (81% yield) of a product.

### (4) Synthesis example of Sub 1-20

Sub 1-20-c (21 g, 46.6 mmol) obtained in the above synthesis, acetic acid (116 mL), and concentrated hydrochloric acid (19 mL) were obtained by using the synthesis method of Sub 1-1 above to obtain 15.9 g (yield 79%) of the product.

### 4. Synthesis example of Sub 1-55

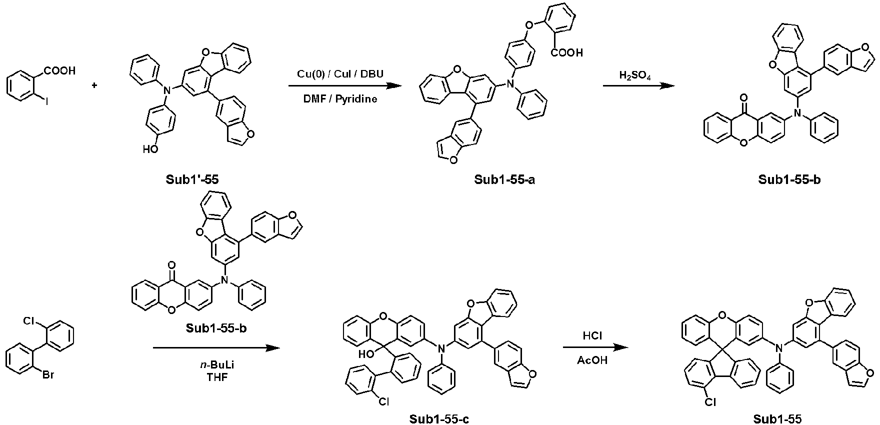

### (1) Synthesis example of Sub 1-55-a

2-Iodobenzoic acid (15 g, 60.5 mmol), Sub1'-55 (37.9 g, 120.1 mmol), 1,8-diazabicyclo[5.4.0]undec-7-ene (DBU) (27.6 g, 181.4 mmol), pyridine (0.98 mL), copper powder (0.5 g, 7.9 mmol), CuI (0.5 g, 2.7 mmol) were used to obtain 27.7 g of a product (78% yield) using the synthesis method of Sub 1-1-a.

### (2) Synthesis example of Sub 1-55-b

Sub 1-55-a (27.7 g, 47.1 mmol) and H₂SO₄ (0.35 mL) obtained in the above synthesis was used for the synthesis of Sub 1-1-b to obtain 17.7 g (64% yield) of a product.

### (3) Synthesis example of Sub 1-55-c

2-Bromo-2'-chloro-1,1'-biphenyl (7.8 g, 29 mmol), *n*-BuLi (11.7 mL), Sub 1-55-b (17 g, 29 mmol) obtained in the above synthesis was used for the synthesis of Sub 1-1-c to obtain 17.3 g (77% yield) of a product.

### (4) Synthesis example of Sub 1-55

Sub 1-55-c (17.3 g, 22.3 mmol) obtained in the above synthesis, acetic acid (56 mL), and concentrated hydrochloric acid (9.3 mL) were obtained by using the synthesis method of Sub 1-1 above to obtain 13.5 g (yield 80%) of the product.

### 5. Synthesis example of Sub 1-140

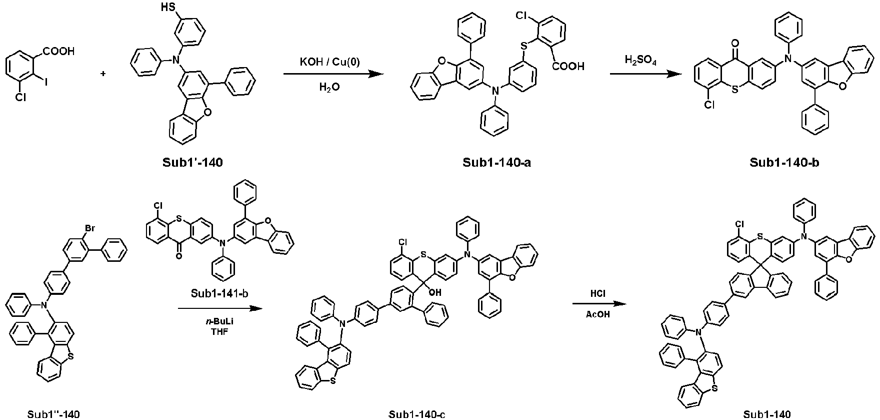

### (1) Synthesis example of Sub 1-140-a

3-Chloro-2-iodobenzoic acid (18 g, 63.7 mmol), Sub1'-140 (28.3 g, 63.7 mmol), potassium hydroxide (17.9 g, 318.6 mmol), copper powder (0.4 g, 6.4 mmol) were used to obtain 30.1 g of a product (79% yield) using the synthesis method of Sub 1-11-a.

### (2) Synthesis example of Sub 1-140-b

Sub 1-140-a (30.1 g, 46.3 mmol) and H₂SO₄ (0.35 mL) obtained in the above synthesis was used for the synthesis of Sub 1-1-b to obtain 18.1 g (62% yield) of a product.

### (3) Synthesis example of Sub 1-140-c

Sub1"-140 (19.9 g, 30.3 mmol), *n*-BuLi (12.2 mL), Sub1-141-b (18.1 g, 30.3 mmol) obtained in the above synthesis were used for the synthesis of Sub 1-1-c to obtain 24.5 g (70% yield) of a product.

### (4) Synthesis example of Sub 1-140

Sub 1-140-c (24.5 g, 21.1 mmol) obtained in the above synthesis, acetic acid (53 mL), and concentrated hydrochloric acid (8.8 mL) were obtained by using the synthesis method of Sub 1-1 above to obtain 18.1 g (yield 75%) of the product.

### 6. Synthesis example of Sub 1-152

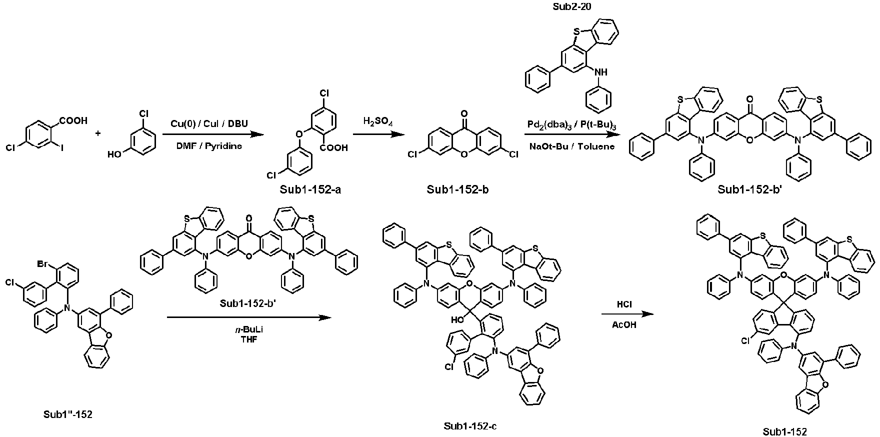

### (1) Synthesis example of Sub 1-152-a

4-Chloro-2-iodobenzoic acid (12 g, 42.5 mmol), 3-chlorophenol (10.9 g, 84.97 mmol), 1,8-diazabicyclo[5.4.0]undec-7-ene (DBU) (19.4 g, 127.5 mmol), pyridine (0.69 mL), copper powder (0.35 g, 5.5 mmol), CuI (0.36 g, 1.9 mmol) were used to obtain 9.7 g of a product (81% yield) using the synthesis method of Sub 1-1-a.

### (2) Synthesis example of Sub 1-152-b

Sub 1-152-a (9.7 g, 34.3 mmol) and H₂SO₄ (0.26 mL) obtained in the above synthesis was used for the synthesis of Sub 1-1-b to obtain 6.6 g (73% yield) of a product.

### (3) Synthesis example of Sub 1-152-b'

The obtained Sub 1-152-b (6.6 g, 24.9 mmol), Sub 2-20 (17.5 g, 49.8 mmol), Pd₂(dba)₃ (0.68 g, 0.75 mmol), P(t-Bu)₃ (50wt% Sol.) (0.6 mL, 1.5 mmol), t-BuONa (7.18 g, 74.7 mmol) were added to anhydrous toluene (150 mL) and reacted for 3 hours. After confirming the completion of the reaction, extraction was performed with CH₂Cl₂ and water, and the organic layer was dried over MgSO₄ and concentrated. The resulting compound was silica gel column and recrystallized to give a product 14.8 g (yield 60%).

### (4) Synthesis example of Sub 1-152-c

Sub 1"-152 (8.97 g, 14.9 mmol), *n*-BuLi (6 mL), Sub 1-152-b' (14.8 g, 14.9 mmol) obtained in the above synthesis was used to obtain a product 14.0 g (62% yield) using the synthesis method of Sub 1-1-c.

### (5) Synthesis example of Sub 1-152

Sub 1-152-c (14 g, 9.3 mmol) obtained in the above synthesis, acetic acid (23 mL), and concentrated hydrochloric acid (3.8 mL) were used to obtain 8.7 g (63% yield) of the product using the synthesis method of Sub 1-1.

Meanwhile, the compound belonging to Sub 1 may be the following compound, but is not limited thereto, and Table 1 shows FD-MS (Field Desorption-Mass Spectrometry) values of the compound belonging to Sub 1.

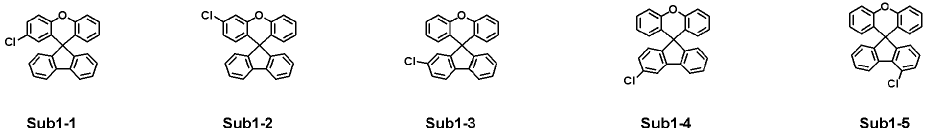

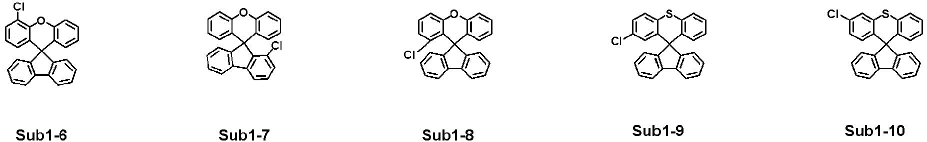

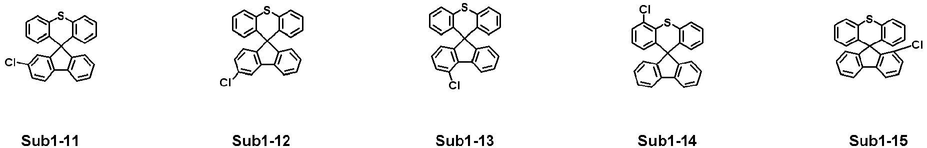

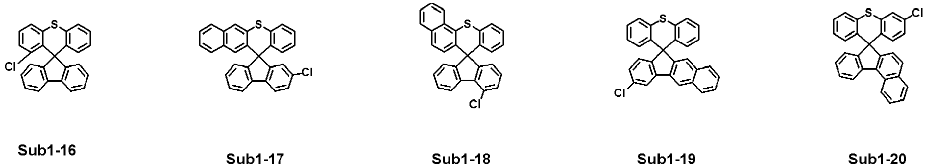

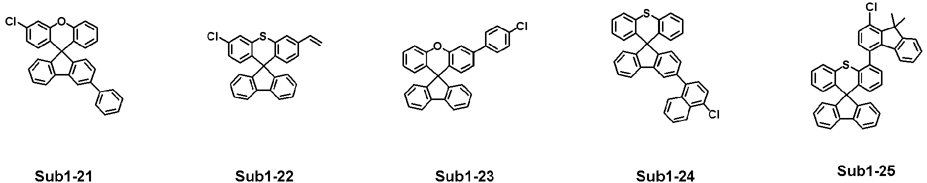

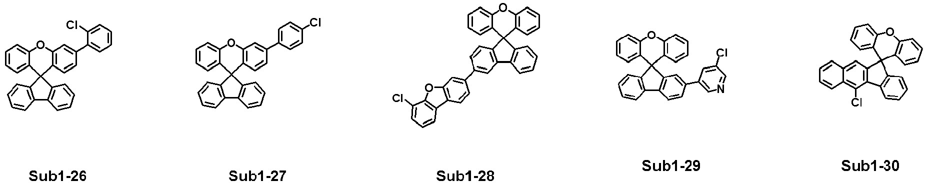

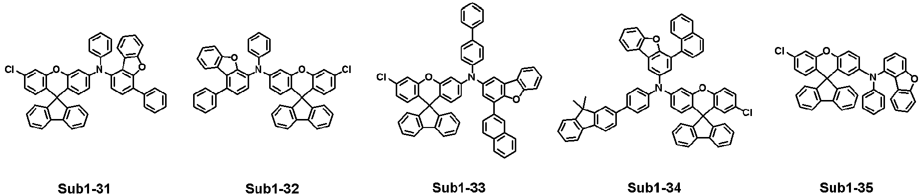

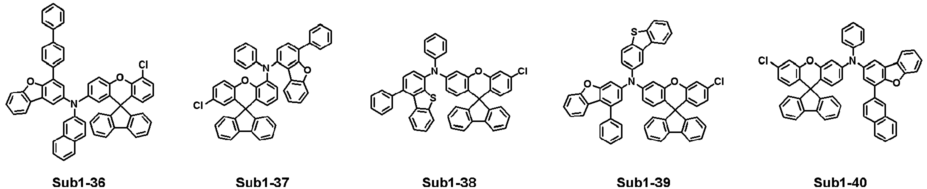

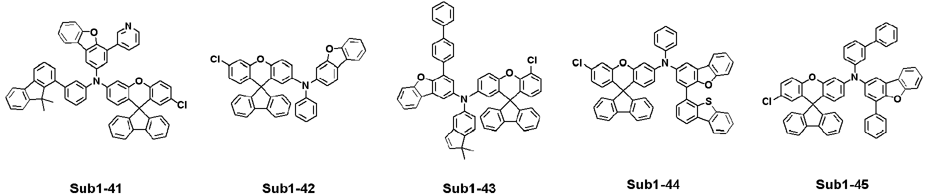

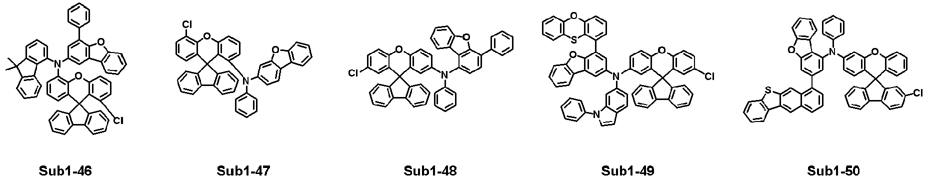

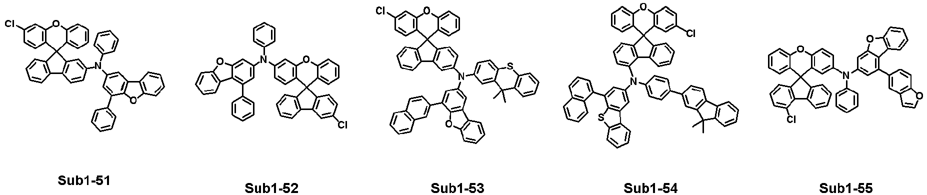

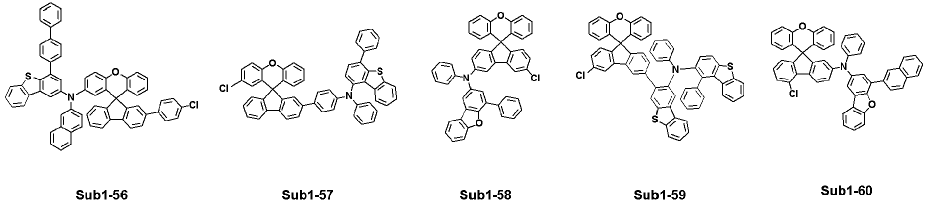

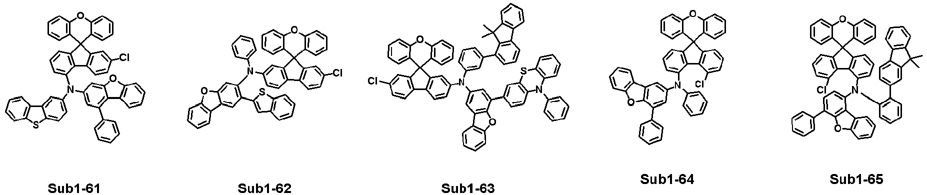

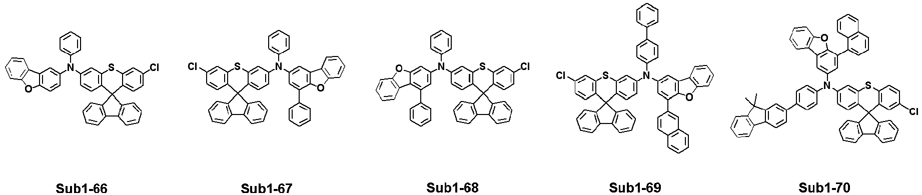

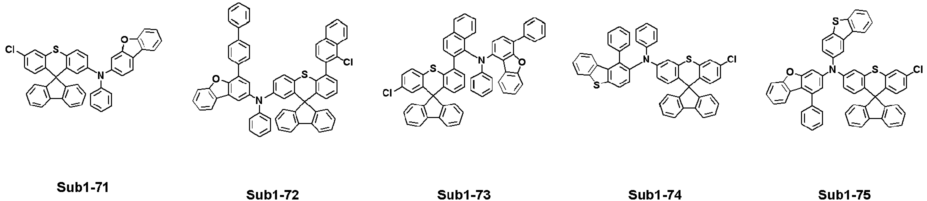

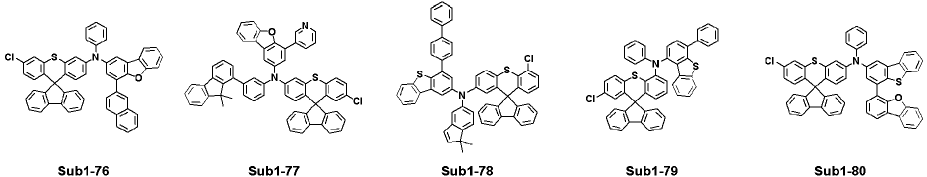

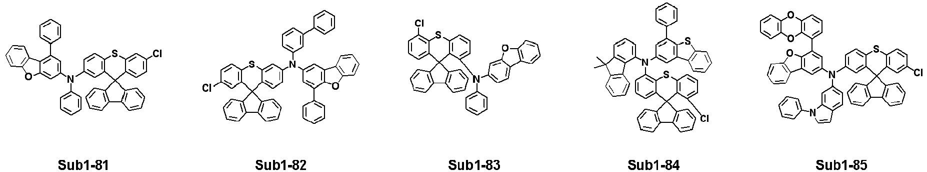

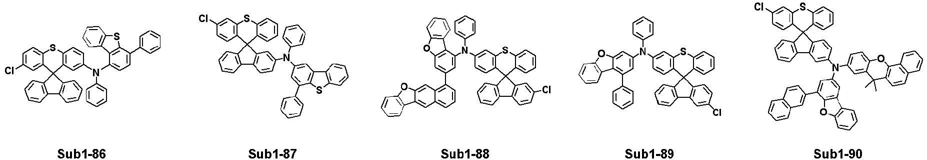

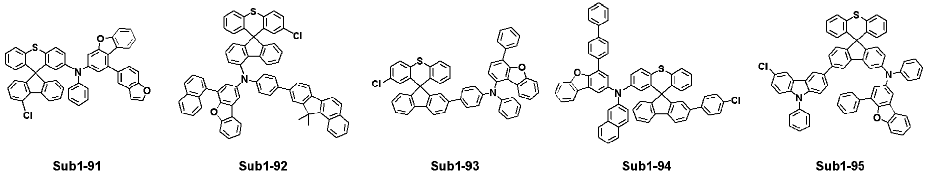

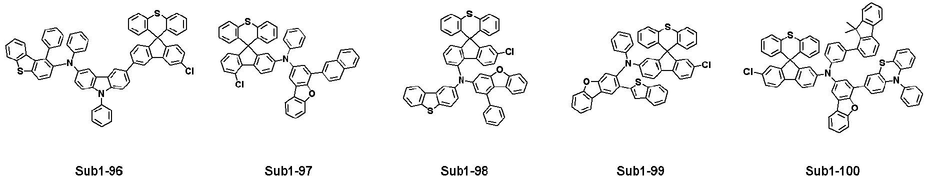

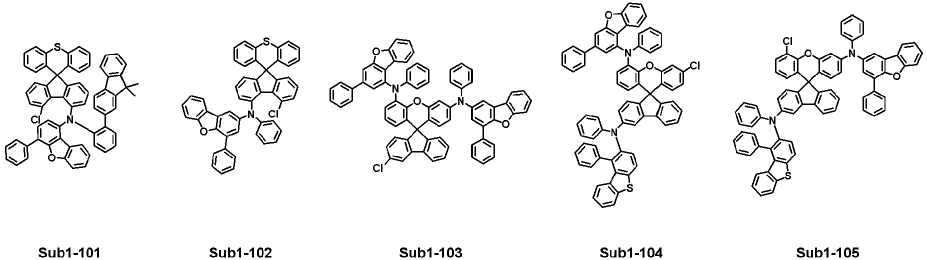

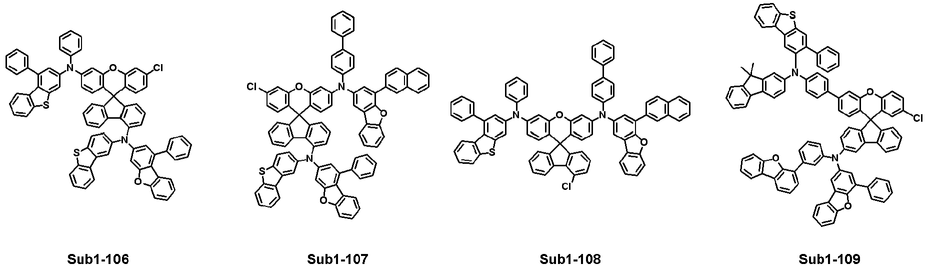

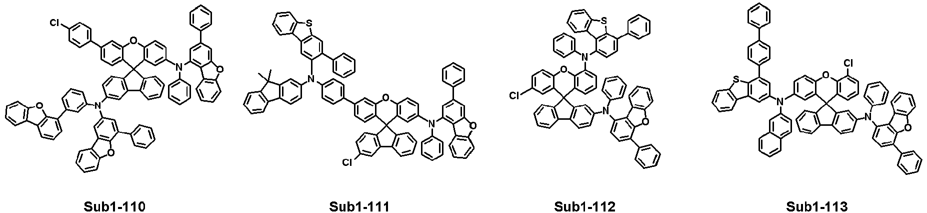

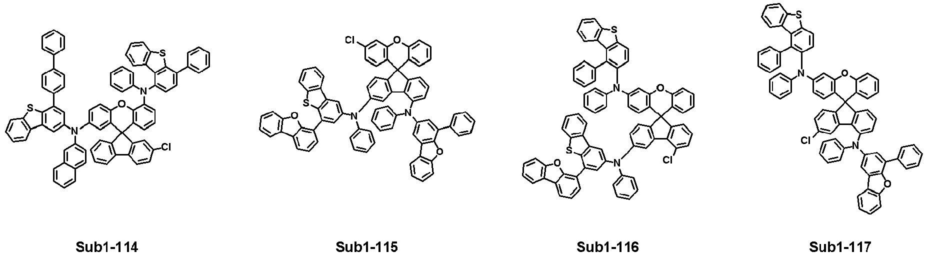

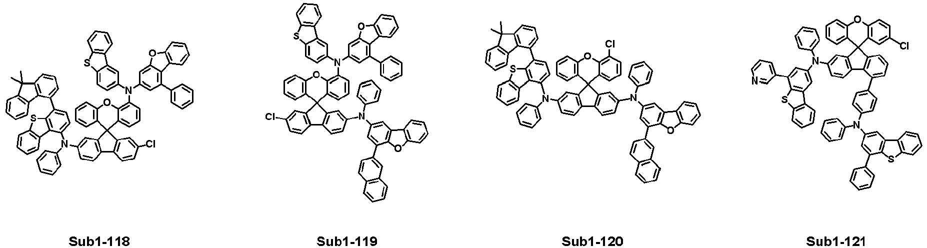

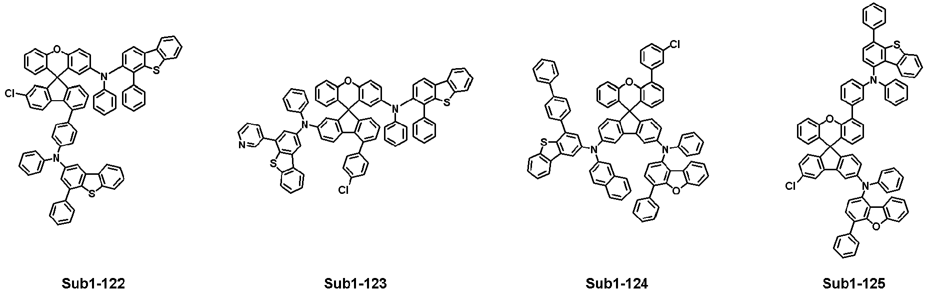

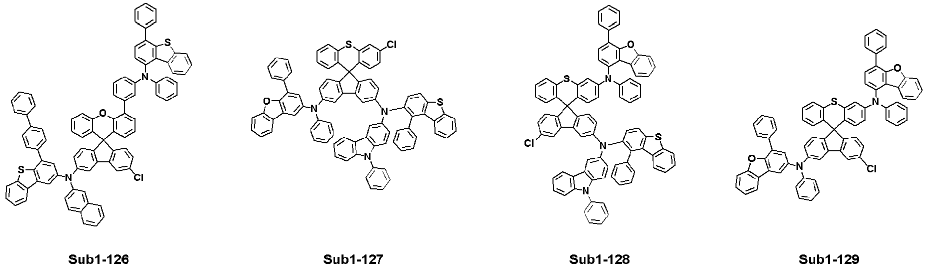

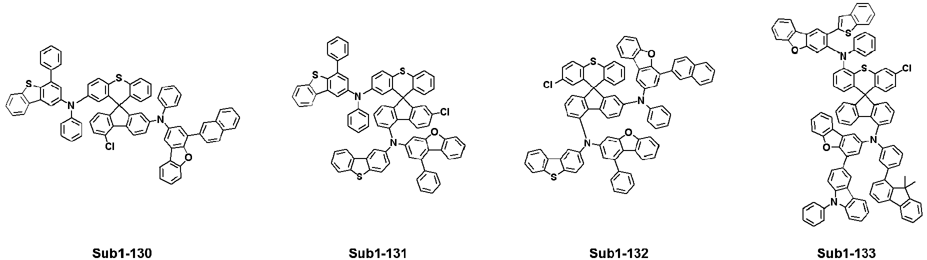

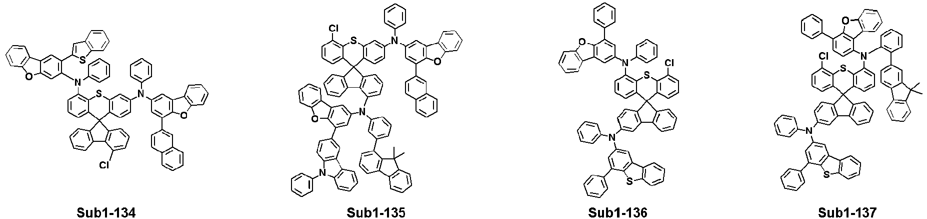

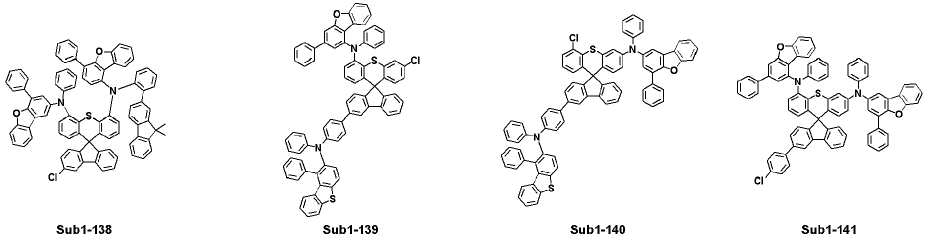

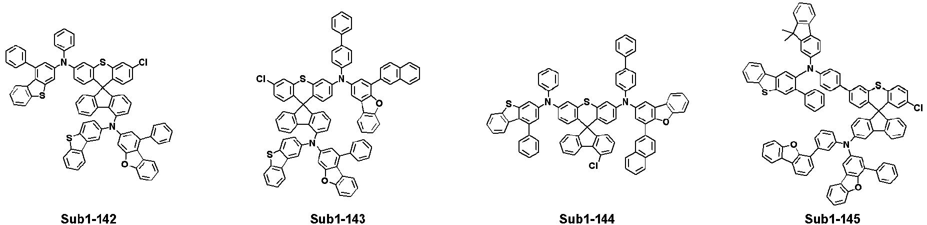

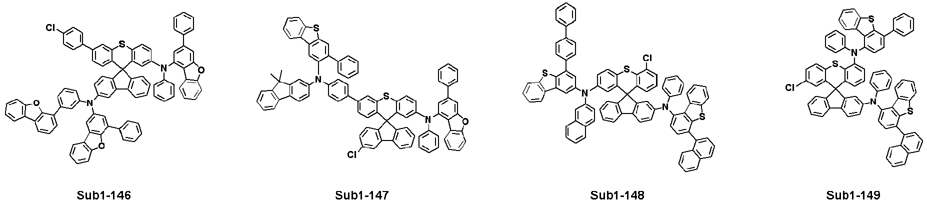

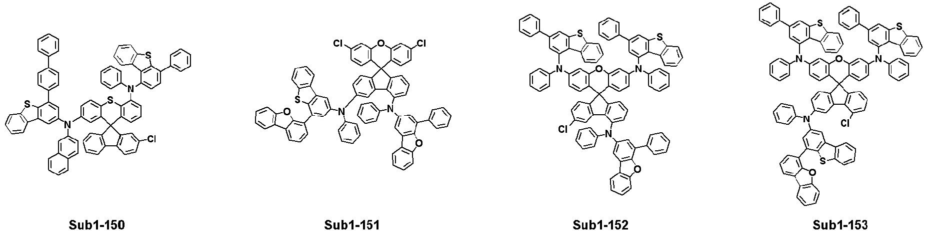

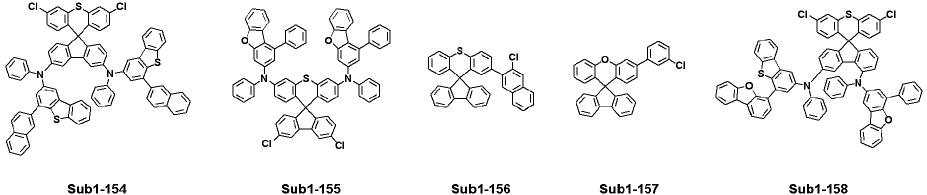

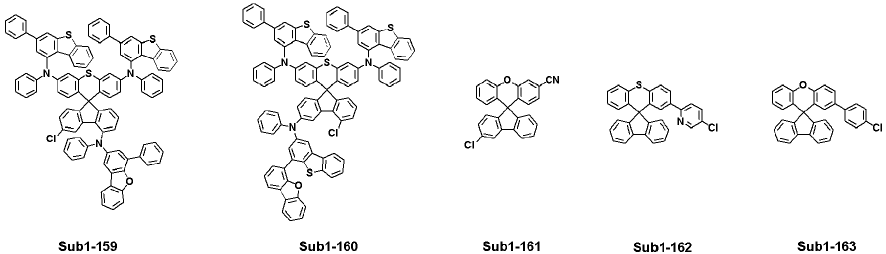

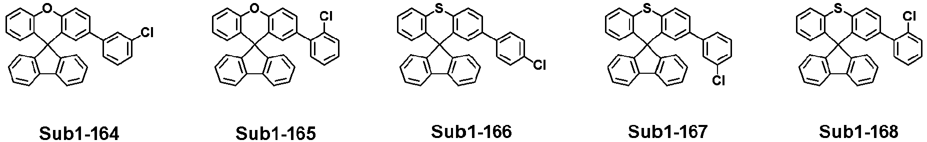

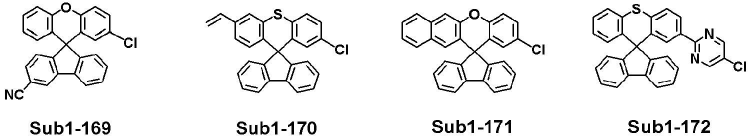

**[Table 1]**

| compound | FD-MS | compound | FD-MS |
|---|---|---|---|
| Sub1-1 | m/z=366.08(C₂₅H₁₅ClO=366.84) | Sub1-2 | m/z=366.08(C₂₅H₁₅ClO=366.84) |
| Sub1-3 | m/z=366.08(C₂₅H₁₅ClO=366.84) | Sub1-4 | m/z=366.08(C₂₅H₁₅ClO=366.84) |
| Sub1-5 | m/z=366.08(C₂₅H₁₅ClO=366.84) | Sub1-6 | m/z=366.08(C₂₅H₁₅ClO=366.84) |
| Sub1-7 | m/z=366.08(C₂₅H₁₅ClO=366.84) | Sub1-8 | m/z=366.08(C₂₅H₁₅ClO=366.84) |
| Sub1-9 | m/z=382.06(C₂₅H₁₅ClS=382.91) | Sub1-10 | m/z=382.06(C₂₅H₁₅ClS=382.91) |
| Sub1-11 | m/z=382.06(C₂₅H₁₅ClS=382.91) | Sub1-12 | m/z=382.06(C₂₅H₁₅ClS=382.91) |
| Sub1-13 | m/z=382.06(C₂₅H₁₅ClS=382.91) | Sub1-14 | m/z=382.06(C₂₅H₁₅ClS=382.91) |
| Sub1-15 | m/z=382.06(C₂₅H₁₅ClS=382.91) | Sub1-16 | m/z=382.06(C₂₅H₁₅ClS=382.91) |
| Sub1-17 | m/z=432.07(C₂₉H₁₇ClS=432.97) | Sub1-18 | m/z=432.07(C₂₉H₁₇ClS=432.97) |
| Sub1-19 | m/z=432.07(C₂₉H₁₇ClS=432.97) | Sub1-20 | m/z=432.07(C₂₉H₁₇ClS=432.97) |
| Sub1-21 | m/z=442.11(C₃₁H₁₉ClO=442.94) | Sub1-22 | m/z=408.07(C₂₇H₁₇ClS=408.94) |
| Sub1-23 | m/z=442.11(C₃₁H₁₉ClO=442.94) | Sub1-24 | m/z=508.11(C₃₅H₂₁ClS=509.06) |
| Sub1-25 | m/z=574.15(C₄₀H₂₇ClS=575.17) | Sub1-26 | m/z=442.11(C₃₁H₁₉ClO=442.94) |
| Sub1-27 | m/z=442.11(C₃₁H₁₉ClO=442.94) | Sub1-28 | m/z=532.12(C₃₇H₂₁ClO₂=533.02) |
| Sub1-29 | m/z=443.11(C₃₀H₁₈ClNO=443.93) | Sub1-30 | m/z=416.10(C₂₉H₁₇ClO=416.90) |
| Sub1-31 | m/z=699.20(C₄₉H₃₀ClNO₂=700.23) | Sub1-32 | m/z=699.20(C₄₉H₃₀ClNO₂=700.23) |
| Sub1-33 | m/z=825.24(C₅₉H₃₆ClNO₂=826.39) | Sub1-34 | m/z=941-31(C₆₈H₄₄ClNO₂=942.56) |
| Sub1-35 | m/z=623.17(C₄₃H₂₆ClNO₂=624.14) | Sub1-36 | m/z=825.24(C₅₉H₃₆ClNO₂=826.39) |
| Sub1-37 | m/z=699.20(C₄₉H₃₀ClNO₂=700.23) | Sub1-38 | m/z=715.17(C₄₉H₃₀ClNOS=716.3) |
| Sub1-39 | m/z=805.18(C₅₅H₃₂ClNO₂S=806.38) | Sub1-40 | m/z=749.21(C₅₃H₃₂ClNO₂=750.29) |
| Sub1-41 | m/z=892.29(C₆₃H₄₁ClN₂O₂=893.48) | Sub1-42 | m/z=623.17(C₄₃H₂₆ClNO₂=624.14) |
| Sub1-43 | m/z=841.27(C₆₀H₄₀ClNO₂=842.44) | Sub1-44 | m/z=805.18(C₅₅H₃₂ClNO₂S=806.38) |
| Sub1-45 | m/z=775.23(C₅₅H₃₄ClNO₂=776.33) | Sub1-46 | m/z=815.26(C₅₈H₃₈ClNO₂=816.40) |
| Sub1-47 | m/z=623.17(C₄₃H₂₆ClNO₂=624.14) | Sub1-48 | m/z=699.20(C₄₉H₃₀ClNO₂=700.23) |
| Sub1-49 | m/z=936.22(C₆₃H₃₇ClN₂O₃S=937.51) | Sub1-50 | m/z=855.20(C₅₉H₃₄ClNO₂S=856.44) |
| Sub1-51 | m/z=699.20(C₄₉H₃₀ClNO₂=700.23) | Sub1-52 | m/z=699.20(C₄₉H₃₀ClNO₂=700.23) |
| Sub1-53 | m/z=897.25(C₆₂H₄₀ClNO₂S=898.52) | Sub1-54 | m/z=957.28(C₆₈H₄₄ClNOS=958.62) |
| Sub1-55 | m/z=739.19(C₅₁H₃₀ClNO₃=740.26) | Sub1-56 | m/z=917.25(C₆₅H₄₀ClNOS=918.55) |
| Sub1-57 | m/z=791.20(C₅₅H₃₄ClNOS=792.39) | Sub1-58 | m/z=699.20(C₄₉H₃₀ClNO₂=700.23) |
| Sub1-59 | m/z=897.19(C₆₁H₃₆ClNOS₂=898.54) | Sub1-60 | m/z=749.21(C₅₃H₃₂ClNO₂=750.29) |
| Sub1-61 | m/z=805.18(C₅₅H₃₂ClNO₂S=806.38) | Sub1-62 | m/z=755.17(C₅₁H₃₀ClNO₂S=756.32) |
| Sub1-63 | m/z=1088.32(C₇₆H₄₉ClN₂O₂S=1089.75) | Sub1-64 | m/z=699.20(C₄₉H₃₀ClNO₂=700.23) |
| Sub1-65 | m/z=891.29(C₆₄H₄₂ClNO₂=892.50) | Sub1-66 | m/z=639.14(C₄₃H₂₆ClNOS=640.20) |
| Sub1-67 | m/z=715.17(C₄₉H₃₀ClNOS=716.30) | Sub1-68 | m/z=715.17(C₄₉H₃₀ClNOS=716.3) |
| Sub1-69 | m/z=841.22(C₅₉H₃₆ClNOS=842.45) | Sub1-70 | m/z=957.28(C₆₈H₄₄ClNOS=958.62) |
| Sub1-71 | m/z=639.14(C₄₃H₂₆ClNOS=640.20) | Sub1-72 | m/z=917.25(C₆₅H₄₀ClNOS=918.55) |
| Sub1-73 | m/z=841.22(C₅₉H₃₆ClNOS=842.45) | Sub1-74 | m/z=731.15(C₄₉H₃₀ClNS₂=732.36) |
| Sub1-75 | m/z=821.16(C₅₅H₃₂ClNOS₂=822.44) | Sub1-76 | m/z=765.19(C₅₃H₃₂ClNOS=766.36) |
| Sub1-77 | m/z=908.26(C₆₃H₄₁ClN₂OS=909.54) | Sub1-78 | m/z=873.23(C₆₀H₄₀ClNS₂=874.56) |
| Sub1-79 | m/z=731.15(C₄₉H₃₀ClNS₂=732.36) | Sub1-80 | m/z=821.16(C₅₅H₃₂ClNOS₂=822.44) |
| Sub1-81 | m/z=715.17(C₄₀H₃₀ClNOS=716.30) | Sub1-82 | m/z=791.20(C₅₅H₃₄ClNOS=792.39) |
| Sub1-83 | m/z=639.14(C₄₃H₂₆ClNOS=640.20) | Sub1-84 | m/z=847.21(C₅₈H₃₈ClNS₂=848.52) |
| Sub1-85 | m/z=936.22(C₆₃H₃₇ClN₂O₃S=937.51) | Sub1-86 | m/z=731.15(C₄₉H₃₀ClNS₂=732.36) |
| Sub1-87 | m/z=731.15(C₄₉H₃₀ClNS₂=732.36) | Sub1-88 | m/z=855.20(C₅₉H₃₄ClNO₂S=856.44) |
| Sub1-89 | m/z=715.17(C₄₀H₃₀ClNOS=716.30) | Sub1-90 | m/z=947.26(C₆₆H₄₂ClNO₂S=948.58) |
| Sub1-91 | m/z=755.17(C₅₁H₃₀ClNO₂S=756.32) | Sub1-92 | m/z=1007.30(C₇₂H₄₆ClNOS=1008.68) |
| Sub1-93 | m/z=791.20(C₅₅H₃₄ClNOS=792.39) | Sub1-94 | m/z=917.25(C₆₅H₄₀ClNOS=918.55) |
| Sub1-95 | m/z=956.26(C₆₇H₄₁ClN₂OS=957.59) | Sub1-96 | m/z=972.24(C₆₇H₄₁ClN₂S₂=973.65) |
| Sub1-97 | m/z=765.19(C₅₃H₃₂ClNOS=766.36) | Sub1-98 | m/z=821.16(C₅₅H₃₂ClNOS₂=822.44) |
| Sub1-99 | m/z=771.15(C₅₁H₃₀ClNOS₂=772.38) | Sub1-100 | m/z=1104.30(C₇₆H₄₉ClN₂OS₂=1105.81) |
| Sub1-101 | m/z=907.27(C₆₄H₄₂ClNOS=908.56) | Sub1-102 | m/z=715.17(C₄₉H₃₀ClNOS=716.03) |
| Sub1-103 | m/z=1032.31(C₇₃H₄₅ClN₂O₃=1033.62) | Sub1-104 | m/z=1048.29(C₇₃H₄₅ClN₂O₂S=1049.69) |
| Sub1-105 | m/z=1048.29(C₇₃H₄₅ClN₂O₂S=1049.69) | Sub1-106 | m/z=1154.28(C₇₉H₄₇ClN₂O₂S₂=1155.83) |
| Sub1-107 | m/z=1264.35(C₈₉H₅₃ClN₂O₃S=1265.92) | Sub1-108 | m/z=1174.34(C₈₃H₅₁ClN₂O₂S=1175.84) |
| Sub1-109 | m/z=1406.42(C₁₀₀H₆₃ClN₂O₃S=1408.13) | Sub1-110 | m/z=1274.39(C₉₁H₅₅ClN₂O₄=1275.90) |
| Sub1-111 | m/z=1240.38(C₈₈H₅₇ClN₂O₂S=1241.95) | Sub1-112 | m/z=1048.29(C₇₃H₄₅ClN₂O₂S=1049.69) |
| Sub1-113 | m/z=1174.34(C₈₃H₅₁ClN₂O₂S=1175.84) | Sub1-114 | m/z=1190.31(C₈₃H₅₁ClN₂OS₂=1191.90) |
| Sub1-115 | m/z=1138.30(C₇₉H₄₇ClN₂O₃S=1139.77) | Sub1-116 | m/z=1154. 28(C₇₉H₄₇ClN₂O₂S₂=1155.83) |
| Sub1-117 | m/z=1048.29(C₇₃H₄₅ClN₂O₂S=1049.69) | Sub1-118 | m/z=1270.34(C₈₈H₅₅ClN₂O₂S₂=1271.99) |
| Sub1-119 | m/z=1188.32(C₈₃H₄₉ClN₂O₃S=1189.83) | Sub1-120 | m/z=1214.37(C₈₆H₅₅ClN₂O₂S=1215.91) |
| Sub1-121 | m/z=1141.29(C₇₈H₄₈ClN₃OS₂=1142.83) | Sub1-122 | m/z=1140.30(C₇₉H₄₉ClN₂OS₂=1141.84) |
| Sub1-123 | m/z=1141.29(C₇₈H₄₈ClN₃OS₂=1142.83) | Sub1-124 | m/z=1250.37(C₈₉H₅₅ClN₂O₂S=1251.94) |
| Sub1-125 | m/z=1124.32(C₇₉H₄₉ClN₂O₂S=1125.78) | Sub1-126 | m/z=1266.34(C₈₉H₅₅ClN₂OS₂=1268.00) |
| Sub1-127 | m/z=1229.32(C₈₅H₅₂ClN₃OS₂=1230.94) | Sub1-128 | m/z=1229.32(C₈₅H₅₂ClN₃OS₂=1230.94) |
| Sub1-129 | m/z=1048.29(C₇₃H₄₅ClN₂O₂S=1049.69) | Sub1-130 | m/z=1114.28(C₇₇H₄₇ClN₂OS₂=1115.81) |
| Sub1-131 | m/z=1170.25(C₇₉H₄₇ClN₂OS₃=1171.89) | Sub1-132 | m/z=1204. 29(C₈₃H₄₉ClN₂O₂S₂=1205.89) |
| Sub1-133 | m/z=1477.44(C₁₀₃H₆₈ClN₂O₂S₂=1479.27) | Sub1-134 | m/z=1154. 28(C₇₉H₄₇ClN₂O₂S₂=1155.83) |
| Sub1-135 | m/z=1471.49(C₁₀₅H₇₀ClN₃O₂S=1473.24) | Sub1-136 | m/z=1064.27(C₇₃H₄₅ClN₂OS₂=1065.75) |
| Sub1-137 | m/z=1256.36(C₈₈H₅₇ClN₂OS₂=1258.01) | Sub1-138 | m/z=1240.38(C₈₈H₅₇ClN₂O₂S=1241.95) |
| Sub1-139 | m/z=1140.30(C₇₉H₄₉ClN₂OS₂=1141.84) | Sub1-140 | m/z=1140.30(C₇₉H₄₉ClN₂OS₂=1141.84) |
| Sub1-141 | m/z=1124.32(C₇₉H₄₉ClN₂O₂S=1125.78) | Sub1-142 | m/z=1170.25(C₇₉H₄₇ClN₂OS₃=1171.89) |
| Sub1-143 | m/z=1280.32(C₈₉H₅₃ClN₂O₂S₂=1281.99) | Sub1-144 | m/z=1190.31(C₈₃H₅₁ClN₂OS₂=1191.90) |
| Sub1-145 | m/z=1422.40(C₁₀₀H₆₃ClN₂O₂S₂=1424.19) | Sub1-146 | m/z=1290.36(C₉₁H₅₅ClN₂O₃S=1291.96) |
| Sub1-147 | m/z=1256.36(C₈₈H₅₇ClN₂OS₂=1258.01) | Sub1-148 | m/z=1256.31(C₈₇H₅₃ClN₂S₃=1258.03) |
| Sub1-149 | m/z=1130.26(C₇₇H₄₇ClN₂S₃=1131.87) | Sub1-150 | m/z=1206.29(C₈₃H₅₁ClN₂S₃=1207.97) |
| Sub1-151 | m/z=1236.39(C₈₃H₆₂Cl₂N₂O₃S=1238.38) | Sub1-152 | m/z=1493.57(C₁₀₃H₈₄ClN₃O₂S₂=1495.39) |
| Sub1-153 | m/z=1599. 56(C₁₀₉H₈₆ClN₃O₂S₃=1601.54) | Sub1-154 | m/z=1214.24(C₈₁H₄₈Cl₂N₂S₃=1216.37) |
| Sub1-155 | m/z=1082.25(C₇₃H₄₄Cl₂N₂O₂S=1084.13) | Sub1-156 | m/z=508.11(C₃₅H₂₁ClS=509.06) |
| Sub1-157 | m/z=442.11(C₃₁H₁₉ClO=442.94) | Sub1-158 | m/z=1252.36(C₈₃H₆₂Cl₂N₂O₂S₂=1254.44) |
| Sub1-159 | m/z=1509 55(C₁₀₃H₈₄ClN₃OS₃=1511.46) | Sub1-160 | m/z=1615. 53(C₁₀₉H₈₆ClN₃OS₄=1617.60) |
| Sub1-161 | m/z=391.08(C₂₆H₁₄ClNO=391.85) | Sub1-162 | m/z=459.08(C₃₀H₁₈ClNS=459.99) |
| Sub1-163 | m/z=442.11(C₃₁H₁₉ClO=442.94) | Sub1-164 | m/z=442.11(C₃₁H₁₉ClO=442.94) |
| Sub1-165 | m/z=442.11(C₃₁H₁₉ClO=442.94) | Sub1-166 | m/z=458.09(C₃₁H₁₉ClS=459.00) |
| Sub1-167 | m/z=458.09(C₃₁H₁₉C₁₅=453.00) | Sub1-168 | m/z=458.09(C₃₁H₁₉ClS=459.00) |
| Sub1-169 | m/z=391.08(C₂₆HuClNO=391.85) | Sub1-170 | m/z=408.07(C₂₇H₁₇C1S=408.94) |
| Sub1-171 | m/z=416.10(C₂₉H₁₇ClO=416.90) | Sub1-172 | m/z=460.08(C₂₉H₁₇ClN₂S=460.98) |

### II. Synthesis of Sub 2

Sub 2 of Reaction Scheme 1 may be synthesized by the reaction route of Scheme 3, but is not limited thereto. Sub 3 and Sub 4 are also included in Sub 2.

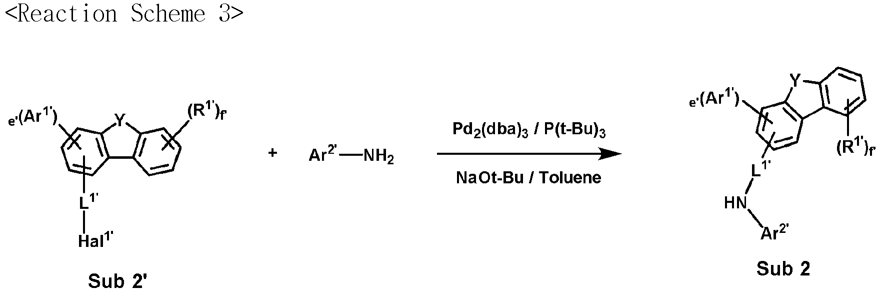

In Reaction Scheme 3,
Y, Ar¹', Ar²' , R¹', L¹', e' and f' are the same as defined above, Hal¹' is Br or Cl.

Synthesis examples of specific compounds belonging to Sub 2 are as follows.

### 1. Synthesis example of Sub 2-8

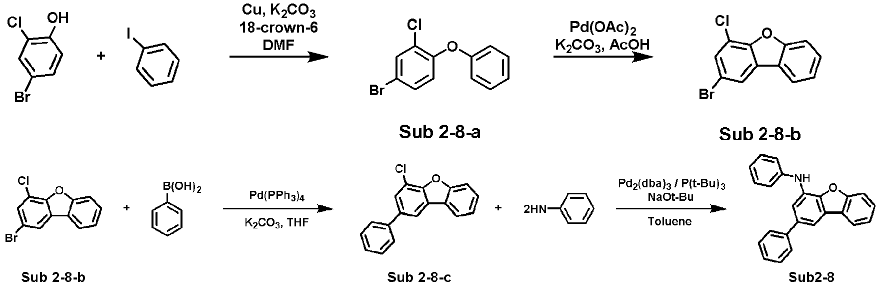

### (1) Synthesis example of Sub 2-8-a

4-Bromo-2-chlorophenol (15 g, 73.5 mmol) was dissolved in a round bottom flask with DMF (400 mL) and iodobenzene (16.8 g, 80.9 mmol), K₂CO₃ (20.3 g 147.1 mmol), Cu (2.3 g, 36.8 mmol), dibenzo-18-crown-6 (1.6 g, 4.4 mmol) were added and stirred at 120° C. When the reaction was completed, the solvent was removed, extracted with CH₂Cl₂ and water, the organic layer was dried over MgSO₄, concentrated, and the resulting compound was silica gel column to obtain 16.5 g (yield 79%) of the product.

### (2) Synthesis example of Sub 2-8-b

Acetic acid (400 mL) was added to 4-bromo-2-chloro-1-phenoxybenzene (16 g, 56.4 mmol), Pd(OAc)₂ (0.63 g, 2.8 mol), K₂CO₃ (7.8 g, 56.4 mol) and stirred at 120° C for 48 hours. When the reaction was completed, the solvent was removed, extracted with ethyl acetate and water, the organic layer was dried over MgSO₄, concentrated, and the resulting compound was silica gel column to obtain 6.7 g (yield 42%) of the product.

### (3) Synthesis example of Sub 2-8-c

Sub 2-8-b (6.7 g, 23.8 mmol) obtained in the above synthesis, phenylboronic acid (2.9 g, 23.8 mmol) was added, dissolved in THF (100 mL), and Pd(PPh₃)₄ (1.4 g, 1.2 mmol) and K₂CO₃ (19.9 g, 71.4 mmol) were added, respectively, and then refluxed for 24 hours. When the reaction was completed, the solvent was removed, extracted with CH₂Cl₂ and water, the organic layer was dried over MgSO₄, concentrated, and the resulting compound was silica gel column to obtain 4.98 g (yield 75%) of the product.

### (4) Synthesis example of Sub 2-8

After dissolving Sub 2-8-c (4.98 g, 17.9 mmol) obtained in the above synthesis with toluene (100 ml) in a round bottom flask, aniline (1.83 g, 19.7 mmol), Pd₂(dba)₃ (0.49 g, 0.54 mmol), P(*t*-Bu)₃ (50wt% Sol.) (0.43 mL, 1.07 mmol), NaOt-Bu (5.2 g, 53.6 mmol) were added and stirred at 80° C. When the reaction was completed, the solvent was removed, extracted with CH₂Cl₂ and water, the organic layer was dried over MgSO₄, concentrated, and the resulting compound was silica gel column to obtain 4.8 g (yield 80%) of the product.

### 2. Synthesis example of Sub 2-43

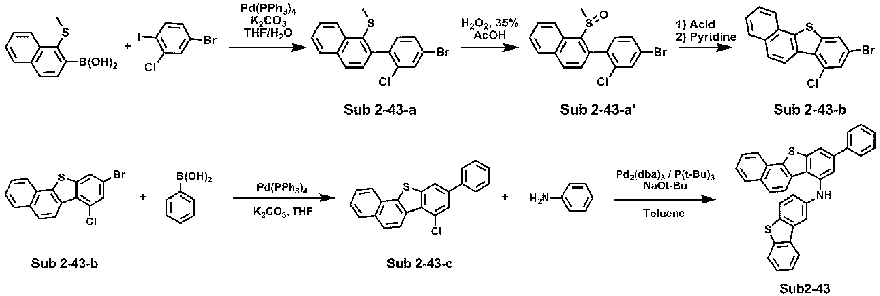

### (1) Synthesis example of Sub 2-43-a

(2-(Methylthio)phenyl)boronic acid (30 g, 137.6 mmol) was dissolved in a round bottom flask with THF (300 mL) and 4-bromo-2-chloroiodobenzene (43.7g, 137.6 mmol), Pd(PPh₃)₄ (4.8 g, 4.1 mmol), K₂CO₃ (57 g, 412.7 mmol), and water (100 mL) were added and stirred at 80° C. When the reaction was completed, the solvent was removed, extracted with CH₂Cl₂ and water, the organic layer was dried over MgSO₄, concentrated, and the resulting compound was silicagel column to obtain 37.5 g (yield 75%) of the product.

### (2) Synthesis example of Sub 2-43-a'

Sub 2-43-a (37 g, 101.7 mmol) obtained in the above synthesis was dissolved in AcOH (300 mL) in a round bottom flask, H₂O₂ (8.7 mL, 101.7 mmol) was added and stirred at room temperature. When the reaction was completed, the solvent was removed, neutralized with 1M NaOH, extracted with ethyl acetate, and recrystallized to obtain 35.5 g (92% yield) of the product.

### (3) Synthesis example of Sub 2-43-b

Sub 2-43-a' (35 g, 92.2 mmol) obtained in the above synthesis was added to 300 g of trifluoromethanesulfonic acid and stirred at 65° C. When the reaction was completed, pyridine was added and refluxed for 30 minutes, and the resulting compound was recrystallized in a silica gel column to obtain 26 g (yield 81%) of the product.

### (4) Synthesis example of Sub 2-43-c

Sub 2-43-b (26 g, 74.8 mmol) obtained in the above synthesis, phenylboronic acid (9.12 g, 74.8 mmol), Pd(PPh₃)₄ (4.32 g, 3.7 mmol), K₂CO₃ (62.54 g, 224.4 mmol) was added to THF (180 mL), and by using the synthesis method of Sub 2-8-c, the product 20.1 g (78% yield) was obtained.

### (5) Synthesis example of Sub 2-43

Sub 2-43-c (20.1 g, 58.3 mmol) obtained in the above synthesis was dissolved in a round bottom flask with toluene (400 ml), and aniline (6 g, 64.1 mmol), Pd₂(dba)₃ (1.6 g, 1.75 mmol), P(*t*-Bu)₃ (50wt% Sol.) (1.42 mL, 3.5 mmol), NaOt-Bu (16.8 g, 174.85 mmol) were added, and product 23.4 g (yield 79%) was obtained using the synthesis method of Sub 2-8.

Meanwhile, the compound belonging to Sub 2 may be the following compound, but is not limited thereto. Sub 3 and Sub 4 are also compounds belonging to Sub 2, and Table 2 shows the FD-MS values of the compounds belonging to Sub 2.

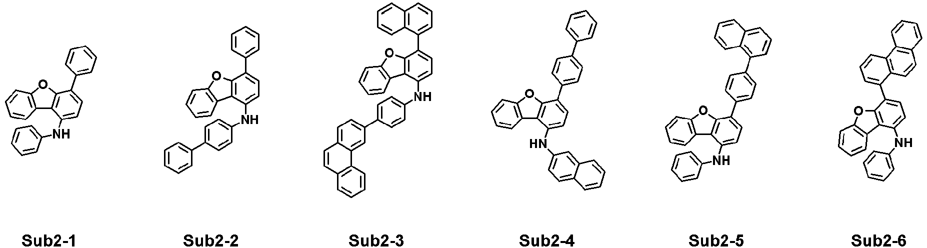

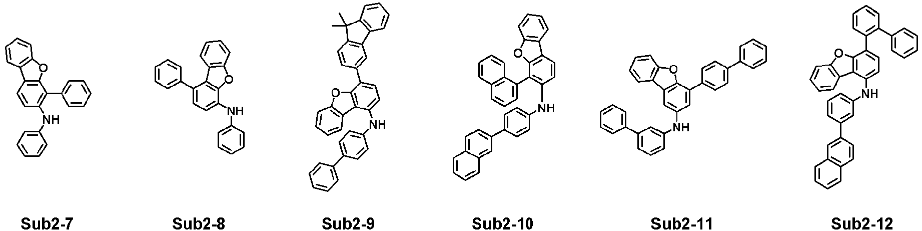

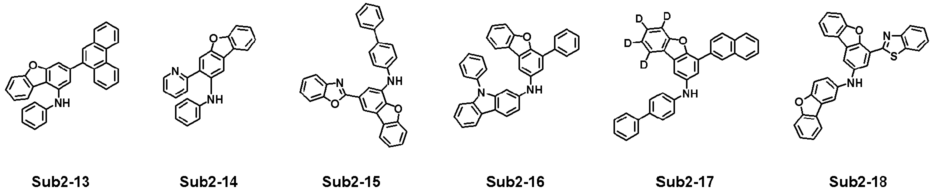

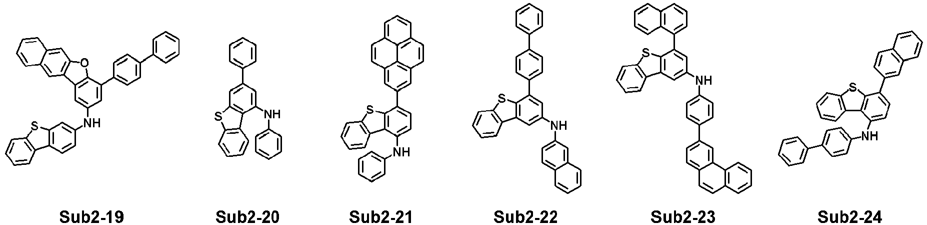

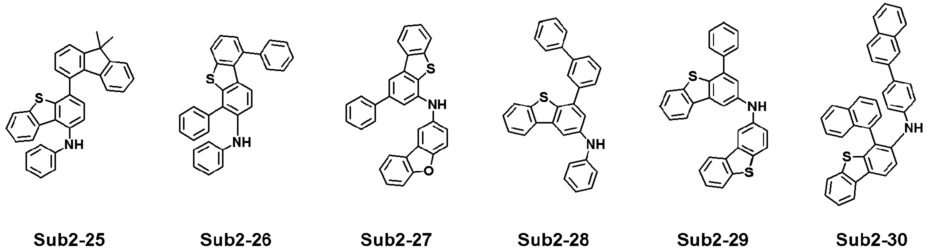

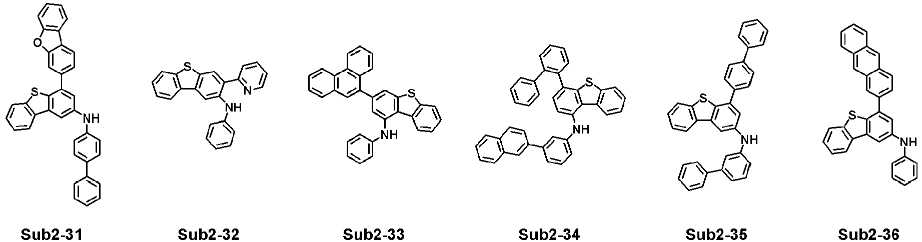

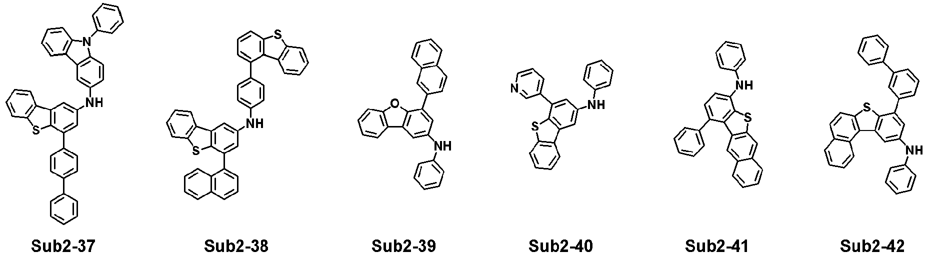

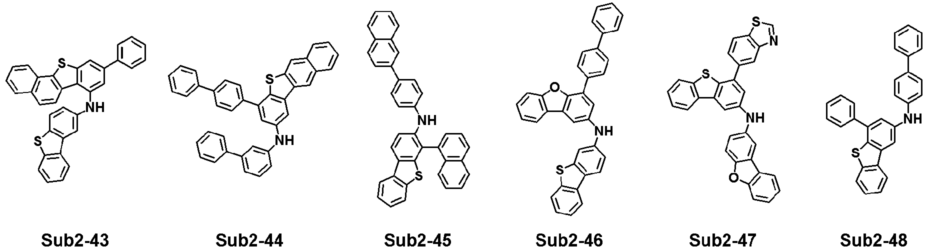

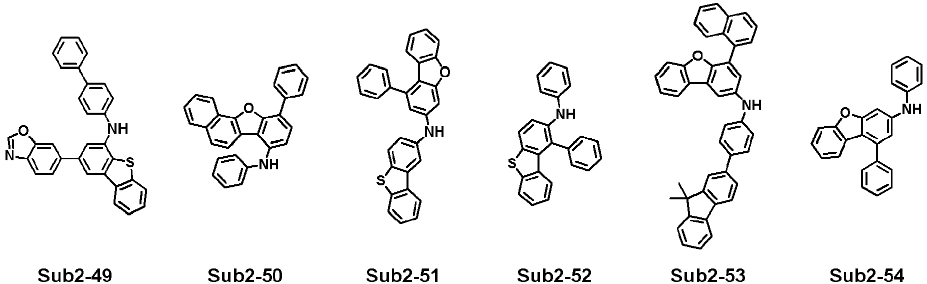

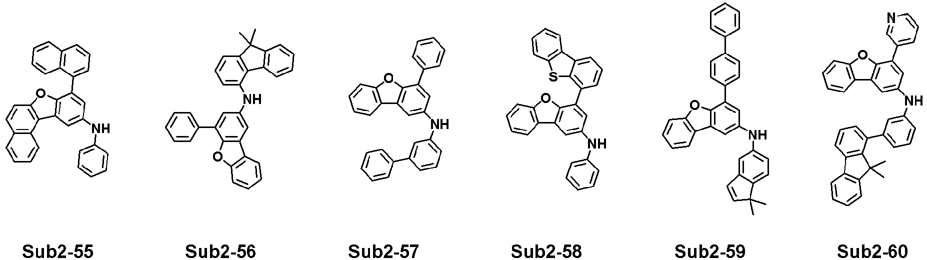

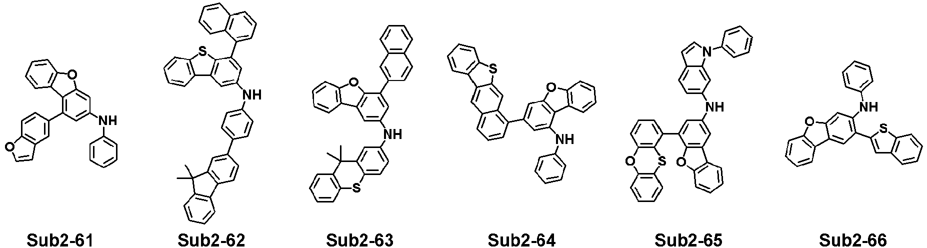

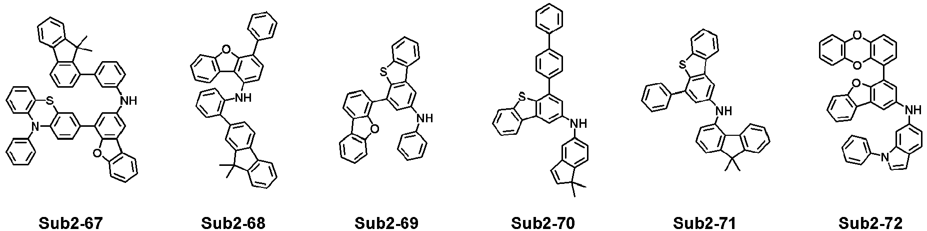

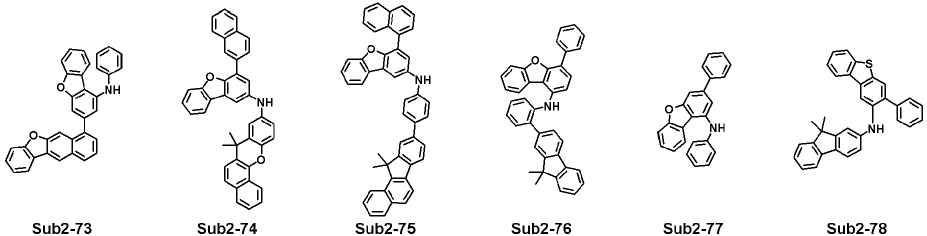

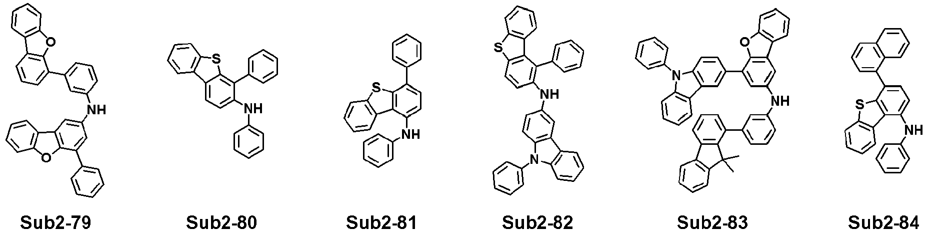

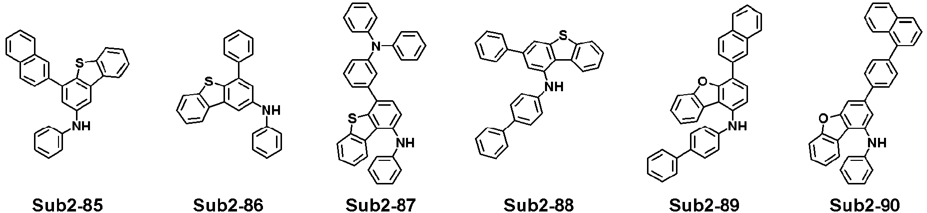

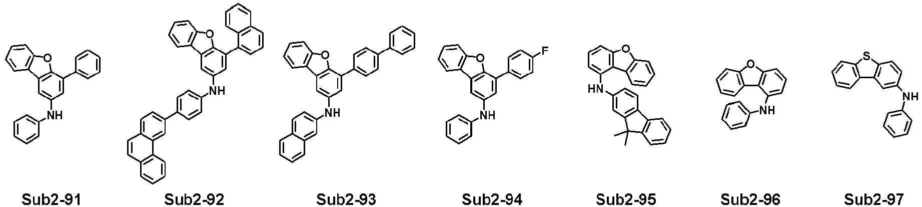

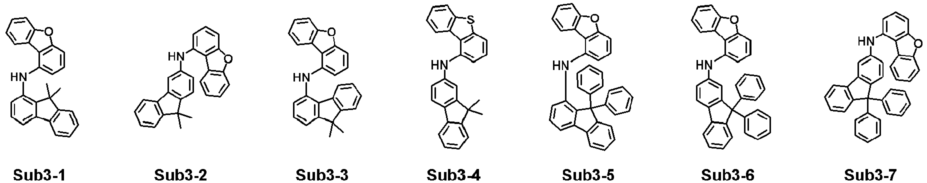

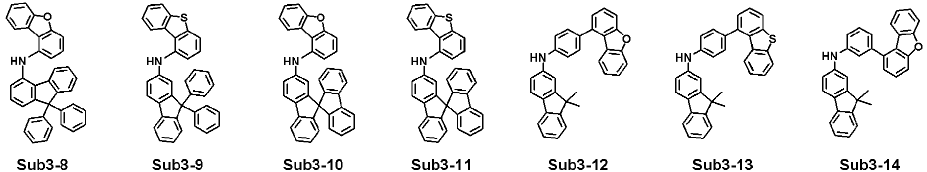

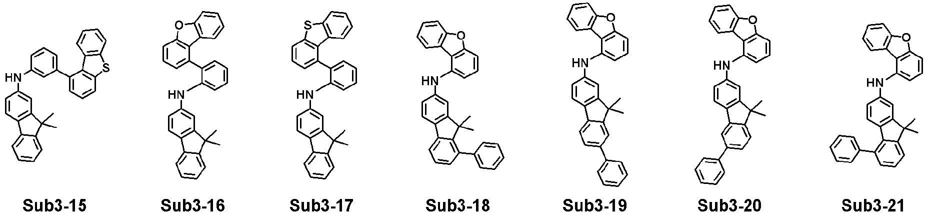

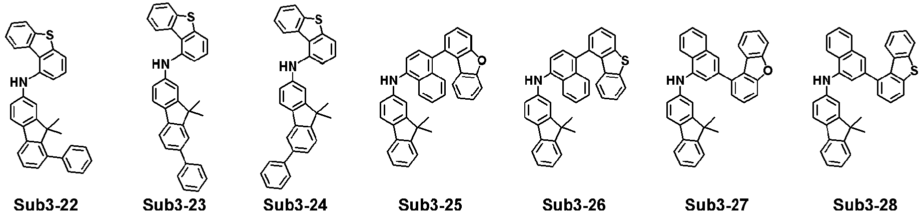

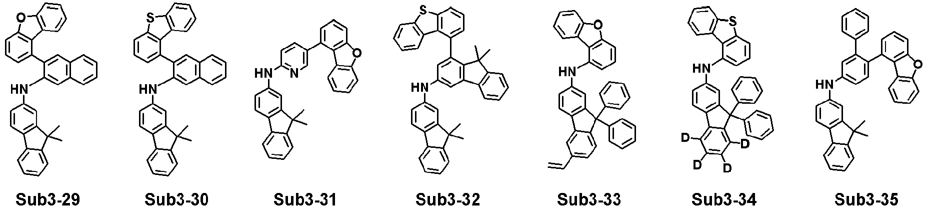

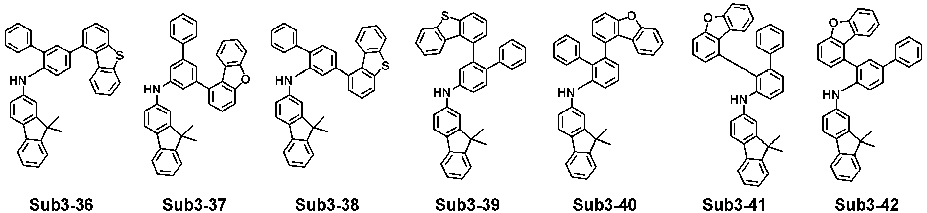

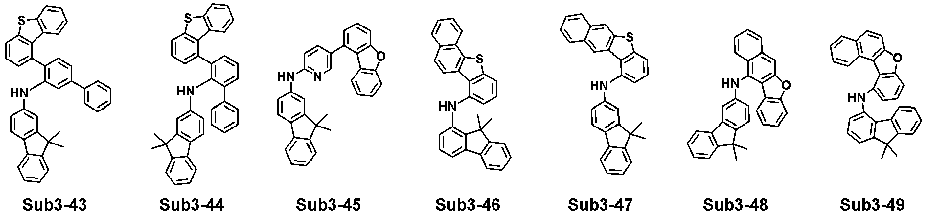

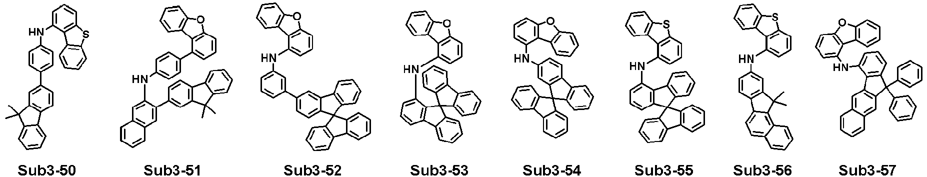

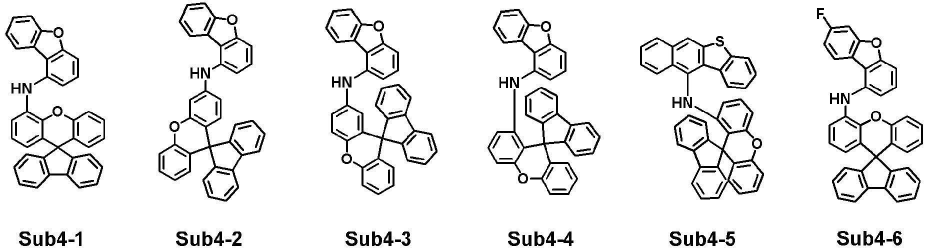

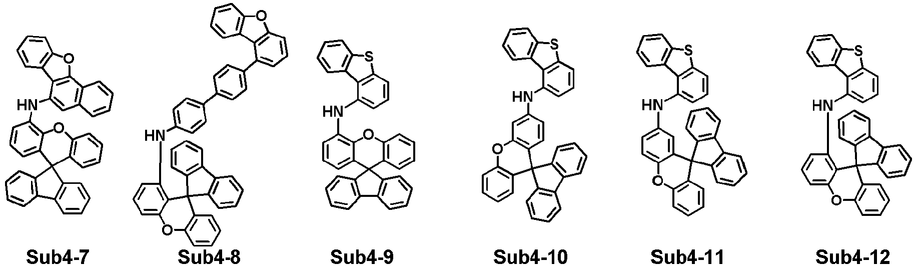

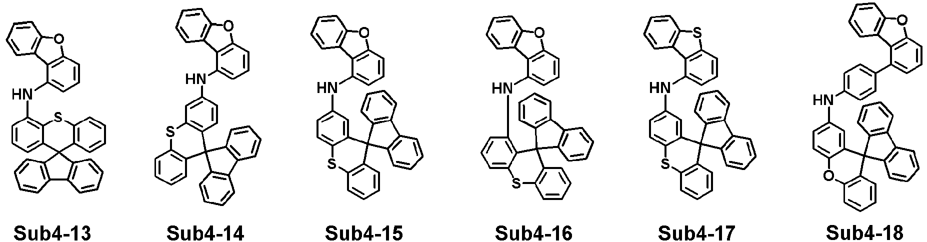

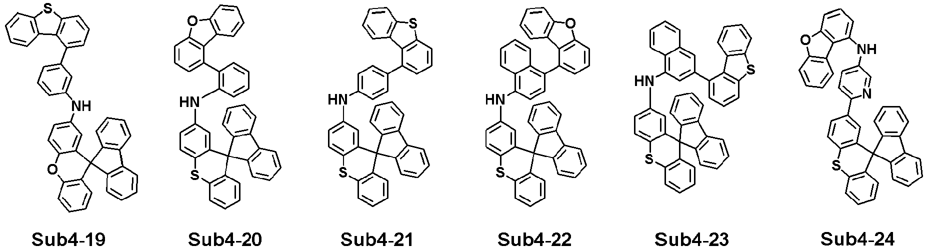

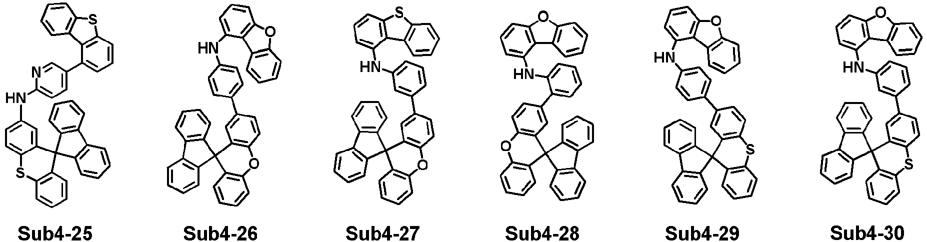

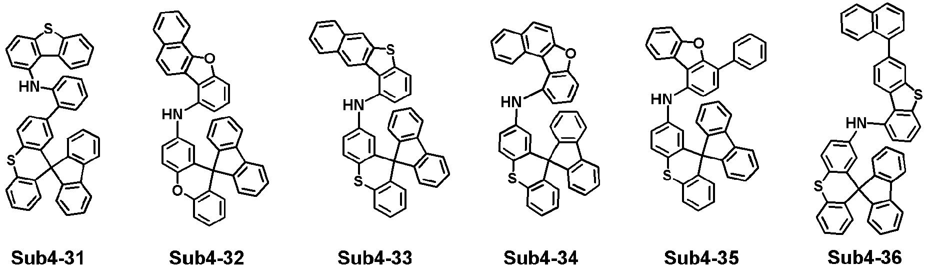

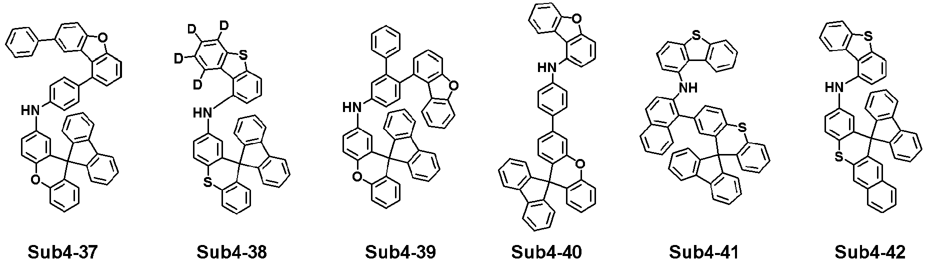

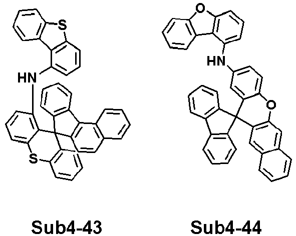

**[Table 2]**

| compound | FD-MS | compound | FD-MS |
|---|---|---|---|
| Sub2-1 | m/z=335.13(C₂₄H₁₇NO=335.41) | Sub2-2 | m/z=427.19(C₃₁H₂₅NO=427.55) |
| Sub2-3 | m/z=561.21(C₄₂H₂₇NO=561.68) | Sub2-4 | m/z=461.18(C₃₄H₂₃NO=461.56) |
| Sub2-5 | m/z=461.18(C₃₄H₂₃NO=461.56) | Sub2-6 | m/z=435.16(C₂₄H₁₇NO=435.53) |
| Sub2-7 | m/z=335.13(C₂₄H₁₇NO=335.41) | Sub2-8 | m/z=335.13(C₃₂H₂₁NO=335.41) |
| Sub2-9 | m/z=527.22(C₃₉H₂₉NO=527.67) | Sub2-10 | m/z=511.19(C₃₈H₂₅NO=511.62) |
| Sub2-11 | m/z=487.19(C₃₆H₂₅NO=487.60) | Sub2-12 | m/z=537.21(C₄₀H₂₇NO=537.66) |
| Sub2-13 | m/z=435.16(C₃₂H₂₁NO=435.53) | Sub2-14 | m/z=336.13(C₂₃H₁₆N₂O=336.39) |
| Sub2-15 | m/z=452.15 (₃₁H₂₀N₂O₂=452.51) | Sub2-16 | m/z=500.19(C₃₆H₂₄N₂O=500.60) |
| Sub2-17 | m/z=465.20(C₃₄H₁₉D₄NO=465.59) | Sub2-18 | m/z=482.11(C₃₁H₁₈N₂O₂S=482.56) |
| Sub2-19 | m/z=567.17(C₄₀H₂₅NOS=567.71) | Sub2-20 | m/z=351.11(C₂₄H₁₇NS=351.47) |
| Sub2-21 | m/z=475.14(C₃₄H₂₁NS=475.61) | Sub2-22 | m/z=477.16(C₃₄H₂₃NS=477.63) |
| Sub2-23 | m/z=577.19(C₄₂H₂₇NS=577.75) | Sub2-24 | m/z=477.16(C₃₄H₂₃NS=477.63) |
| Sub2-25 | m/z=467.17(C₃₃H₂₅NS=467.63) | Sub2-26 | m/z=427.14(C₃₀H₂₁NS=427.57) |
| Sub2-27 | m/z=441.12(C₃₀H₁₉NOS=441.55) | Sub2-28 | m/z=427.14(C₃₀H₂₁NS=427.57) |
| Sub2-29 | m/z=457.10(C₃₀H₁₉NS₂=457.61) | Sub2-30 | m/z=527.17(C₃₈H₂₅NS=527.69) |
| Sub2-31 | m/z=517.15(C₃₆H₂₃NOS=517.65) | Sub2-32 | m/z=352.10(C₂₃H₁₆N₂S=352.46) |
| Sub2-33 | m/z=451.14(C₃₂H₂₁NS=451.59) | Sub2-34 | m/z=553.19(C₄₀H₂₇NS=553.72) |
| Sub2-35 | m/z=503.17(C₃₆H₂₅NS=503.66) | Sub2-36 | m/z=451.4(C₃₂H₂₁NS=451.59) |
| Sub2-37 | m/z=592.20(C₄₂H₂₈N₂S=592.76) | Sub2-38 | m/z=583.14(C₄₀H₂₅NS₂=583.77) |
| Sub2-39 | m/z=385.15(C₂₈H₁₉NO=385.47) | Sub2-40 | m/z=352.10(C₂₃H₁₆N₂S=352.46) |
| Sub2-41 | m/z=401.12(C₂₈H₁₉NS=401.53) | Sub2-42 | m/z=477.16(C₃₄H₂₃NS=477.63) |
| Sub2-43 | m/z=507.11(C₃₄H₂₁NS₂=507.67) | Sub2-44 | m/z=553.19(C₄₀H₂₇NS=553.72) |
| Sub2-45 | m/z=527.17(C₃₈H₂₅NS=527.69) | Sub2-46 | m/z=517.15(C₃₆H₂₃NOS=517.65) |
| Sub2-47 | m/z=498.09(C₃₁H₁₈N₂OS₂=498.62) | Sub2-48 | m/z=427.14(C₃₀H₂₁NS=427.57) |
| Sub2-49 | m/z=468.13(C₃₁H₂₀N₂OS=468.57) | Sub2-50 | m/z=385.15(C₂₈H₁₉NO=385.47) |
| Sub2-51 | m/z=441.12(C₃₀H₁₉NOS=441.55) | Sub2-52 | m/z=367.14(C₂₅H₂₁NS=367.51) |
| Sub2-53 | m/z=577.24(C₄₃H₃₁NO=577.73) | Sub2-54 | m/z=351.16(C₂₅H₂₁NO=351.45) |
| Sub2-55 | m/z=435.16(C₃₂H₂₁NO=435.53) | Sub2-56 | m/z=451.19(C₃₃H₂₅NO=451.57) |
| Sub2-57 | m/z=427.19(C₃₁H₂₅NO=427.55) | Sub2-58 | m/z=441.12(C₃₀H₁₉NOS=441.55) |
| Sub2-59 | m/z=477.21(C₃₅H₂₇NO=477.61) | Sub2-60 | m/z=528.22(C₃₈H₂₈N₂O=528.66) |
| Sub2-61 | m/z=375.13(C₂₆H₁₇NO₂=375.43) | Sub2-62 | m/z=609.25(C₄₄H₃₅NS=609.83) |
| Sub2-63 | m/z=533.18(C₃₇H₂₇NOS=533.69) | Sub2-64 | m/z=507.17(C₃₅H₂₅NOS=507.65) |
| Sub2-65 | m/z=572.16(C₃₈H₂₄N₂O₂S=572.68) | Sub2-66 | m/z=391.10(C₂₆H₁₇NOS=391.49) |
| Sub2-67 | m/z=724.25(C₅₁H₃₆N₂OS=724.92) | Sub2-68 | m/z=527.22 (C₃₉H₂₉NO=527.67) |
| Sub2-69 | m/z=441.12(C₃₀H₁₉NOS=441.55) | Sub2-70 | m/z=493.19(C₃₅H₂₇NS=493.67) |
| Sub2-71 | m/z=467.17(C₃₃H₂₅NS=467.63) | Sub2-72 | m/z=556.18(C₃₈H₂₄N₂O₃=556.62) |
| Sub2-73 | m/z=475.16(C₃₄H₂₁NO₂=475.55) | Sub2-74 | m/z=567.22(C₄₁H₂₉NO₂=567.69) |
| Sub2-75 | m/z=627.26(C₄₇H₃₃NO=627.79) | Sub2-76 | m/z=527.22 (C₃₉H₂₉NO=527.67) |
| Sub2-77 | m/z=335.13(C₂₄H₁₇NO=335.41) | Sub2-78 | m/z=467.17(C₃₃H₂₅NS=467.63) |
| Sub2-79 | m/z=501.17(C₃₆H₂₃NO₂=501.59) | Sub2-80 | m/z=351.11(C₂₄H₁₇NS=351.47) |
| Sub2-81 | m/z=351.11(C₂₄H₁₇NS=351.47) | Sub2-82 | m/z=516.17(C₃₆H₂₄N₂S=516.66) |
| Sub2-83 | m/z=708.31(C₅₂H₄₀N₂O=708.91) | Sub2-84 | m/z=401.12(C₂₈H₁₉NS=401.53) |
| Sub2-85 | m/z=401.12(C₂₈H₁₉NS=401.53) | Sub2-86 | m/z=351.11(C₂₄H₁₇NS=351.47) |
| Sub2-87 | m/z=518.18(C₃₆H₂₆N₂S=518.68) | Sub2-88 | m/z=427.14(C₃₀H₂₁NS=427.57) |
| Sub2-89 | m/z=461.18(C₃₄H₂₃NO=461.56) | Sub2-90 | m/z=461.18(C₃₄H₂₃NO=461.56) |
| Sub2-91 | m/z=335.13(C₂₄H₁₇NO=335.41) | Sub2-92 | m/z=561.21(C₄₂H₂₇NO=561.68) |
| Sub2-93 | m/z=461.18(C₃₄H₂₃NO=461.56) | Sub2-94 | m/z=353.12(C₂₄H₁₆FNO=353.40) |
| Sub2-95 | m/z=391.19(C₂₈H₂₅NO=391.51) | Sub2-96 | m/z=259.10( C₁₈H₁₃NO=259.31) |
| Sub2-97 | m/z=275.08(C₁₈H₁₃NS=275.37) | | |
| Sub3-1 | m/z=375.16(C₂₇H₂₁NO=375.47) | Sub3-2 | m/z=375.16(C₂₇H₂₁NO=375.47) |
| Sub3-3 | m/z=375.16(C₂₇H₂₁NO=375.47) | Sub3-4 | m/z=391.14(C₂₇H₂₁NS=391.53) |
| Sub3-5 | m/z=499.19(C₃₇H₂₅NO=499.61) | Sub3-6 | m/z=499.19(C₃₇H₂₅NO=499.61) |
| Sub3-7 | m/z=499.19(C₃₇H₂₅NO=499.61) | Sub3-8 | m/z=499.19(C₃₇H₂₅NO=499.61) |
| Sub3-9 | m/z=515.17(C₃₇H₂₅NS=515.67) | Sub3-10 | m/z=497.18 (C₃₇H₂₃NO=497.60) |
| Sub3-11 | m/z=513.16(C₃₇H₂₃NS=513.66) | Sub3-12 | m/z=451.19(C₃₃H₂₅NO=451.57) |
| Sub3-13 | m/z=467.17(C₃₃H₂₅NS=467.63) | Sub3-14 | m/z=451.19(C₃₃H₂₅NO=451.57) |
| Sub3-15 | m/z=467.17(C₃₃H₂₅NS=467.63) | Sub3-16 | m/z=451.19(C₃₃H₂₅NO=451.57) |
| Sub3-17 | m/z=467.17(C₃₃H₂₅NS=467.63) | Sub3-18 | m/z=451.19(C₃₃H₂₅NO=451.57) |
| Sub3-19 | m/z=451.19(C₃₃H₂₅NO=451.57) | Sub3-20 | m/z=451.19(C₃₃H₂₅NO=451.57) |
| Sub3-21 | m/z=451.19(C₃₃H₂₅NO=451.57) | Sub3-22 | m/z=467.17(C₃₃H₂₅NS=467.63) |
| Sub3-23 | m/z=467.17(C₃₃H₂₅NS=467.63) | Sub3-24 | m/z=467.17(C₃₃H₂₅NS=467.63) |
| Sub3-25 | m/z=501.21(C₃₇H₂₇NO=501.63) | Sub3-26 | m/z=517.19(C₃₇H₂₇NS=517.69) |
| Sub3-27 | m/z=501.21(C₃₇H₂₇NO=501.63) | Sub3-28 | m/z=517.19(C₃₇H₂₇NS=517.69) |
| Sub3-29 | m/z=501.21(C₃₇H₂₇NO=501.63) | Sub3-30 | m/z=517.19(C₃₇H₂₇NS=517.69) |
| Sub3-31 | m/z=452.19(C₃₂H₂₄N₂O=452.56) | Sub3-32 | m/z=583.23(C₄₂H₃₃NS=583.79) |
| Sub3-33 | m/z=525.21(C₃₉H₂₇NO=525.65) | Sub3-34 | m/z=519.20(C₃₇H₂₁D₄NS=519.70) |
| Sub3-35 | m/z=527.22 (C₃₉H₂₉NO=527.67) | Sub3-36 | m/z=543.20(C₃₉H₂₉NS=543.73) |
| Sub3-37 | m/z=527.22 (C₃₉H₂₉NO=527.67) | Sub3-38 | m/z=543.20(C₃₉H₂₉NS=543.73) |
| Sub3-39 | m/z=543.20(C₃₉H₂₉NS=543.73) | Sub3-40 | m/z=527.22 (C₃₉H₂₉NO=527.67) |
| Sub3-41 | m/z=527.22(C₃₉H₂₉NO=527.67) | Sub3-42 | m/z=527.22(C₃₉H₂₉NO=527.67) |
| Sub3-43 | m/z=543.20(C₃₉H₂₉NS=543.73) | Sub3-44 | m/z=543.20(C₃₉H₂₉NS=543.73) |
| Sub3-45 | m/z=452.19(C₃₂H₂₄N₂O=452.56) | Sub3-46 | m/z=441.16(C₃₁H₂₃NS=441.59) |
| Sub3-47 | m/z=441.16(C₃₁H₂₃NS=441.59) | Sub3-48 | m/z=425.18(C₃₁H₂₃NO=425.53) |
| Sub3-49 | m/z=425.18(C₃₁H₂₃NO=425.53) | Sub3-50 | m/z=467.17(C₃₃H₂₅NS=467.63) |
| Sub3-51 | m/z=577.24(C₄₃H₃₁NO=577.73) | Sub3-52 | m/z=573.21(C₄₃H₂₇NO=573.70) |
| Sub3-53 | m/z=497.18(C₃₇H₂₃NO=497.60) | Sub3-54 | m/z=497.18(C₃₇H₂₃NO=497.60) |
| Sub3-55 | m/z=513.16(C₃₇H₂₃NS=513.66) | Sub3-56 | m/z=441.16(C₃₁H₂₃NS=441.59) |
| Sub3-57 | m/z=549.21(C₄₁H₂₇NO=549.67) | | |
| Sub4-1 | m/z=513.17(C₃₇H₂₃NO₂=513.60) | Sub4-2 | m/z=513.17(C₃₇H₂₃NO₂=513.60) |
| Sub4-3 | m/z=513.17(C₃₇H₂₃NO₂=513.60) | Sub4-4 | m/z=513.17(C₃₇H₂₃NO₂=513.60) |
| Sub4-5 | m/z=579.17(C₄₁H₂₅NOS=579.72) | Sub4-6 | m/z=531.16(C₃₇H₂₂FNO₂=531.59) |
| Sub4-7 | m/z=563.19(C₄₁H₂₅NO₂=563.66) | Sub4-8 | m/z=665.24(C₄₉H₃₁NO₂=665.79) |
| Sub4-9 | m/z=529.15(C₃₇H₂₃NOS=529.66) | Sub4-10 | m/z=529.15(C₃₇H₂₃NOS=529.66) |
| Sub4-11 | m/z=529.15(C₃₇H₂₃NOS=529.66) | Sub4-12 | m/z=529.15(C₃₇H₂₃NOS=529.66) |
| Sub4-13 | m/z=529.15(C₃₇H₂₃NOS=529.66) | Sub4-14 | m/z=529.15(C₃₇H₂₃NOS=529.66) |
| Sub4-15 | m/z=529.15(C₃₇H₂₃NOS=529.66) | Sub4-16 | m/z=529.15(C₃₇H₂₃NOS=529.66) |
| Sub4-17 | m/z=545.13(C₃₇H₂₃NS₂=545.72) | Sub4-18 | m/z=589.20(C₄₃H₂₇NO₂=589.69) |
| Sub4-19 | m/z=605.18(C₄₃H₂₇NOS=605.76) | Sub4-20 | m/z=605. 18(C₄₃H₂₇NOS=605.76) |
| Sub4-21 | m/z=621.16(C₄₃H₂₇NS₂=621.82) | Sub4-22 | m/z=655.20(C₄₇H₂₉NOS=655.82) |
| Sub4-23 | m/z=671.17(C₄₇H₂₉NS₂=671.88) | Sub4-24 | m/z=606.18(C₄₂H₂₆N₂OS=606.74) |
| Sub4-25 | m/z=622.15(C₄₂H₂₆N₂S₂=622.80) | Sub4-26 | m/z=589.20(C₄₃H₂₇NO₂=589.69) |
| Sub4-27 | m/z=605.18(C₄₃H₂₇NOS=605.76) | Sub4-28 | m/z=589.20(C₄₃H₂₇NO₂=589.69) |
| Sub4-29 | m/z=605.18(C₄₃H₂₇NOS=605.76) | Sub4-30 | m/z=605.18(C₄₃H₂₇NOS=605.76) |
| Sub4-31 | m/z=621.16(C₄₃H₂₇NS₂=621.82) | Sub4-32 | m/z=563.19(C₄₁H₂₅NO₂=563.66) |
| Sub4-33 | m/z=595.14(C₄₁H₂₅NS₂=595.78) | Sub4-34 | m/z=579.17(C₄₁H₂₅NOS=579.72) |
| Sub4-35 | m/z=605.18(C₄₃H₂₇NOS=605.76) | Sub4-36 | m/z=671.17(C₄₇H₂₉NS₂=671.88) |
| Sub4-37 | m/z=665.24(C₄₉H₃₁NO₂=665.79) | Sub4-38 | m/z=549.15(C₃₇H₁₉D₄NS₂=549.74) |
| Sub4-39 | m/z=665.24(C₄₉H₃₁NO₂=665.79) | Sub4-40 | m/z=589.20(C₄₃H₂₇NO₂=589.69) |
| Sub4-41 | m/z=671.17(C₄₇H₂₉NS₂=671.88) | Sub4-42 | m/z=595.14(C₄₁H₂₅NS₂=595.78) |
| Sub4-43 | m/z=595.14(C₄₁H₂₅NS₂=595.78) | Sub4-44 | m/z=563.19(C₄₁H₂₅NO₂=563.66) |

### III. Synthesis of Final Product

### 1. Synthesis example of reference compound P-1

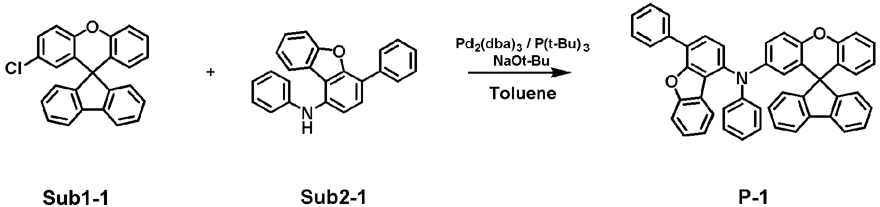

Sub1-1 (20 g, 54.5 mmol) was dissolved in a round bottom flask with toluene (400 mL) and Sub2-1 (18.3 g, 54.5 mmol), Pd₂(dba)₃ (1.5 g, 1.64 mmol), P(t-Bu)₃ (50wt% Sol.) (1.3 mL, 3.3 mmol), NaOt-Bu (15.7 g, 163.6 mmol) were added and stirred at 80° C. When the reaction was completed, the solvent was removed, extracted with CH₂Cl₂ and water, the organic layer was dried over MgSO₄, concentrated, and the resulting compound was purified by silica gel column and sublimation to obtain 29.8 g (82% yield) of the product.

### 2. Synthesis example of reference P-73

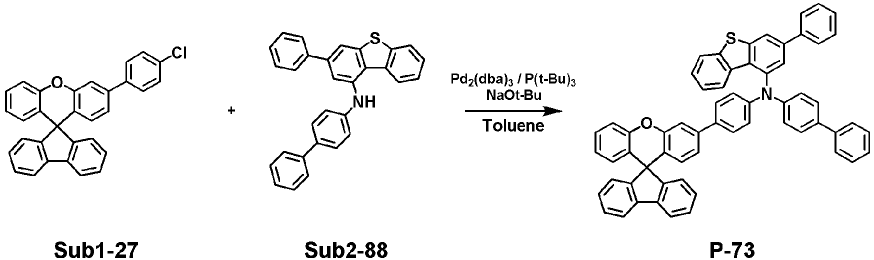

Sub1-27 (17 g, 38.4 mmol) and Sub2-88 (16.4 g, 38.4 mmol), Pd₂(dba)₃ (1.05 g, 1.15 mmol), NaOt-Bu (11.06 g, 115.14 mmol), anhydrous toluene (340 mL), P(t-Bu)₃ (50wt% Sol.) (0.93 mL, 2.3 mmol) were obtained 20.4 g (80% yield) of P-73 using the synthesis method of P-1.

### 3. Synthesis example of reference compound P-113

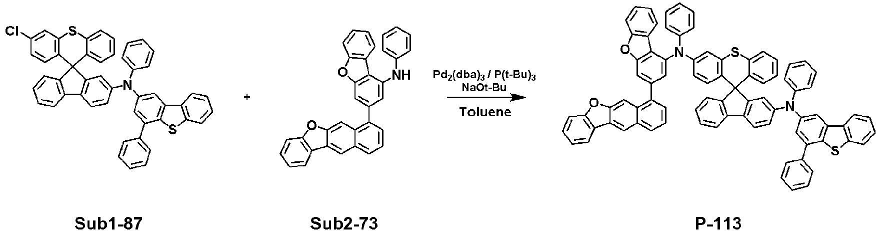

Sub1-87 (12 g, 16.4 mmol) and Sub2-73 (7.8 g, 16.4 mmol), Pd₂(dba)₃ (0.45 g, 0.5 mmol), NaOt-Bu (4.7 g, 49.2 mmol), anhydrous toluene (240 mL), P(*t*-Bu)₃ (50wt% Sol.) (0.2 mL, 0.98 mmol) were obtained 14.2 g (74% yield) of P-113 using the synthesis method of P-1.

### 4. Synthesis example of reference compound P-121

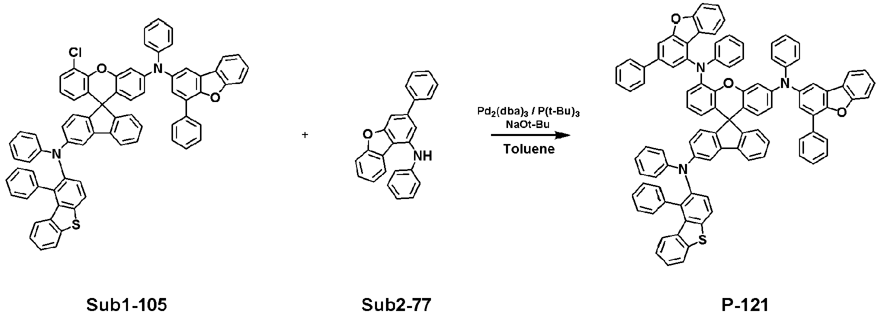

Sub1-105 (12 g, 11.4 mmol) and Sub2-77 (3.8 g, 11.4 mmol), Pd₂(dba)₃ (0.3 g, 0.34 mmol), NaOt-Bu (3.3 g, 34.3 mmol), anhydrous toluene (240 mL), P(*t*-Bu)₃ (50wt% Sol.) (0.3 mL, 0.7 mmol) were obtained 11.7 g (76% yield) of P-121 using the synthesis method of P-1.

### 5. Synthesis example of reference compound P-139

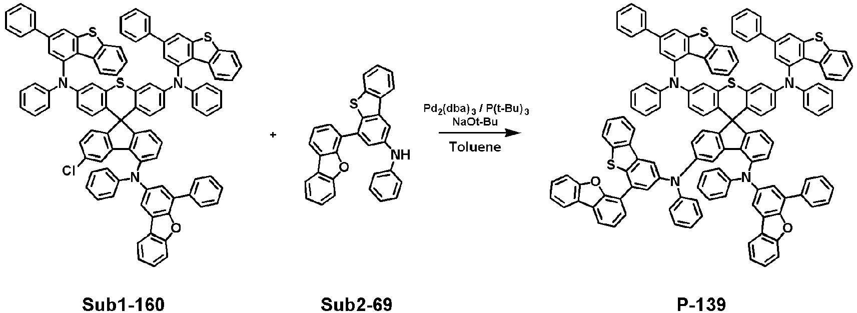

Sub1-160 (9 g, 7.8 mmol) and Sub2-69 (3.5 g, 7.8 mmol), Pd₂(dba)₃ (0.2 g, 0.23 mmol), NaOt-Bu (2.3 g, 23.5 mmol), anhydrous toluene (180 mL), P(*t*-Bu)₃ (50wt% Sol.) (0.2 mL, 0.5 mmol) were obtained 10.9 g (73% yield) of P-139 using the synthesis method of P-1.

### 6. Synthesis example of P 1-2

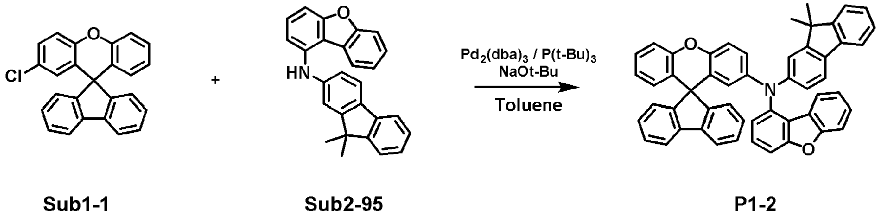

Sub1-1 (15 g, 40.9 mmol) and Sub2-95 (16.0 g, 40.9 mmol), Pd₂(dba)₃ (1.12 g, 1.2 mmol), NaOt-Bu (11.8 g, 122.7 mmol), anhydrous toluene (300 mL), P(t-Bu)₃ (50wt% Sol.) (1.00 mL, 2.5 mmol) were obtained 24.2 g (84% yield) of P1-2 using the synthesis method of P-1.

### 7. Synthesis example of P 1-13

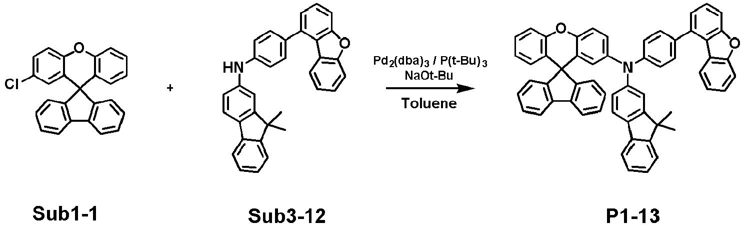

Sub1-1 (10.2 g, 27.8 mmol) and Sub3-12 (12.6 g, 27.8 mmol), Pd₂(dba)₃ (0.8 g, 0.76 mmol), NaOt-Bu (8.0 g, 83.4 mmol), anhydrous Toluene (210 mL), P(*t*-Bu)₃ (50wt% Sol.) (0.7 mL, 1.7 mmol) were obtained 17.6 g (81% yield) of P1-13 using the synthesis method of P-1.

### 8. Synthesis example of reference compound P 2-3

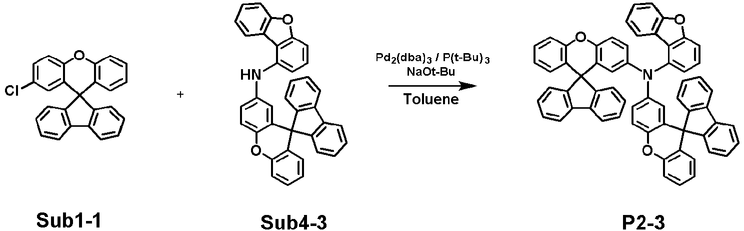

Sub1-1 (9.5 g, 25.9 mmol) and Sub4-3 (13.3 g, 25.9 mmol), Pd₂(dba)₃ (0.7 g, 0.8 mmol), NaOt-Bu (7.5 g, 77.7 mmol), anhydrous toluene (200 mL), P(*t*-Bu)₃ (50wt% Sol.) (0.6 mL, 0.8 mmol) were obtained 18.1 g (83% yield) of P2-3 using the synthesis method of P-1.

The FD-MS values of reference compounds P-1 to P-139, and P2-1 to P2-55, and of inventive compounds P1-1 to P1-86, prepared according to the synthesis example as described above, are shown in Table 3.

**[Table 3]**

| Compou nd | FD-MS | compou nd | FD-MS |
|---|---|---|---|
| P-1 | m/z=665.24(C₄₉H₃₁NO₂=665.79) | P-2 | m/z=791.28(C₅₉H₃₇NO₂=791.95) |
| P-3 | m/z=891.31(C₆₇H₄₁NO₂=892.07) | P-4 | m/z=741.27(C₅₅H₃₅NO₂=741.89) |
| P-5 | m/z=791.28(C₅₉H₃₇NO₂=791.95) | P-6 | m/z=665.24(C₄₉H₃₁NO₂=665.79) |
| P-7 | m/z=665.24(C₄₉H₃₁NO₂=665.79) | P-8 | m/z=665. 24(C₄₉H₃₁NO₂=665.79) |
| P-9 | m/z=665.24(C₄₉H₃₁NO₂=665.79) | P-10 | m/z=857.33(C₆₄H₄₃NO₂=858.05) |
| P-11 | m/z=841.30(C₆₃H₃₉NO₂=842.01) | P-12 | m/z=817.30(C₆₁H₃₉NO₂=817.99) |
| P-13 | m/z=867.31(C₆₅H₄₁NO₂=868.05) | P-14 | m/z=833.30(C₆₀H₃₉N₃O₂=833.99) |
| P-15 | m/z=666 . 23 (C₄₈H₃₀N₂O₂=666. 78) | P-16 | m/z=782 . 26(C₅₆H₃₄N₂O₃=782.90) |
| P-17 | m/z=830.29(C₆₁H₃₈N₂O₂=830.99) | P-18 | m/z=795.31(C₅₉H₃₃D₄NO₂=795.97) |
| P-19 | m/z=812.21(C₅₆H₃₂N₂O₃S=812.94) | P-20 | m/z=897.27(C₆₅H₃₉NO₂S=898.09) |
| P-21 | m/z=681.21(C₄₉H₃₁NOS=681.85) | P-22 | m/z=807.26(C₅₉H₃₇NOS=808.01) |
| P-23 | m/z=907.29(C₆₇H₄₁NOS=908.13) | P-24 | m/z=807.26(C₅₉H₃₇NOS=808.01) |
| P-25 | m/z=805.24(C₅₉H₃₅NOS=806.00) | P-26 | m/z=797.28(C₅₈H₃₉NOS=798.02) |
| P-27 | m/z=757.24(C₅₅H₃₅NOS=757.95) | P-28 | m/z=771.22(C₅₅H₃₃NO₂S=771.93) |
| P-29 | m/z=757.24(C₅₅H₃₅NOS=757.95) | P-30 | m/z=787.20(C₅₅H₃₃NOS₂=788.00) |
| P-31 | m/z=857.28(C₆₃H₃₉NOS=858.07) | P-32 | m/z=833.28(C₆₁H₃₉NOS=834.05) |
| P-33 | m/z=883.29(C₆₅H₄₁NOS=884.11) | P-34 | m/z=781.24(C₅₇H₃₅NOS=781.97) |
| P-35 | m/z=682.21(C₄₈H₃₀N₂OS=682.84) | P-36 | m/z=681.21(C₄₉H₃₁NOS=681.85) |
| P-37 | m/z=807.26(C₅₉H₃₇NOS=808.01) | P-38 | m/z=907. 29(C₆₇H₄₁NOS=908.13) |
| P-39 | m/z=807.26(C₅₉H₃₇NOS=808.01) | P-40 | m/z=807. 26(C₅₉H₃₇NOS=808.01) |
| P-41 | m/z=681.21(C₄₉H₃₁NOS=681.85) | P-42 | m/z=681.21(C₄₉H₃₁NOS=681.85) |
| P-43 | m/z=681.21(C₄₉H₃₁NOS=681.85) | P-44 | m/z=681.21(C₄₉H₃₁NOS=681.85) |
| P-45 | m/z=873.31(C₆₄H₄₃NOS=874.11) | P-46 | m/z=857. 28(C₆₃H₃₉NOS=858.07) |
| P-47 | m/z=833.28(C₆₁H₃₉NOS=834.05) | P-48 | m/z=883.29(C₆₅H₄₁NOS=884.11) |
| P-49 | m/z=781.24(C₅₇H₃₅NOS=781.97) | P-50 | m/z=682. 21(C₄₈H₃₀N₂OS=682.84) |
| P-51 | m/z=798.23(C₅₆H₃₄N₂O₂S=798.96) | P-52 | m/z=846.27(C₆₁H₃₈N₂OS=847.05) |
| P-53 | m/z=811.28(C₅₉H₃₃D₄NOS=812.04) | P-54 | m/z=828. 19(C₅₆H₃₂N₂O₂S₂=829.00) |
| P-55 | m/z=863.23(C₆₁H₃₇NOS₂=864.09) | P-56 | m/z=823. 24(C₅₉H₃₇NS₂=824.07) |
| P-57 | m/z=863.23(C₆₁H₃₇NOS₂=864.09) | P-58 | m/z=929. 22(C₆₅H₃₉NS₃=930.21) |
| P-59 | m/z=938.28(C₆₇H₄₂N₂S₂=939.21) | P-60 | m/z=797 . 22(C₅₇H₃₅NS₂=798.03) |
| P-61 | m/z=813.25(C₅₈H₃₉NS₂=814.08) | P-62 | m/z=773.22(C₅₅H₃₅NS₂=774.01) |
| P-63 | m/z=747.21(C₅₃H₃₃NS₂=747.97) | P-64 | m/z=823.24(C₅₉H₃₇NS₂=824.07) |
| P-65 | m/z=853.19(C₅₉H₃₅NS₃=854.12) | P-66 | m/z=923.27(C₆₇H₄₁NS₂=924.19) |
| P-67 | m/z=949.28(C₆₉H₄₃NS₂=950.23) | P-68 | m/z=781.24(C₅₇H₃₅NOS=781.97) |
| P-69 | m/z=847.24(C₆₁H₃₇NS₂=848.09) | P-70 | m/z=698. 19(C₄₈H₃₀N₂S₂=698.90) |
| P-71 | m/z=874.27(C₆₂H₃₈N₂O₂S=875.06) | P-72 | m/z=872.29(C₆₃H₄₀N₂OS=873.09) |
| P-73 | m/z=833.28(C₆₁H₃₉NOS=834.05) | P-74 | m/z=970.21(C₆₆H₃₈N₂OS₃=971.22) |
| P-75 | m/z=1055.33(C₇₆H₄₉NOS₂=1056.35) | P-76 | m/z=741.27(C₅₅H₃₅NO₂=741.89) |
| P-77 | m/z=883.29(C₆₅H₄₁NOS=884.11) | P-78 | m/ z=931.31(C₆₉H₄₁NO₃=932.09) |
| P-79 | m/z=868.31C₆₄H₄₀N₂O₂=869. 04) | P-80 | m/z=841.30(C₆₃H₃₉NO₂=842.01) |
| P-81 | m/z=922.32(C₆₇H₄₂N₂O₃=923.08) | P-82 | m/z=1124.40(C₈₃H₅₂N₂O₃=1125.34) |
| P-83 | m/z=1164.43(C₈₆H₅₆N₂O₃=1165.41) | P-84 | m/z=1124.40(C₈₃H₅₂N₂O₃=1125.34) |
| P-85 | m/z=1014.33(C₃₇H₄₆N₂O₂S=1015.24) | P-86 | m/z=1154.35(C₈₃H₅₀N₂O₃S=1155.38) |
| P-87 | m/z=1115.41(C₈₁H₅₃N₃O₃=1116.33) | P-88 | m/z=1140.43(C₈₄H₅₆N₂O₃=1141.38) |
| P-89 | m/z=1104.34(C₇₉H₄₈N₂O₃S=1105.32) | P-90 | m/z=1074.38(C₇₉H₅₀N₂O₃=1075.28) |
| P-91 | m/z=1038.38(C₇₆H₅₀N₂O₃=1039.25) | P-92 | m/z=1235.38(C₈₇H₅₃N₃O₄S=1236.46) |
| P-93 | m/z=1154. 35(C₈₃H₅₀N₂O₃S=1155.38) | P-94 | m/z=1196.40(C₈₆H₅₆N₂O₃S=1197.47) |
| P-95 | m/z=1296.43(C₉₄H₆₀N₂O₃S=1297.59) | P-96 | m/z=1232.38(C₈₉H₅₆N₂OS₂=1233.56) |
| P-97 | m/z=1196.35(C₈₅H₅₂N₂O₂S₂=1197.48) | P-98 | m/z=1154.35(C₈₃H₅₀N₂O₃S=1155.38) |
| P-99 | m/z=1443 .45(C₁₀₂H₆₅N₃O₃S₂=1444.78) | P-100 | m/z=1190.44(C₈₈H₅₈N₂O₃=1191.44) |
| P-101 | m/z=938.30(C₆₇H₄₂N₂O₂S=939.15) | P-102 | m/z=1140.37(C₈₃H₅₂N₂O₂S=1141.40) |
| P-103 | m/z=1180.41(C₈₆H₅₆N₂O₂S=1181.47) | P-104 | m/z=1216.41(C₈₉H₅₆N₂O₂S=1217.50) |
| P-105 | m/z=1030.31(C₇₃H₄₆N₂OS₂=1031.30) | P-106 | m/z=1170.33(C₈₃H₅₀N₂O₂S₂=1171.45) |
| P-107 | m/z=1131.39(C₈₁H₅₃N₃O₂S=1132.39) | P-108 | m/z=1188.36(C₈₄H₅₆N₂S₃=1189.57) |
| P-109 | m/z=1120.32(C₇₉H₄₈N₂O₂S₂=1121.39) | P-110 | m/z=1090.36(C₇₉H₅₀N₂O₂S=1091.34) |
| P-111 | m/z=1070.34(C₇₆H₅₀N₂OS₂=1071.37) | P-112 | m/z=1251.35(C₈₇H₅₃N₃O₃S₂=1252.52) |
| P-113 | m/z=1170.33(C₈₃H₅₀N₂O₂S₂=1171.45) | P-114 | m/z=1246.42(C₉₀H₅₈N₂O₃S=1247.53) |
| P-115 | m/z=1346.45(C₉₈H₆₂N₂O₃S=1347.65) | P-116 | m/z=1216.41(C₈₉H₅₆N₂O₂S=1217.50) |
| P-117 | m/z=1271.39(C₉₁H₅₇N₃OS₂=1272.60) | P-118 | m/z=1170.33(C₈₃H₅₀N₂O₂S₂=1171.45) |
| P-119 | m/z=1459.42(C₁₀₂H₆₅N₃O₂S₃=1460.84) | P-120 | m/z=1206.42(C₈₈H₅₈N₂O₂S=1207.50) |
| P-121 | m/z=1347.44(C₉₇H₆₁N₃O₃S=1348.63) | P-122 | m/z=1579.48(C₁₁₃H₆₉N₃O_{S}S₂=1580.93) |
| P-123 | m/z=1705.58(C₁₂₄H₇₀N₃O₄S=1707.07) | P-124 | m/z=1489.47(C₁₀₇H₆₇N₃O₂S₂=1490.85) |
| P-125 | m/z=1453.43(C₁₀₃H₆₃N₃O₃S₂=1454.78) | P-126 | m/z=1619.51(C₁₁₆H₇₃N₃O₃S₂=1621.00) |
| P-127 | m/z= 1456.42(C₁₀₂H₆₄N₄OS₃=1457.84) | P-128 | m/z=1565.50(C₁₁₃H₇₁N₃O₂S₂=1566.95) |
| P-129 | m/z=1528.48(C₁₀₉H₆₈N₄O₂S₂=1529.89) | P-130 | m/z=1519.42(C₁₀₇H₆₅N₃O₂S₃=1520.90) |
| P-131 | m/z=1826.61(C₁₃₁H₈₆N₄O₃S₂=1828.27) | P-132 | m/z=1555.51(C₁₁₂H₇₃N₃O₂S₂=1556.96) |
| P-133 | m/z=1439.45(C₁₀₃H₆₅N₃O₂S₂=1440.79) | P-134 | m/z=1595.46(C₁₁₃H₆₉N₃O₂S₂=1596.99) |
| P-135 | m/z=1721.56(C₁₂₄H₇₉N₃O₃S₂=1723.13) | P-136 | m/z=1571.44(C₁₁₁H₆₉N₃S₄=1573.03) |
| P-137 | m/z=1898.71(C₁₃₃H₁₀₂N₄O₅S₅=1900.48) | P-138 | m/z=1812.54(C₁₂₉H₈₀N₄O₂S₃=1814.27) |
| P-139 | m/z=1914.69(C₁₃₃H₁₀₂N₄O₂S₄=1916.55) | | |
| P1-1 | m/z=705.27(C₅₂H₃₅NO₂=705.86) | P1-2 | m/z=705.27(C₅₂H₃₅NO₂=705.86) |
| P1-3 | m/z=705.27(C₅₂H₃₅NO₂=705.86) | P1-4 | m/z=705.27(C₅₂H₃₅NO₂=705.86) |
| P1-5 | m/z=721.24(C₅₂H₃₅NOS=721.92) | P1-6 | m/z=829.30(C₆₂H₃₉NO₂=830.00) |
| P1-7 | m/z=829.30(C₆₂H₃₉NO₂=830.00) | P1-8 | m/z=829.30(C₆₂H₃₉NO₂=830.00) |
| P1-9 | m/z=829.30(C₆₂H₃₉NO₂=830.00) | P1-10 | m/z=845.28(C₆₂H₃₉NOS=846.06) |
| P1-11 | m/z=827.28(C₆₂H₃₇NO₂=827.98) | P1-12 | m/z=843.26(C₆₂H₃₇NOS=844.04) |
| P1-13 | m/z=781.30(C₅₈H₃₉NO₂=781.96) | P1-14 | m/z=797.28(C₅₈H₃₉NOS=798.02) |
| P1-15 | m/z=781.30(C₅₈H₃₉NO₂=781.96) | P1-16 | m/z=797.28(C₅₈H₃₉NOS=798.02) |
| P1-17 | m/z=781.30(C₅₈H₃₉NO₂=781.96) | P1-18 | m/z=797.28(C₅₈H₃₉NOS=798.02) |
| P1-19 | m/z=781.30(C₅₈H₃₉NO₂=781.96) | P1-20 | m/z=781.30(C₅₈H₃₉NO₂=781.96) |
| P1-21 | m/z=781.30(C₅₈H₃₉NO₂=781.96) | P1-22 | m/z=781.30(C₅₈H₃₉NO₂=781.96) |
| P1-23 | m/z=797.28(C₅₈H₃₉NOS=798.02) | P1-24 | m/z=797.28(C₅₈H₃₉NOS=798.02) |
| P1-25 | m/z=797.28(C₅₈H₃₉NOS=798.02) | P1-26 | m/z=831.31(C₆₂H₄₁NO₂=832.02) |
| P1-27 | m/z=847.29(C₆₂H₄₁NOS=848.08) | P1-28 | m/z=831.31(C₆₂H₄₁NO₂=832.02) |
| P1-29 | m/z=847.29(C₆₂H₄₁NOS=848.08) | P1-30 | m/z=831.31(C₆₂H₄₁NO₂=832.02) |
| P1-31 | m/z=847.29(C₆₂H₄₁NOS=848.08) | P1-32 | m/z=782.29(C₅₇H₃₈N₂O₂=782.94) |
| P1-33 | m/z=913.34(C₆₇H₄₇NOS=914.18) | P1-34 | m/z=855. 31(C₆₄H₄₁NO₂=856.04) |
| P1-35 | m/z=849.30(C₆₂H₃₅D₄NOS=850.08) | P1-36 | m/z=857.33(C₆₄H₄₃NO₂=858.05) |
| P1-37 | m/z=873.31(C₆₄H₄₃NOS=874.11) | P1-38 | m/z=857.33(C₆₄H₄₃NO₂=858.05) |
| P1-39 | m/z=873.31(C₆₄H₄₃NOS=874.11) | P1-40 | m/z=873.31(C₆₄H₄₃NOS=874.11) |
| P1-41 | m/z=857.33(C₆₄H₄₃NO₂=858.05) | P1-42 | m/z=857.33(C₆₄H₄₃NO₂=858.05) |
| P1-43 | m/z=857.33(C₆₄H₄₃NO₂=858.05) | P1-44 | m/z=873.31(C₆₄H₄₃NOS=874.11) |
| P1-45 | m/z=873.31(C₆₄H₄₃NOS=874.11) | P1-46 | m/z=797.28(C₅₈H₃₉NOS=798.02) |
| P1-47 | m/z=721.24(C₅₂H₃₅NOS=721.92) | P1-48 | m/z=721.24(C₅₂H₃₅NOS=721.92) |
| P1-49 | m/z=721.24(C₅₂H₃₅NOS=721.92) | P1-50 | m/z=737.22(C₅₂H₃₅NS₂=737.98) |
| P1-51 | m/z=845.28(C₆₂H₃₉NOS=846.06) | P1-52 | m/z=861.25(C₆₂H₃₉NS₂=862.12) |
| P1-53 | m/z=861.25(C₆₂H₃₉NS₂=862.12) | P1-54 | m/z=861.25(C₆₂H₃₉NS₂=862.12) |
| P1-55 | m/z=861.25(C₆₂H₃₉NS₂=862.12) | P1-56 | m/z=921.28(C₆₆H₃₉N₃OS=922.12) |
| P1-57 | m/z=859.24(C₆₂H₃₇NS₂=860.11) | P1-58 | m/z=797.28(C₅₈H₃₉NOS=798.02) |
| P1-59 | m/z=813.25(C₅₈H₃₉NS₂=814.08) | P1-60 | m/z=797.28(C₅₈H₃₉NOS=798.02) |
| P1-61 | m/z=813.25(C₅₈H₃₉NS₂=814.08) | P1-62 | m/z=797.28(C₅₈H₃₉NOS=798.02) |
| P1-63 | m/z=813.25(C₅₈H₃₉NS₂=814.08) | P1-64 | m/z=797.28(C₅₈H₃₉NOS=798.02) |
| P1-65 | m/z=813.25(C₅₈H₃₉NS₂=814.08) | P1-66 | m/z=847.29(C₆₂H₄₁NOS=848.08) |
| P1-67 | m/z=863.27(C₆₂H₄₁NS₂=864.14) | P1-68 | m/z=847.29(C₆₂H₄₁NOS=848.08) |
| P1-69 | m/z=863.27(C₆₂H₄₁NS₂=864.14) | P1-70 | m/z=847.29(C₆₂H₄₁NOS=848.08) |
| P1-71 | m/z=863.27(C₆₂H₄₁NS₂=864.14) | P1-72 | m/z=798.27(C₅₇H₃₈N₂OS=799.00) |
| P1-73 | m/z=797.28(C₅₈H₃₉NOS=798.02) | P1-74 | m/z=787.24(C₅₆H₃₇NS₂=788.04) |
| P1-75 | m/z=787.24(C₅₆H₃₇NS₂=788.04) | P1-76 | m/z=771.26(C₅₆H₃₇NOS=771.98) |
| P1-77 | m/z=771.26(C₅₆H₃₇NOS=771.98) | P1-78 | m/z=813.25(C₅₈H₃₉NS₂=814.08) |
| P1-79 | m/z=907.35(C₆₈H₄₅NO₂=908.11) | P1-80 | m/z=903.31(C₆₈H₄₁NO₂=904.08) |
| P1-81 | m/z=827.28(C₆₂H₃₇NO₂=827.98) | P1-82 | m/z=843.26(C₆₂H₃₇NOS=844.04) |
| P1-83 | m/z=843.26(C₆₂H₃₇NOS=844.04) | P1-84 | m/z=787.24(C₅₆H₃₇NS₂=788.04) |
| P1-85 | m/z=879.31(C₆₆H₄₁NO₂=880.06) | P1-86 | m/z=781.30(C₅₈H₃₉NO₂=781.96) |
| P2-1 | m/z=843. 28(C₆₂H₃₇NO₃=843.98) | P2-2 | m/z=843.28(C₆₂H₃₇NO₃=843.98) |
| P2-3 | m/z=843.28(C₆₂H₃₇NO₃=843.98) | P2-4 | m/z=843.28(C₆₂H₃₇NO₃=843.98) |
| P2-5 | m/z=909.27(C₆₆H₃₉NO₂S=910.10) | P2-6 | m/z=861.27(C₆₂H₃₆FNO₃=861. 97) |
| P2-7 | m/z=893.29(C₆₆H₃₉NO₃=894.04) | P2-8 | m/z=995.34(C₇₄H₄₅NO₃=996.18) |
| P2-9 | m/z=859.25(C₆₂H₃₇NO₂S=860.04) | P2-10 | m/z=859.25(C₆₂H₃₇NO₂S=860.04) |
| P2-11 | m/z=859.25(C₆₂H₃₇NO₂S=860.04) | P2-12 | m/z=859. 25(C₆₂H₃₇NO₂S=860.04) |
| P2-13 | m/z=859.25(C₆₂H₃₇NO₂S=860.04) | P2-14 | m/z=859. 25(C₆₂H₃₇NO₂S=860.04) |
| P2-15 | m/z=859.25(C₆₂H₃₇NO₂S=860.04) | P2-16 | m/z=859. 25(C₆₂H₃₇NO₂S=860.04) |
| P2-17 | m/z=875.23(C₆₂H₃₇NOS₂=876.10) | P2-18 | m/z=875.23(C₆₂H₃₇NOS₂=876.1) |
| P2-19 | m/z=891.21(C₆₂H₃₇NS₃=892.17) | P2-20 | m/z=919.31(C₆₈H₄₁NO₃=920.08) |
| P2-21 | m/z=935.29(C₆₈H₄₁NO₂S=936.14) | P2-22 | m/z=951.26(C₆₈H₄₁NOS₂=952.20) |
| P2-23 | m/z=967.24(C₆₈H₄₁NS₃=968.26) | P2-24 | m/z=985.30(C₇₂H₄₃NO₂S=986.20) |
| P2-25 | m/z=1001.28(C₇₂H₄₃NOS₂=1002.26) | P2-26 | m/z=952. 26(C₆₇H₄₀N₂OS₂=953.19) |
| P2-27 | m/z=952 .26(C₆₇H₄₀N₂OS₂=953.19) | P2-28 | m/z=919. 31(C₆₈H₄₁NO₃=920.08) |
| P2-29 | m/z=935.29(C₆₈H₄₁NO₂S=936.14) | P2-30 | m/z=919. 31(C₆₈H₄₁NO₃=920.08) |
| P2-31 | m/z=935.29(C₆₈H₄₁NO₂S=936.14) | P2-32 | m/z=935.29(C₆₈H₄₁NO₂S=936.14) |
| P2-33 | m/z=967.24(C₆₈H₄₁NS₃=968.26) | P2-34 | m/z=900.23(C₆₃H₃₆N₂OS₂=901.11) |
| P2-35 | m/z=901.25(C₆₄H₃₉NOS₂=902.14) | P2-36 | m/z=893.29(C₆₆H₃₉NO₃=894.04) |
| P2-37 | m/z=941.22(C₆₆H₃₉NS₃=942.23) | P2-38 | m/z=909.27(C₆₆H₃₉NO₂S=910.10) |
| P2-39 | m/z=919.31(C₆₈H₄₁NO₃=920.08) | P2-40 | m/z=941.22(C₆₆H₃₉NS₃=942.23) |
| P2-41 | m/z=909.27(C₆₆H₃₉NO₂S=910.1) | P2-42 | m/z=893.29(C₆₆H₃₉NO₃=894.04) |
| P2-43 | m/z=941.22(C₆₆H₃₉NS₃=942.23) | P2-44 | m/z=909.27(C₆₆H₃₉NO₂S=910.10) |
| P2-45 | m/z=893.29(C₆₆H₃₉NO₃=894.04) | P2-46 | m/z=951.26(C₆₈H₄₁NOS₂=952.2) |
| P2-47 | m/z=1017.26(C₇₂H₄₃NS₃=1018.32) | P2-48 | m/z=995.34(C₇₄H₄₅NO₃=996.18) |
| P2-49 | m/z=895.23(C₆₂H₃₃D₄NS₃=896.19) | P2-50 | m/z=995.34(C₇₄H₄₅NO₃=996.18) |
| P2-51 | m/z=995.34(C₇₄H₄₅NO₃=996.18) | P2-52 | m/z=1093.29(C₇₈H₄₇NS₃=1094.42) |
| P2-53 | m/z=975.26(C₇₀H₄₁NOS₂=976.22) | P2-54 | m/z=991.24(C₇₀H₄₁NS₃=992.29) |
| P2-55 | m/z=943.31(C₇₀H₄₁NO₃=944.10) | | |

### Evaluation of Manufacture of Organic Electronic Element

### [Reference Example 1] Red organic light emitting diode (emitting auxiliary layer)

An organic light emitting diode was manufactured according to a conventional method using the compound of the present invention as an emitting auxiliary layer material. First, a hole injection layer was formed by vacuum depositing 4,4' ,4' '-tris[2-naphthyl(phenyl)amino]triphenylamine (hereinafter, 2-TNATA) to a thickness of 60 nm on an ITO layer (anode) formed on a glass substrate, and a hole transport layer was formed by vacuum deposition of *N,N'*-bis(1-naphthalenyl)-*N,N*'-bis-phenyl-(1,1'-biphenyl)-4,4'-diamine (hereinafter, NPB) to a thickness of 60 nm on the hole injection layer. Subsequently, compound PA-1 was vacuum-deposited to a thickness of 20 nm on the hole transport layer to form an emission auxiliary layer, 4,4'*-N,N*'-dicarbazole-biphenyl (hereinafter, CBP) was used as a host material and bis-(1-phenylisoquinolyl)iridium(III)acetylacetonate (hereinafter, (piq)₂Ir(acac)) was used as a dopant material on the emitting auxiliary layer, doped at a weight ratio of 95:5, and vacuum deposited to a thickness of 30 nm to form an emitting layer.

Subsequently, (1,1'-biphenyl-4-olato)bis(2-methyl-8-quinolinolato)aluminum (hereinafter, BAlq) was vacuum deposited to a thickness of 10 nm on the emitting layer to form a hole blocking layer, an electron transport layer was formed by vacuum depositing tris-(8-hydroxyquinoline)aluminum (hereinafter, Alq₃) to a thickness of 40 nm on the hole blocking layer. Thereafter, an electron injection layer was formed by depositing LiF, an alkali metal halide, to a thickness of 0.2 nm, then, Al was deposited to a thickness of 150 nm to form a cathode, thereby manufacturing an organic light emitting diode.

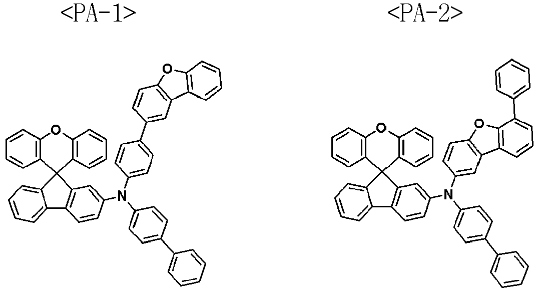

### [Reference Example 2] to [Reference Example 21] Red organic light emitting diode (emitting auxiliary layer)

An organic light emitting diode was manufactured in the same manner as in Reference Example 1, except that the compound PA-2 and the compound as described in Table 4 were used instead of the compound PA-1 as the emitting auxiliary layer material.

### [Comparative example 1]

An organic light emitting diode was manufactured in the same manner as in Reference Example 1, except that the comparative example 1 were used instead of the compound PA-1 as the emitting auxiliary layer material.

### < comparative example 1>

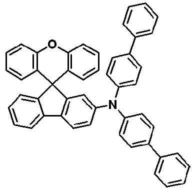

Electroluminescence (EL) characteristics were measured by PR-650 of photoresearch company by applying a forward bias direct current voltage to the organic light emitting diodes prepared according to Reference Examples 1 to 21 and Comparative Example 1, T95 life was measured through a life measurement equipment manufactured by McScience at a luminance of 2500 cd/m², and the measurement results are shown in Table 4.

**[Table 4]**

| | compoun d | Voltage (V) | Current Density (mA/cm²) | Brightness (cd/m²) | Efficiency (cd/A) | Lifetime T(95) | CIE | |
|---|---|---|---|---|---|---|---|---|
| | | | | | | | x | y |
| comparative example(1) | Compara tive compoun d 1 | 6.3 | 26.6 | 2500.0 | 9.4 | 75.6 | 0.64 | 0.31 |
| example(1) | PA-1 | 6.0 | 21.9 | 2500.0 | 11.4 | 98.3 | 0.63 | 0.35 |
| example(2) | PA-2 | 5.6 | 18.1 | 2500.0 | 13.8 | 110.5 | 0.61 | 0.31 |
| example(3) | P-1 | 4.8 | 11.1 | 2500.0 | 22.6 | 116.4 | 0.64 | 0.30 |
| example(4) | P-4 | 4.9 | 10.3 | 2500.0 | 24.3 | 112.7 | 0.63 | 0.31 |
| example(5) | P-12 | 4.8 | 9.7 | 2500.0 | 25.7 | 118.3 | 0.62 | 0.30 |
| example(6) | P-14 | 4.9 | 9.7 | 2500.0 | 25.9 | 117.2 | 0.61 | 0.31 |
| example(7) | P-22 | 4.8 | 9.7 | 2500.0 | 25.7 | 119.0 | 0.64 | 0.30 |
| example(8) | P-30 | 5.0 | 10.7 | 2500.0 | 23.5 | 113.4 | 0.65 | 0.35 |
| example(9) | P-37 | 4.9 | 10.8 | 2500.0 | 23.2 | 116.9 | 0.62 | 0.33 |
| example(10) | P-57 | 5.2 | 10.9 | 2500.0 | 22.9 | 112.0 | 0.64 | 0.32 |
| example(11) | P-59 | 5.2 | 9.7 | 2500.0 | 25.9 | 115.4 | 0.61 | 0.30 |
| example(12) | P-68 | 5.2 | 10.6 | 2500.0 | 23.6 | 114.7 | 0.64 | 0.30 |
| example(13) | P-77 | 5.1 | 10.3 | 2500.0 | 24.4 | 119.5 | 0.61 | 0.30 |
| example(14) | P-81 | 5.0 | 11.6 | 2500.0 | 21.6 | 122.8 | 0.62 | 0.30 |
| example(15) | P-82 | 5.2 | 11.9 | 2500.0 | 21.1 | 120.7 | 0.63 | 0.30 |
| example(16) | P-94 | 5.1 | 11.5 | 2500.0 | 21.8 | 120.9 | 0.65 | 0.32 |
| example(17) | P-109 | 5.0 | 11.6 | 2500.0 | 21.5 | 120.8 | 0.63 | 0.35 |
| example(18) | P-115 | 5.2 | 11.1 | 2500.0 | 22.6 | 123.9 | 0.62 | 0.31 |
| example(19) | P-124 | 5.3 | 12.0 | 2500.0 | 20.8 | 124.1 | 0.60 | 0.34 |
| example(20) | P-136 | 5.3 | 12.3 | 2500.0 | 20.3 | 126.7 | 0.63 | 0.34 |
| example(21) | P-138 | 5.3 | 12.4 | 2500.0 | 20.2 | 127.3 | 0.64 | 0.35 |

As can be seen from the results in Table 4, when a red organic light emitting diode is manufactured using the material for an organic light emitting diode as described herein as a phosphorescent host material, compared to the case of using the compound PA-1, the compound PA-2, and the comparative compound 1, the driving voltage of the organic light emitting diode could be lowered and the efficiency and lifespan were significantly improved.

In detail, the comparative compound and the compound as described herein are the same **in** that they contain a xanthene core and an amine substituent, the device result of compound PA-1 substituted with dibenzofuran was improved compared to Comparative Compound 1 in which a simple aryl group was substituted with the substituent of the amine group, and the device result of compound PA-2 with more secondary substituents bonded was more improved. Also, the compound PA-2 and the compound as described herein differ in the bonding position of the secondary substituent of the amine substituent, dibenzofuran or dibenzothiophene. In other words, it was confirmed that the compound, in which an amine group and a secondary substituent group are bonded to the same ring in dibenzofuran or dibenzothiophene, exhibits more improved device performance than compound PA-2.

This result shows that the difference in hole mobility is large depending on the bonding position of the substituent, and this difference in mobility affects the overall device.

By using the compound as described herein as an emitting auxiliary layer, the HOMO or LUMO energy level of the compound as described herein has an appropriate value between the hole transport layer and the emitting layer, as a result, holes and electrons achieve a charge balance, and light emission occurs inside the emitting layer rather than at the interface of the hole transport layer, maximizing efficiency and lifespan.

Meanwhile, comparing the results of the embodiments as described herein, it can be seen that there are differences in driving voltage and luminous efficiency according to the bonding position and type of the secondary substituent, and different lifespan results are produced depending on the number of substitutions of amino groups in the central core.

In conclusion, the physical properties of the compound such as hole characteristics, light efficiency characteristics, energy level (LUMO, HOMO level, T1 level), hole injection & mobility characteristics, and electron blocking characteristics vary depending on the substituent, resulting in completely different device results.

### [Example 22] Green organic light emitting diode (emitting auxiliary layer)

2-TNATA was vacuum-deposited to a thickness of 60 nm on the ITO layer (anode) formed on the glass substrate to form a hole injection layer, and then NPB was vacuum-deposited to a thickness of 60 nm on the hole injection layer to form a hole transport layer. Subsequently, the following compound PA-3 was vacuum-deposited to a thickness of 20 nm on the hole transport layer to form an emitting auxiliary layer, and on the emitting auxiliary layer, CBP was used as a host material and tris(2-phenylpyridine)-iridium (hereinafter, Ir(ppy)₃) was used as a dopant material, doped at a weight ratio of 95:5, and vacuum deposited to a thickness of 30 nm to form an emitting layer. Then, BAlq was vacuum-deposited to a thickness of 10 nm on the emitting layer to form a hole blocking layer, Alq₃ was vacuum deposited on the hole blocking layer to a thickness of 40 nm on the hole blocking layer to form an electron transport layer. Thereafter, an electron injection layer was formed by depositing LiF, an alkali metal halide, to a thickness of 0.2 nm, and subsequently, Al was deposited to a thickness of 150 nm to form a cathode, thereby manufacturing an organic light emitting diode.

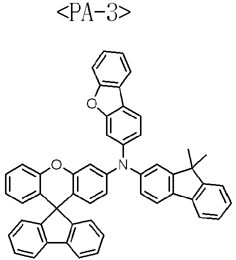

### [Example 23] to [Example 35] Green organic light emitting diode (emitting auxiliary layer)

An organic light emitting diode was manufactured in the same manner as in Example 22, except that the compound of the present invention described in Table 5 was used instead of the compound PA-3 as the emitting auxiliary layer material.

### [Comparative example 2] and [Comparative example 3]

An organic light emitting diode was manufactured in the same manner as in Example 22, except that Comparative Compound 2 and Comparative Compound 3 were used, respectively, instead of the compound PA-3 as the emitting auxiliary layer material.

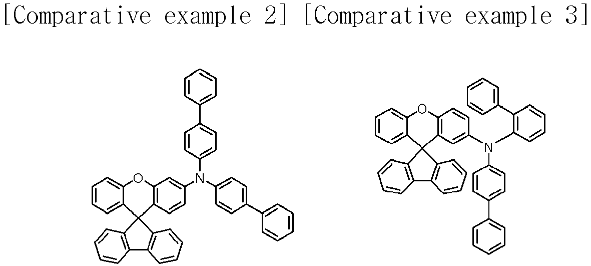

Electroluminescence (EL) characteristics were measured by PR-650 of photoresearch company by applying a forward bias direct current voltage to the organic light emitting diode prepared according to Examples 22 to 35 and Comparative Example 2 to 3 of the present invention, T95 life was measured through a life measurement equipment manufactured by McScience at a luminance of 5000 cd/m², and the measurement results are shown in Table 5.

**[Table 5]**

| | compou nd | Voltag e (V) | Current Density (mA/cm²) | Brightness (cd/m²) | Efficiency (cd/A) | Lifetime T(95) | CIE | |
|---|---|---|---|---|---|---|---|---|
| | | | | | | | x | y |
| comparative example (2) | compar ative compou nd 2 | 6.3 | 36.2 | 5000.0 | 13.8 | 75.6 | 0.30 | 0.61 |
| comparative example (3) | compar ative compou nd 3 | 6.1 | 25.6 | 5000.0 | 19.5 | 75.9 | 0.30 | 0.60 |
| example(22) | PA-3 | 5.6 | 24.5 | 5000.0 | 20.4 | 103.3 | 0.32 | 0.65 |
| example(23) | P1-2 | 5.0 | 10.1 | 5000.0 | 49.7 | 139.6 | 0.32 | 0.62 |
| example(24) | P1-5 | 5.2 | 12.7 | 5000.0 | 39.4 | 129.3 | 0.32 | 0.61 |
| example(25) | P1-6 | 5.1 | 11.7 | 5000.0 | 42.9 | 135.6 | 0.33 | 0.60 |
| example(26) | P1-12 | 5.2 | 12.6 | 5000.0 | 39.6 | 130.5 | 0.31 | 0.64 |
| example(27) | P1-20 | 5.2 | 12.4 | 5000.0 | 40.2 | 129.5 | 0.33 | 0.61 |
| example(28) | P1-44 | 5.1 | 11.4 | 5000.0 | 43.9 | 137.0 | 0.31 | 0.60 |
| example(29) | P1-50 | 5.1 | 12.6 | 5000.0 | 39.5 | 129.7 | 0.30 | 0.62 |
| example(30) | P1-55 | 5.1 | 10.6 | 5000.0 | 47.2 | 137.3 | 0.31 | 0.64 |
| example(31) | P1-73 | 5.0 | 12.1 | 5000.0 | 41.2 | 136.2 | 0.33 | 0.61 |
| example(32) | P1-77 | 5.3 | 11.3 | 5000.0 | 44.3 | 129.7 | 0.35 | 0.61 |
| example(33) | P1-78 | 5.0 | 11.9 | 5000.0 | 42.2 | 132.6 | 0.32 | 0.61 |
| example(34) | P1-84 | 5.3 | 13.8 | 5000.0 | 36.2 | 127.3 | 0.33 | 0.62 |
| example(35) | P1-85 | 5.3 | 14.1 | 5000.0 | 35.5 | 127.6 | 0.32 | 0.63 |

As can be seen from the results in Table 5, when a green organic light emitting diode is manufactured using the material for an organic light emitting diode of the present invention as an emitting auxiliary layer material, compared to the case of using the comparative compound 2, the comparative compound 3, the compound PA-3, and the driving voltage of the organic light emitting diode could be lowered and the efficiency and lifespan were significantly improved.

Specifically, in the case of a comparative compound, the device results using Comparative Compound 3 with arylamine bonded at the 2-position of the xanthene core, compared to Comparative Compound 2 with arylamine bonded at the 3-position of the xanthene core, improved efficiency. Also, it can be seen that the device result of the compound PA-3 in which a heterocyclic compound such as dibenzofuran is bonded as a substituent of an amine group is improved as a whole.

The compound of the present invention is a compound characterized in that an amine group is bonded to the 2-position of the xanthene core, and dibenzofuran or 1-substituted dibenzothiophene is bonded as a substituent of the amine group bonded to the core, it can be seen that the device results of Examples 23 to 35 made of the compounds of the present invention having such characteristics are remarkably excellent.

These results can be explained by the HOMO values of Comparative Compound 2, Comparative Compound 3, Compound PA-3, and the present compound. Referring to Table 6, it can be seen that the HOMO value of Comparative Compound 3 in which an amine group is bonded at the 2-position of the core is higher than Comparative Compound 2 in which an amine group is bonded at the 3-position of the core. Also, it can be seen that the HOMO value of Compound P-14 of the present invention in which the amine group is bonded to the 2-position of the core and 1 substituted dibenzofuran is bonded as a substituent of the bonded amine group is the highest, compared to the compound PA-3 in which an amine group is bonded to the 3-position of the core and 3-substituted dibenzofuran is bonded as a substituent of the bonded amine group.

**[Table 6]**

| | comparative compound 2 | comparative compound 3 | PA-3 | P-14 |
|---|---|---|---|---|
| HOMO (eV) | -4.956 | -4.808 | -4.844 | -4.781 |

That is, it is shown that the compound of the present invention facilitates hole injection compared to the comparative compounds, thereby improving hole mobility, thereby improving the driving voltage, efficiency, and lifespan of the entire element.

In conclusion, even if the core is the same compound, the physical properties of the compound such as hole characteristics, light efficiency characteristics, energy level (LUMO, HOMO level, T1 level), hole injection & mobility characteristics, and electron blocking characteristics vary depending on the bonding position of the substituent, resulting in completely different element results.

### [Reference Example 36] Green organic light emitting diode (emitting auxiliary layer)

2-TNATA was vacuum-deposited to a thickness of 60 nm on the ITO layer (anode) formed on the glass substrate to form a hole injection layer, and then NPB was vacuum-deposited to a thickness of 60 nm on the hole injection layer to form a hole transport layer. Then, after vacuum deposition of the compound P 2-1 to a thickness of 20 nm on the hole transport layer to form an emitting auxiliary layer, and on the emitting auxiliary layer, CBP was used as a host material and tris(2-phenylpyridine)-iridium (hereinafter, Ir(ppy)₃) was used as a dopant material, doped at a weight ratio of 95:5, and vacuum deposited to a thickness of 30 nm to form an emitting layer. Subsequently, BAlq was vacuum deposited to a thickness of 10 nm on the emitting layer to form a hole blocking layer, Bis(10-hydroxybenzo[h]quinolinato)beryllium (hereinafter, BeBq₂) was vacuum deposited to a thickness of 40 nm on the hole blocking layer to form an electron transport layer. Thereafter, an electron injection layer was formed by depositing LiF, an alkali metal halide, to a thickness of 0.2 nm, and subsequently, Al was deposited to a thickness of 150 nm to form a cathode, thereby manufacturing an organic light emitting diode.

### [Reference Example 37] to [Reference Example 47] Green organic light emitting diode (emitting auxiliary layer)

An organic light emitting diode was manufactured in the same manner as in Reference Example 36, except that the compound described in Table 7 was used instead of the compound P2-1 as the emitting auxiliary layer material.

### [Comparative example 4] and [Comparative example 6]

An organic light emitting diode was manufactured in the same manner as in Reference Example 36, except that Comparative Compound 2 and Comparative Compound 4 were used, respectively, instead of the compound P2-1 as the emitting auxiliary layer material.

| |
|---|
| [Comparative example 2] [Comparative example 3] [Comparative example 4] |
| 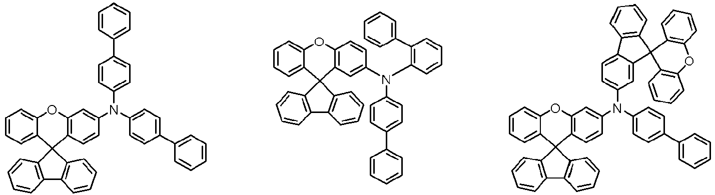 |

Electroluminescence (EL) characteristics were measured by PR-650 of photoresearch company by applying a forward bias direct current voltage to the organic light emitting diode prepared according to Reference Examples 36 to 47 and Comparative Example 4 to 6, T95 life was measured through a life measurement equipment manufactured by McScience at a luminance of 5000 cd/m², and the measurement results are shown in Table 7.

**[Table 7]**

| | compou nd | Voltag e (V) | Current Density (mA/cm²) | Brightness (cd/m²) | Efficiency (cd/A) | Lifetim e T(95) | CIE | |
|---|---|---|---|---|---|---|---|---|
| | | | | | | | x | y |
| comparative example (4) | compar ative compou nd 2 | 6.5 | 33.1 | 5000.0 | 15.1 | 80.4 | 0.32 | 0.64 |
| comparative example (5) | compar ative compou nd 3 | 6.3 | 24.9 | 5000.0 | 20.1 | 88.6 | 0.30 | 0.64 |
| comparative example (6) | compar ative compou nd 4 | 5.9 | 19.3 | 5000.0 | 25.9 | 105.7 | 0.32 | 0.63 |
| example(36) | P2-1 | 5.1 | 10.6 | 5000.0 | 47.2 | 133.2 | 0.33 | 0.60 |
| example(37) | P2-3 | 5.1 | 10.3 | 5000.0 | 48.4 | 135.1 | 0.33 | 0.61 |
| example(38) | P2-7 | 5.3 | 11.2 | 5000.0 | 44.5 | 128.7 | 0.34 | 0.60 |
| example(39) | P2-12 | 5.2 | 10.7 | 5000.0 | 46.7 | 122.6 | 0.30 | 0.62 |
| example(40) | P2-14 | 5.1 | 11.3 | 5000.0 | 44.2 | 127.1 | 0.32 | 0.60 |
| example(41) | P2-22 | 5.1 | 11.2 | 5000.0 | 44.7 | 122.5 | 0.30 | 0.62 |
| example(42) | P2-28 | 5.2 | 10.8 | 5000.0 | 46.4 | 132.5 | 0.34 | 0.65 |
| example(43) | P2-37 | 5.4 | 12.8 | 5000.0 | 39.0 | 120.2 | 0.34 | 0.60 |
| example(44) | P2-39 | 5.3 | 12.7 | 5000.0 | 39.3 | 131.5 | 0.32 | 0.64 |
| example(45) | P2-40 | 5.4 | 12.9 | 5000.0 | 38.8 | 120.8 | 0.34 | 0.61 |
| example(46) | P2-46 | 5.3 | 11.1 | 5000.0 | 45.0 | 124.2 | 0.31 | 0.62 |
| example(47) | P2-50 | 5.2 | 12.2 | 5000.0 | 41.0 | 126.6 | 0.31 | 0.62 |

As can be seen from the results in Table 7, when a green organic light emitting diode is manufactured using the material for an organic light emitting diode as described herein as an emitting auxiliary layer material, compared to the comparative compound 4 to 6, and the driving voltage of the organic light emitting diode could be lowered and the efficiency and lifespan were significantly improved.

First, the device result using Comparative Compound 3 with arylamine bonded at the 2-position of the xanthene core was slightly improved than Comparative Compound 2 with arylamine bonded at the 3-position of the xanthene core, and the device result of Comparative Compound 4 in which one more xanthene was bonded as a substituent of the amine group was improved compared to Comparative Examples 4 and 5.

The compound as described herein is a compound characterized in that an amine group is bonded to 2-position of the xanthene core, and as a substituent of the amine group bonded to the core, one is bonded with 1-position of the dibenzofuran or dibenzothiophene, and the other is bonded with xanthene one more, and it can be seen that the device results of Examples 36 to 47 made of the compounds of the present invention having such characteristics are remarkably excellent.

This is as described in Table 5 above, it is suggested that the compounds of the present invention having a substituent at a specific bonding site may have significantly different chemical and physical properties from those of the Comparative Example compounds, thereby leading to improved device results.

Additionally, referring to Table 8, which lists the HOMO values of Comparative Compound 4 and the compound of the present invention, it can be seen that the HOMO value of compound P 2-3 as described herein in which an amine group is bonded to the 2-position of the core, and one more xanthene is bonded at the same time as 1-position of dibenzofuran as a substituent of the bonded amine group, is higher than that of Comparative Compound 4.

**[Table 8]**

| | Comparative compound 4 | P2-3 |
|---|---|---|
| HOMO (eV) | -4.856 | -4.788 |

That is, it is judged that the compound as described herein can more efficiently transport holes in the hole transport layer, as a result, it is believed that the charge balance of holes and electrons in the emitting layer is increased, thereby improving the driving voltage, efficiency, and lifespan of the entire device.

In the case of the emitting auxiliary layer, it is necessary to grasp the correlation between the hole transport layer and the emitting layer (host), even if a similar core is used, it will be very difficult for a person skilled in the art to infer the characteristics exhibited in the emitting auxiliary layer in which the compound as described herein is used.

In addition, the device characteristics in which the compound as described herein was applied to only one of the emitting auxiliary layers were described in the result of evaluation of manufacture of the element above, but it may be applied to a case where a hole transport layer or both a hole transport layer and an emitting auxiliary layer are formed by using the compound as described herein.

### [Industrial Applicability]

According to the present invention, it is possible to manufacture an organic device having excellent device characteristics of high luminance, high light emission and long lifespan, and thus there is industrial applicability.

## Claims

1. A compound represented by Formula 4-2 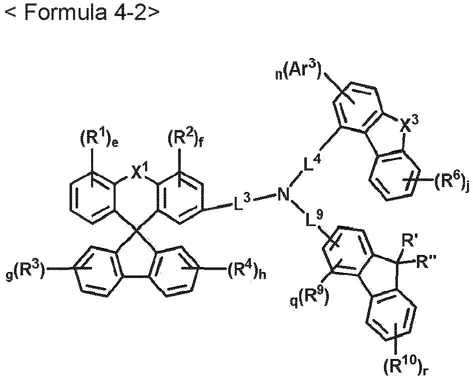 wherein
1) X¹, and X³ are each independently O or S,
2) e, g, h, j, and r, are each independently an integer of 0 to 4, and f and q are an integer of 0 to 3, wherein in case e, f, g, h, j, q and r are 2 or more, R¹, R², R³, R⁴, and R⁶ are each in plural being the same or different, and an adjacent plurality of R¹ or a plurality of R² or a plurality of R³ or a plurality of R⁴ or a plurality of R⁶ or a plurality of R⁷ or a plurality of R⁸ may be bonded to each other to form a ring,
3) n is an integer of 0 to 3,
4) L³, L⁴, and L⁹ are the same or different from each other, and are each independently selected from the group consisting of single bond; a C₆-C₆₀ arylene group; a fluorenylene group; a fused ring group of a C₃-C₆₀ aliphatic ring and a C₆-C₆₀ aromatic ring; a C₂-C₆₀ heterocyclic group;
5) Ar³ is selected from the group consisting of hydrogen; deuterium; tritium; halogen; cyano group; nitro group; C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; C₁-C₅₀ alkyl group; C₂-C₂₀ alkenyl group; C₂-C₂₀ alkynyl group; C₁-C₃₀ alkoxyl group; C₆-C₃₀ aryloxy group; and -L'-N(Rₐ)(R_{b});
6) R¹, R², R³, R⁴, R⁶, R⁹ and R¹⁰ are the same or different from each other, and are each independently selected from the group consisting of hydrogen; deuterium; tritium; halogen; cyano group; nitro group; C₆-C₆₀ aryl group; a fluorenyl group; a C₂-C₆₀ heterocyclic group including at least one heteroatom of O, N, S, Si or P; a fused ring group of a C₃-C₆₀ aliphatic ring and a C₆-C₆₀ aromatic ring; C₁-C₅₀ alkyl group; C₂-C₂₀ alkenyl group; C₂-C₂₀ alkynyl group; C₁-C₃₀ alkoxyl group; C₆-C₃₀ aryloxy group; and -L'-N(Rₐ)(R_{b});
7) wherein L' is selected from the group consisting of a single bond; a C₆-C₆₀ arylene group; a fluorenylene group; C₃-C₆₀ aliphatic ring; a C₂-C₆₀ heterocyclic group including at least one heteroatom of O, N, S, Si or P; a combination thereof; wherein Rₐ and R_{b} are each independently selected from the group consisting of a C₆-C₆₀ aryl group; a fluorenyl group; C₃-C₆₀ aliphatic ring; a C₂-C₆₀ heterocyclic group including at least one heteroatom of O, N, S, Si or P;
8) R' and R" are each independently a hydrogen; C₆-C₆₀ aryl group; a fluorenyl group; a C₃-C₆₀ heterocyclic group; C₁-C₅₀ alkyl group; C₂-C₆₀ alkenyl group; or C₆-C₆₀ aryloxy group; fluorenyl group; a C₂-C₆₀ heterocyclic group including at least one heteroatom of O, N, S, Si or P; C₃-C₆₀ aliphatic ring; or a fused ring group of a C₃-C₆₀ aliphatic ring and a C₆-C₆₀ aromatic ring;
9) wherein, the aryl group, arylene group, heterocyclic group, fluorenyl group, fluorenylene group, fused ring group, alkyl group, alkenyl group, alkoxy group and aryloxy group may be substituted with one or more substituents selected from the group consisting of deuterium; halogen; silane group; siloxane group; boron group; germanium group; cyano group; nitro group; C₁-C₂₀ alkylthio group; C₁-C₂₀ alkoxyl group; C₁-C₂₀ alkyl group; C₂-C₂₀ alkenyl group; C₂-C₂₀ alkynyl group; C₆-C₂₅ aryl group; C₆-C₂₅ aryl group substituted with deuterium; a fluorenyl group; C₂-C₂₀ heterocyclic group; C₃-C₂₀ cycloalkyl group; C₇-C₂₀ arylalkyl group; C₈-C₂₀ arylalkenyl group; and -L'-N(Rₐ)(R_{b}); wherein the substituents may be bonded to each other to form a saturated or unsaturated ring, wherein the term 'ring' means a C₃-C₆₀ aliphatic ring, a C₆-C₆₀ aromatic ring, a C₂-C₆₀ heterocyclic group, a fused ring formed by combination thereof.

2. The compound according to claim 1, wherein Formula 4-2 is represented by any one of Formulas 5-1 to 5-4. 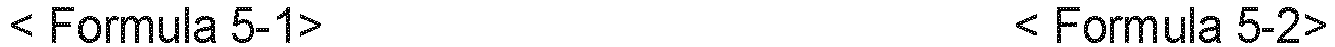 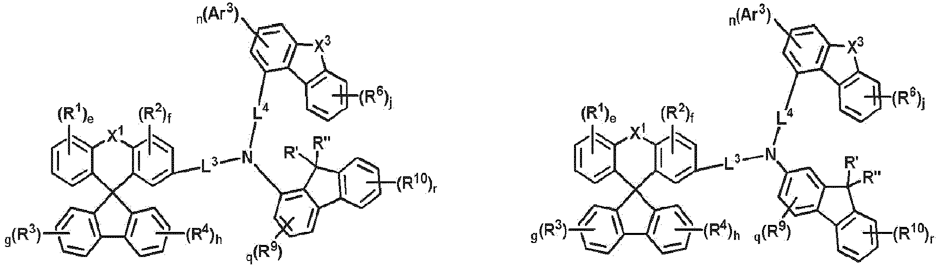 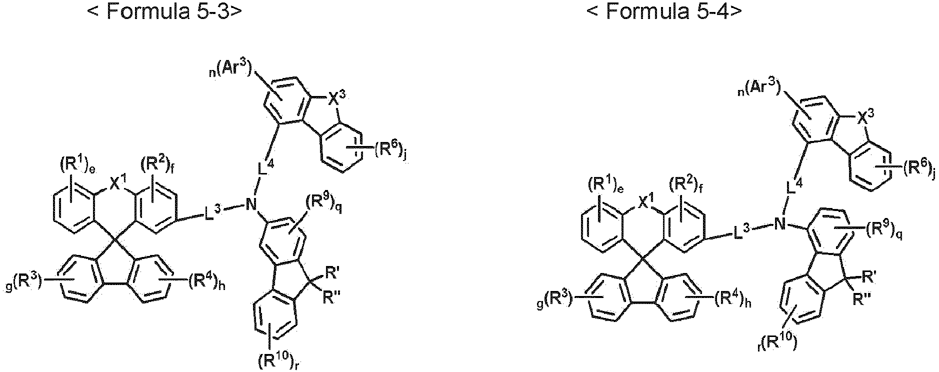 wherein,
1) X¹, X³, R¹, R², R³, R⁴, R⁶, L³, L⁴, Ar³, e, f, g, h, j, n, R⁹, R¹⁰, R', R", q and r are the same as defined in claim 1.

3. The compound according to claim 1, wherein Formula 4-2 is represented by any one of the following compounds:

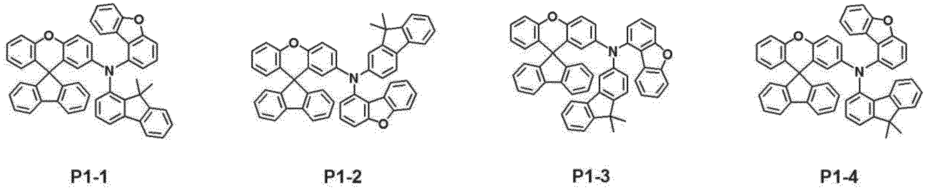

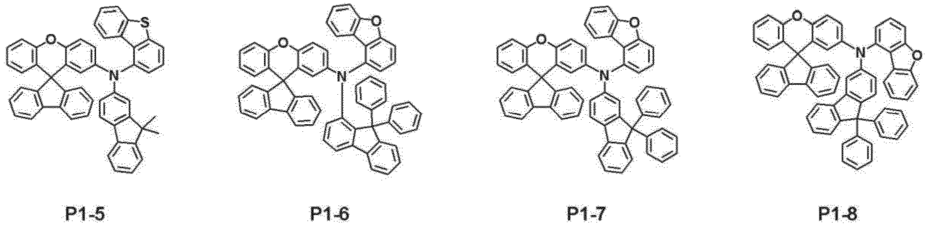

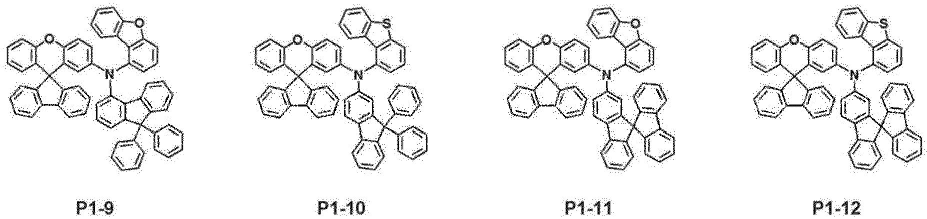

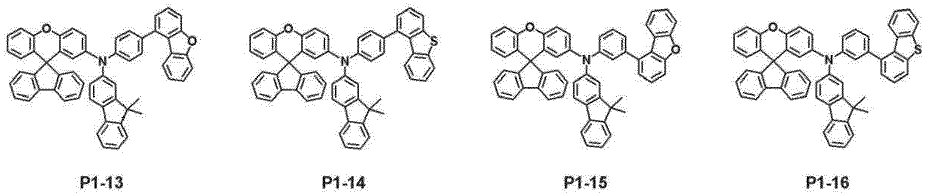

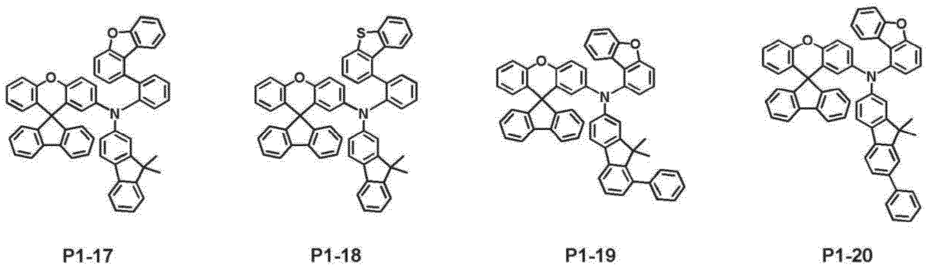

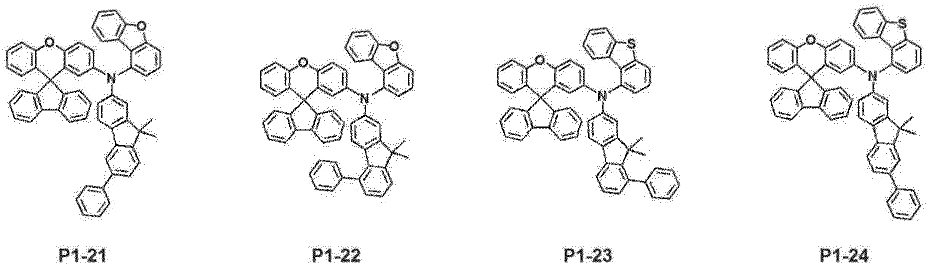

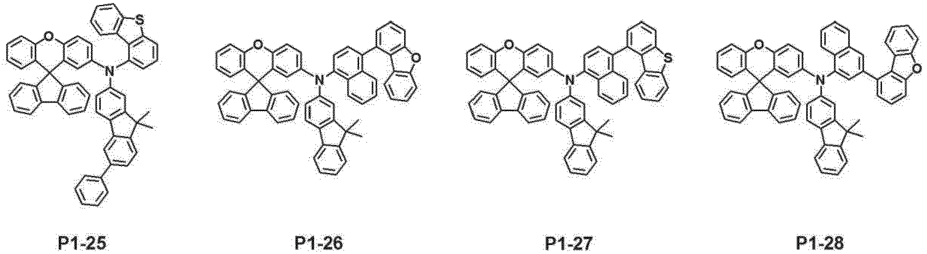

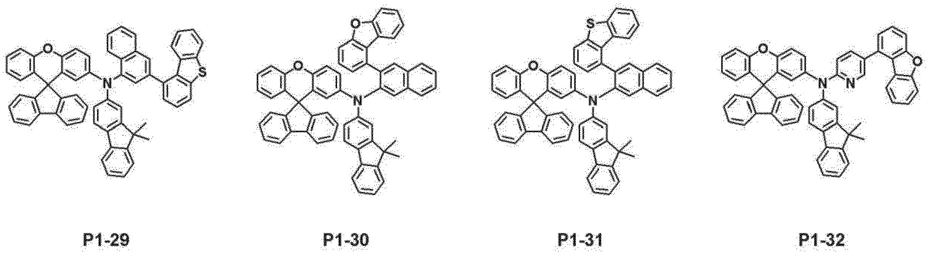

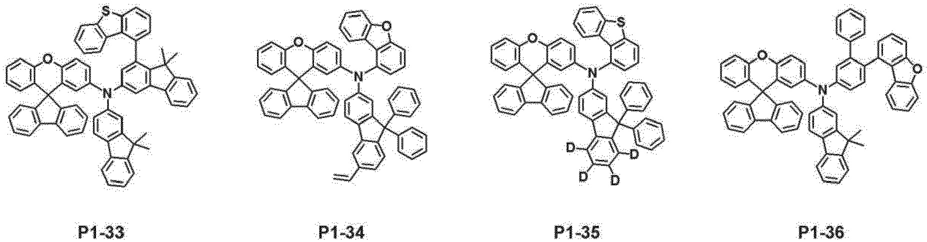

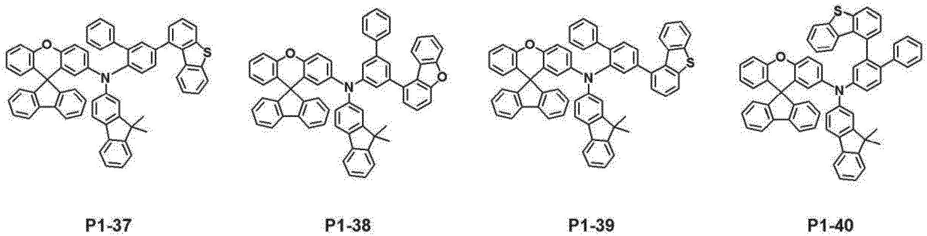

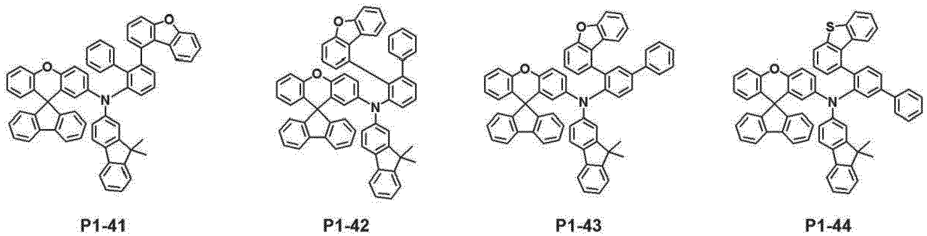

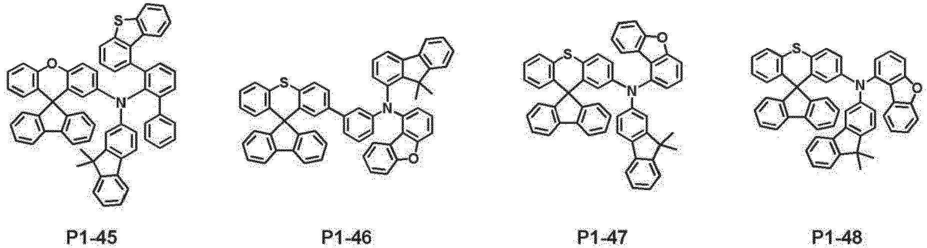

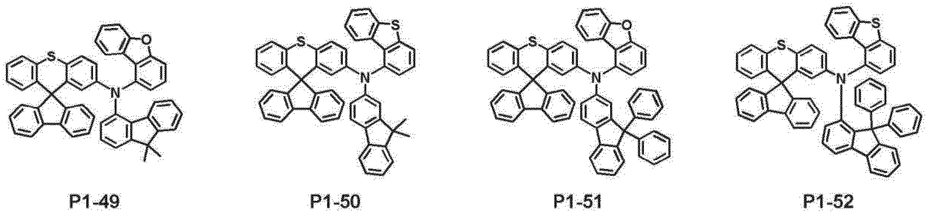

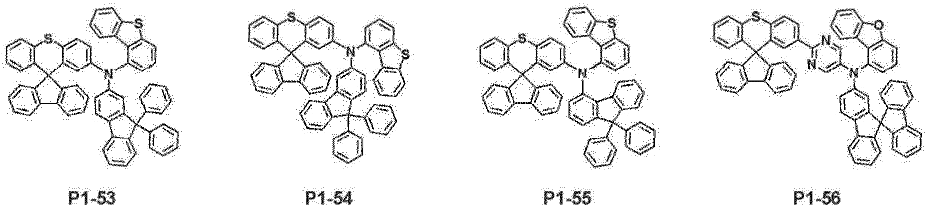

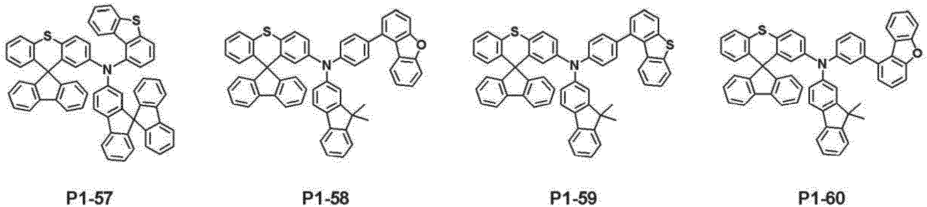

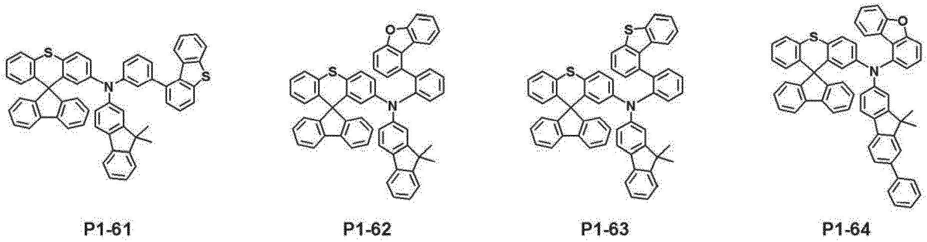

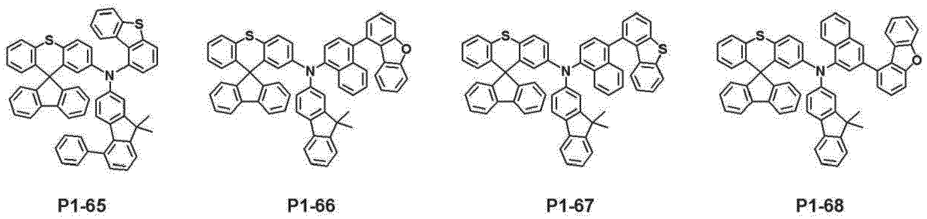

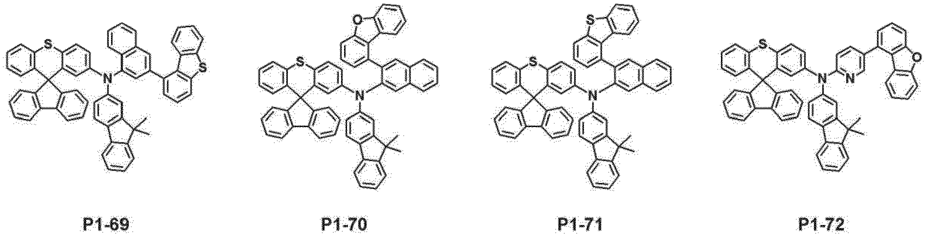

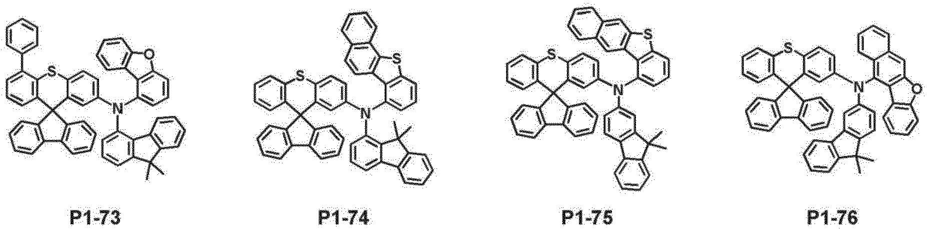

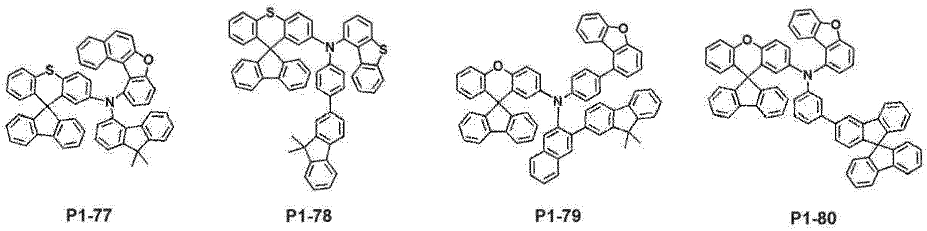

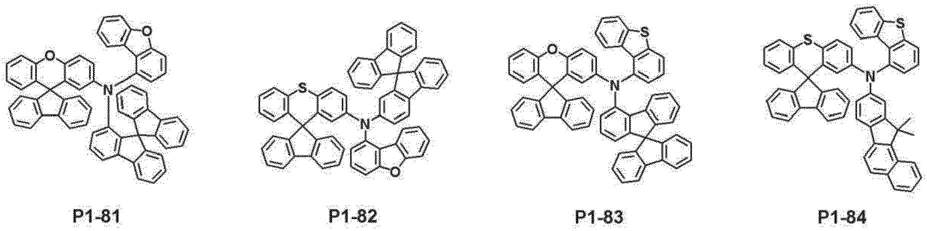

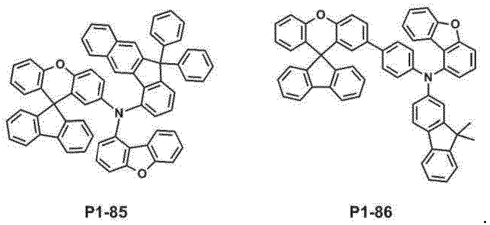

4. An organic electronic element comprising an anode, a cathode, and an organic material layer formed between the anode and the cathode, wherein the organic material layer comprises a single compound or 2 or more compounds represented by Formula 4-2 of claim 1.

5. The organic electronic element of claim 4, wherein the organic material layer comprises at least one of a hole injection layer, a hole transport layer, an emitting auxiliary layer, an emitting layer, an electron transport auxiliary layer, an electron transport layer, and an electron injection layer.

6. The organic electronic element of claim 4, wherein the organic material layer is an emitting auxiliary layer.

7. The organic electronic element of claim 4, further comprising a light efficiency enhancing layer formed on at least one surface of the anode and the cathode opposite to the organic material layer.

8. The organic electronic element of claim 4, wherein the organic material layer comprises 2 or more stacks including a hole transport layer, an emitting layer, and an electron transport layer sequentially formed on the anode.

9. The organic electronic element of claim 8, wherein the organic material layer further comprises a charge generation layer formed between the 2 or more stacks.

10. A display device comprising the organic electronic element of claim 4; and a control unit for driving the display device.

11. The display device according to claim 10, wherein the organic electronic element is at least one of an OLED, an organic solar cell, an organic photo conductor(OPC), organic transistor(organic TFT) and an element for monochromic or white illumination.

## Patentansprüche

1. Verbindung, die durch die Formel 4-2 dargestellt wird, 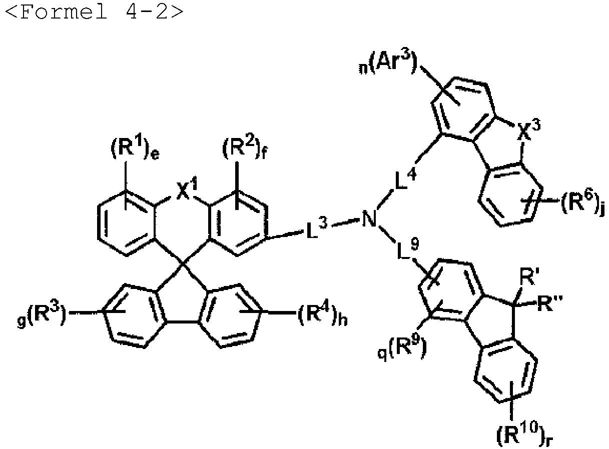 wobei
1) X¹ und X³ jeweils unabhängig O oder S sind,
2) e, g, h, j und r jeweils unabhängig voneinander eine ganze Zahl von 0 bis 4 sind, und f und q eine ganze Zahl von 0 bis 3 sind, wobei in dem Fall, dass e, f, g, h ,j, q und r 2 oder mehr sind, R¹, R², R³, R⁴ und R⁶ jeweils in der Mehrzahl gleich oder unterschiedlich sind, und eine benachbarte Vielzahl von R¹ oder eine Vielzahl von R² oder eine Vielzahl von R³ oder eine Vielzahl von R⁴ oder eine Vielzahl von R⁶ oder eine Vielzahl von R⁷ oder eine Vielzahl von R⁸ aneinander gebunden sein können, um einen Ring zu bilden,
3) n eine ganze Zahl von 0 bis 3 ist,
4) L³, L⁴ und L⁹ gleich oder voneinander unterschiedlich sind, und jeweils unabhängig aus der Gruppe ausgewählt sind, die aus einer Einfachbindung; einer C₆-C₆₀-Arylen-Gruppe; einer Fluorenylen-Gruppe; einer kondensierten Ringgruppe aus einem aliphatischen C₃-C₆₀-Ring und einem aromatischen C₆-C₆₀-Ring; einer heterozyklischen C₂-C₆₀-Gruppe besteht;
5) Ar³ ausgewählt ist aus der Gruppe, die aus Wasserstoff; Deuterium; Tritium; Halogen; einer Cyano-Gruppe; einer Nitro-Gruppe; einer C₆-C₆₀-Aryl-Gruppe; einer Fluorenyl-Gruppe; einer heterozyklischen C₂-C₆₀-Gruppe, die zumindest ein Heteroatom aus O, N, S, Si oder P beinhaltet; einer kondensierten Ringgruppe aus einem aliphatischen C₃-C₆₀-Ring und einem aromatischen C₆-C₆₀-Ring; einer C₁-C₅₀-Alkyl-Gruppe; einer C₂-C₂₀-Alkenyl-Gruppe; einer C₂-C₂₀-Alkinyl-Gruppe; einer C₁-C₃₀-Alkoxyl-Gruppe, einer C₆-C₃₀-Aryloxy-Gruppe; und -L'-N(Rₐ)(R_{b}) besteht;
6) R¹, R², R³, R⁴, R⁶, R⁹ und R¹⁰ gleich oder voneinander unterschiedlich sind, und jeweils unabhängig voneinander aus der Gruppe ausgewählt sind, die aus Wasserstoff; Deuterium; Tritium; Halogen; einer Cyano-Gruppe; einer Nitro-Gruppe; einer C₆-C₆₀-Aryl-Gruppe; einer Fluorenyl-Gruppe; einer heterozyklischen C₂-C₆₀-Gruppe, die zumindest ein Heteroatom aus O, N, S, Si oder P beinhaltet; einer kondensierten Ringgruppe aus einem aliphatischen C₃-C₆₀-Ring und einem aromatischen C₆-C₆₀-Ring; einer C₁-C₅₀-Alkyl-Gruppe; einer C₂-C₂₀-Alkenyl-Gruppe; einer C₂-C₂₀-Alkinyl-Gruppe; einer C₁-C₃₀-Alkoxyl-Gruppe, einer C₆-C₃₀-Aryloxy-Gruppe; und -L'-N(Rₐ)(R_{b}) besteht;
7) wobei L' ausgewählt ist aus der Gruppe, die aus einer Einfachbindung; einer C₆-C₆₀-Arylen-Gruppe; einer Fluorenylen-Gruppe; einem aliphatischen C₃-C₆₀-Ring; einer heterozyklischen C₂-C₆₀-Gruppe, die zumindest ein Heteroatom aus O, N, S, Si oder P beinhaltet; einer Kombination davon besteht; wobei Rₐ und R_{b} jeweils unabhängig voneinander aus der Gruppe ausgewählt sind, die aus einer C₆-C₆₀-Aryl-Gruppe; einer Fluorenyl-Gruppe; einem aliphatischen C₃-C₆₀-Ring; einer heterozyklischen C₂-C₆₀-Gruppe, die zumindest ein Heteroatom aus O, N, S, Si oder P beinhaltet; besteht;
8) R' und R" jeweils unabhängig voneinander ein Wasserstoff; eine C₆-C₆₀-Aryl-Gruppe; eine Fluorenyl-Gruppe; eine heterozyklische C₃-C₆₀-Gruppe; eine C₁-C₅₀-Alkyl-Gruppe; eine C₂-C₆₀-Alkenyl-Gruppe; oder eine C₆-C₆₀-Aryloxy-Gruppe; eine Fluorenyl-Gruppe; eine heterozyklische C₂-C₆₀-Gruppe, die zumindest ein Heteroatom aus O, N, S, Si oder P beinhaltet; ein aliphatischer C₃-C₆₀-Ring; oder eine kondensierte Ringgruppe aus einem aliphatischen C₃-C₆₀-Ring und einem aromatischen C₆-C₆₀-Ring sind;
9) wobei die Aryl-Gruppe, die Arylen-Gruppe, die heterozyklische Gruppe, die Fluorenyl-Gruppe, die Fluorenylen-Gruppe, die kondensierte Ring-Gruppe, die Alkyl-Gruppe, die Alkenyl-Gruppe, die Alkoxy-Gruppe und die Aryloxy-Gruppe mit einem oder mehreren Substituenten substituiert sein können, die aus der Gruppe ausgewählt sind, die aus Deuterium; Halogen; einer Silan-Gruppe; einer Siloxan-Gruppe; einer Bor-Gruppe; einer Germanium-Gruppe; einer Cyano-Gruppe; einer Nitro-Gruppe; einer C₁-C₂₀-Alkylthio-Gruppe; einer C₁-C₂₀-Alkoxyl-Gruppe; einer C₁-C₂₀-Alkyl-Gruppe; einer C₂-C₂₀-Alkenyl-Gruppe; einer C₂-C₂₀-Alkinyl-Gruppe; einer C₆-C₂₅-Aryl-Gruppe; einer mit Deuterium substituierten C₆-C₂₅-Aryl-Gruppe; einer Fluorenyl-Gruppe; einer heterozyklischen C₂-C₂₀-Gruppe; einer C₃-C₂₀-Cycloalkyl-Gruppe; einer C₇-C₂₀-Arylalkyl-Gruppe; einer C₈-C₂₀-Arylalkenyl-Gruppe; und - L'-N(Rₐ)(R_{b}) besteht; wobei die Substituenten aneinander gebunden sein können, um einen gesättigten oder einen ungesättigten Ring zu bilden, wobei der Begriff 'Ring' einen aliphatischen C₃-C₆₀-Ring, einen aromatischen C₆-C₆₀-Ring, eine heterozyklische C₂-C₆₀-Gruppe oder einen durch Kombination davon gebildeten kondensierten Ring bedeutet.

2. Verbindung nach Anspruch 1, wobei die Formel 4-2 durch eine beliebige der Formeln 5-1 bis 5-4 dargestellt wird, 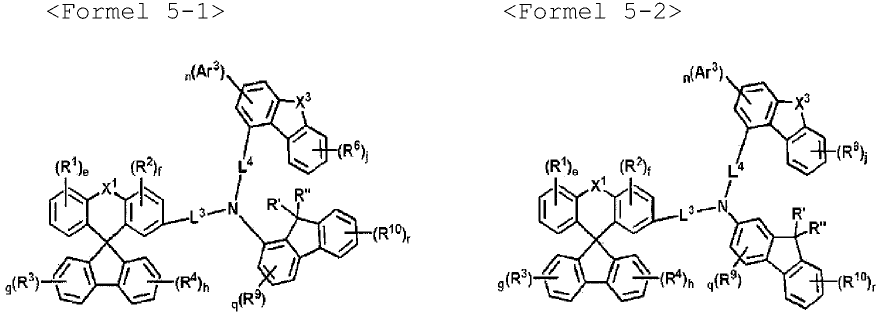 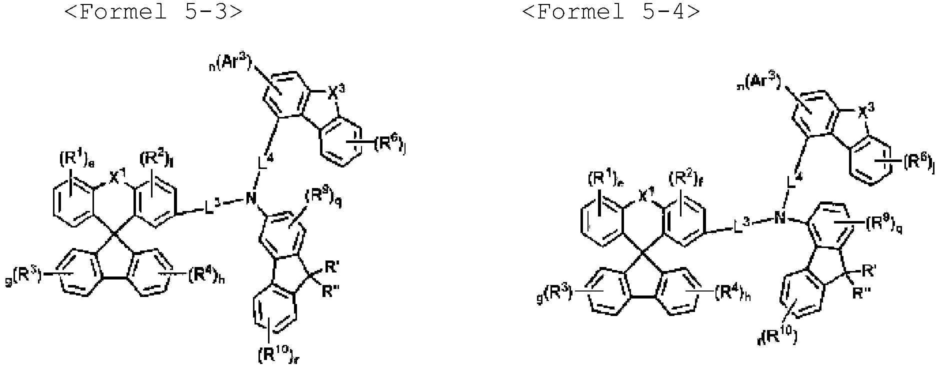 wobei
1) X¹, X³, R¹, R², R³, R⁴, R⁶, L³, L⁴, Ar³, e, f, g, h, j, n, R⁹, R¹⁰, R', R", q und r dieselben sind wie in Anspruch 1 definiert.

3. Verbindung nach Anspruch 1, wobei die Formel 4-2 durch eine beliebige der folgenden Verbindungen dargestellt wird:

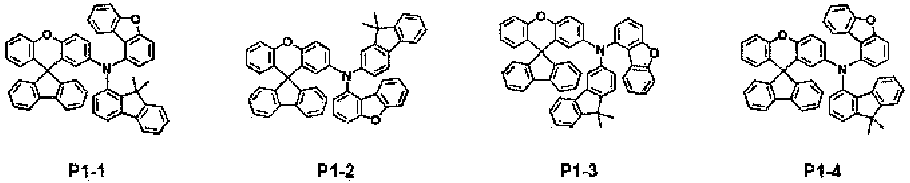

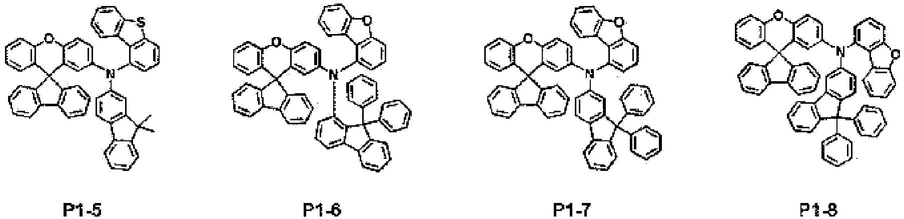

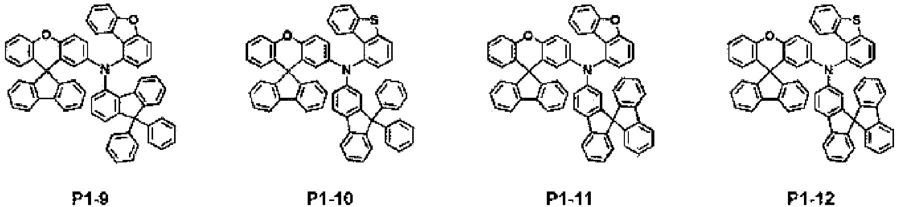

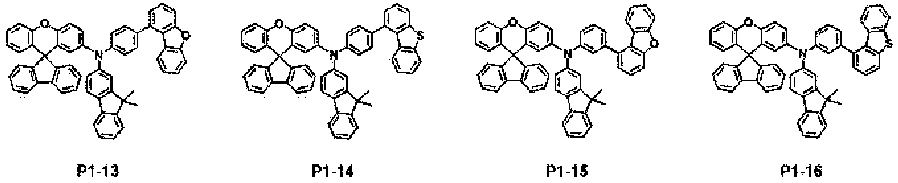

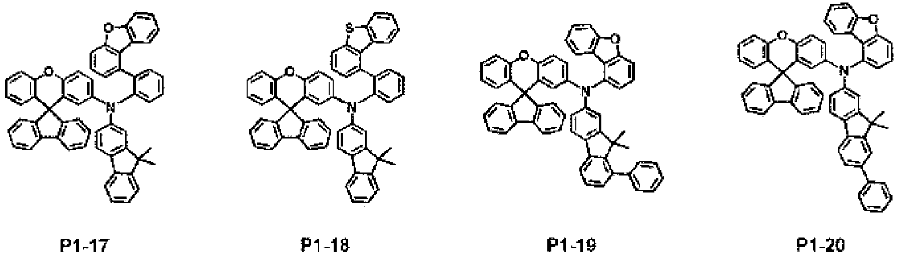

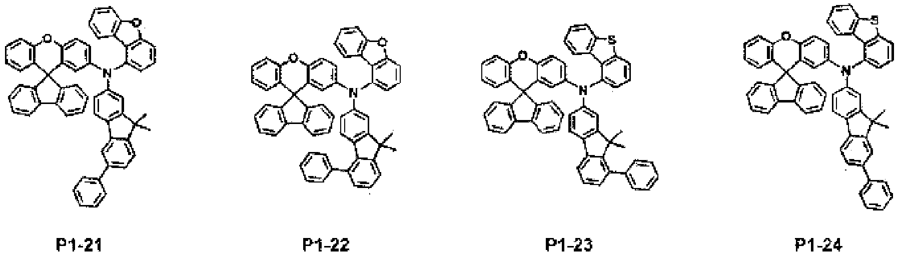

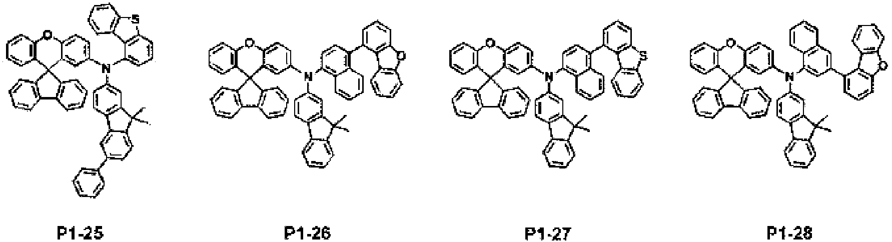

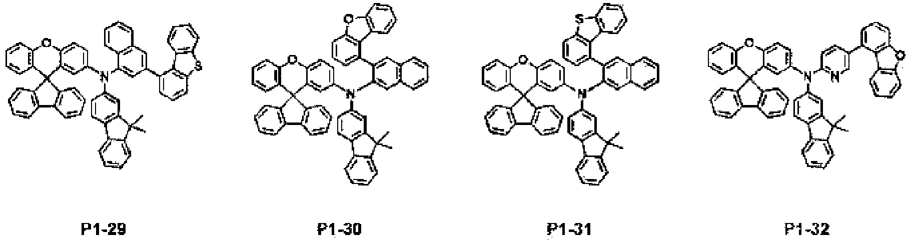

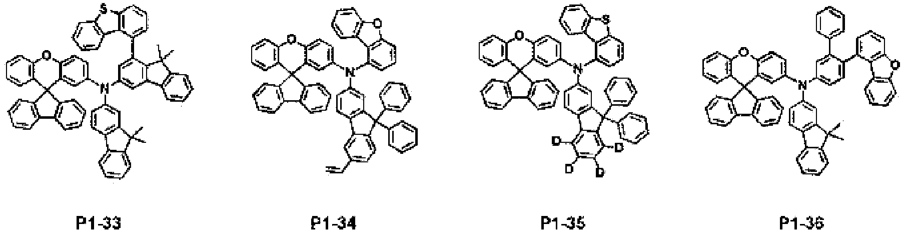

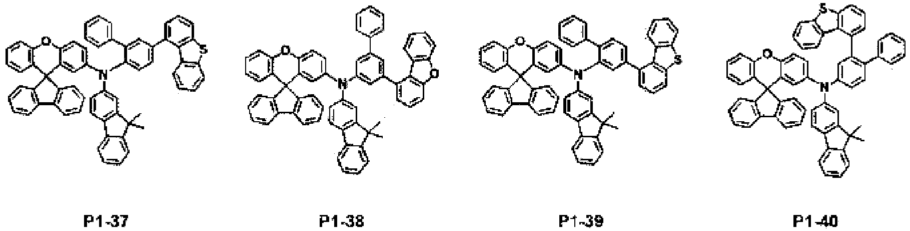

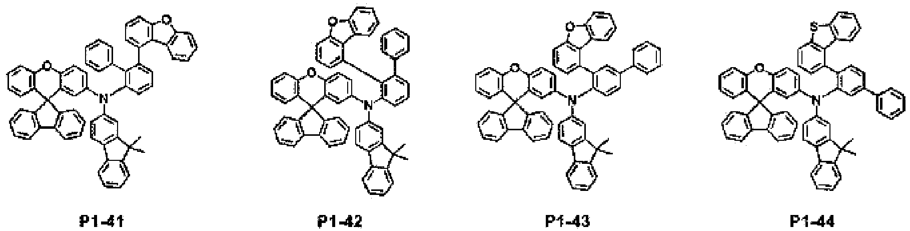

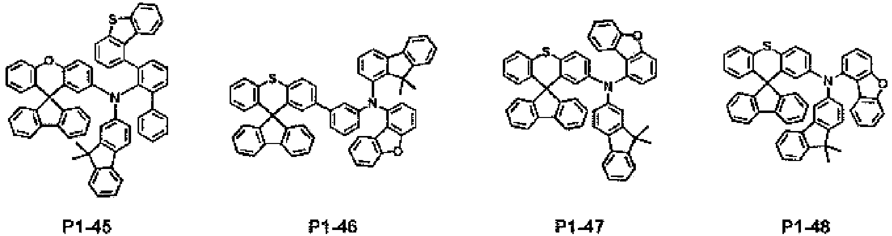

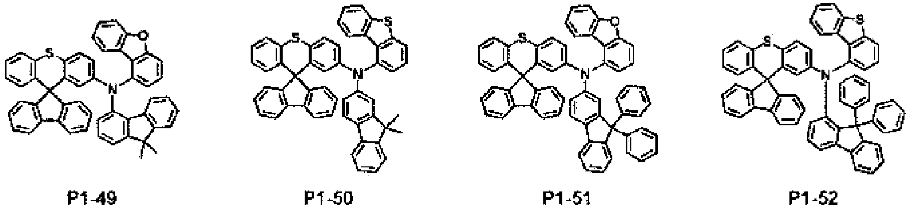

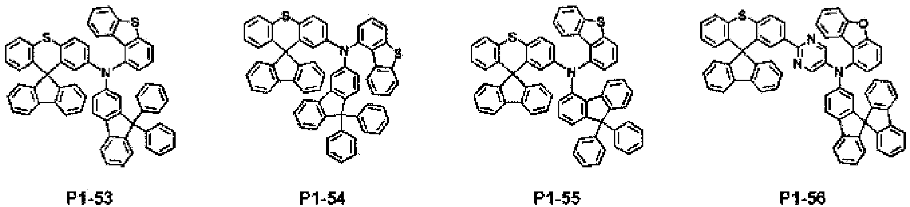

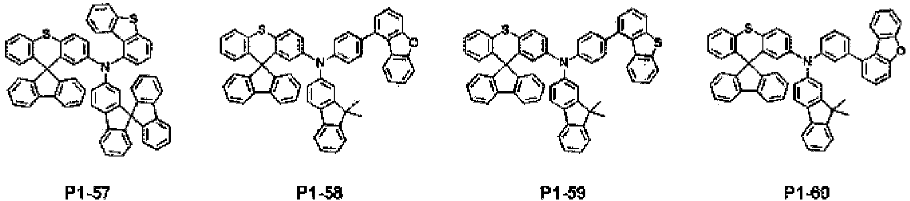

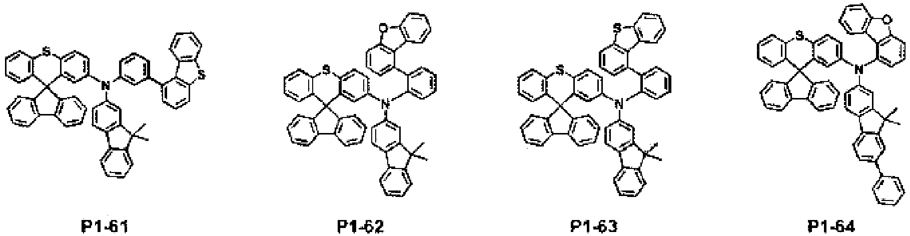

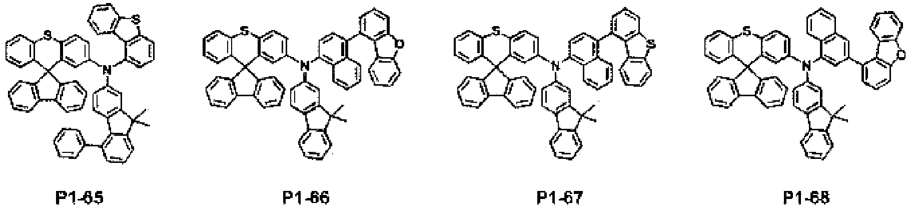

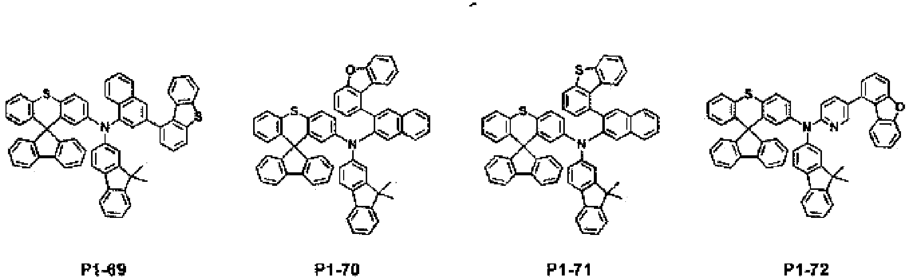

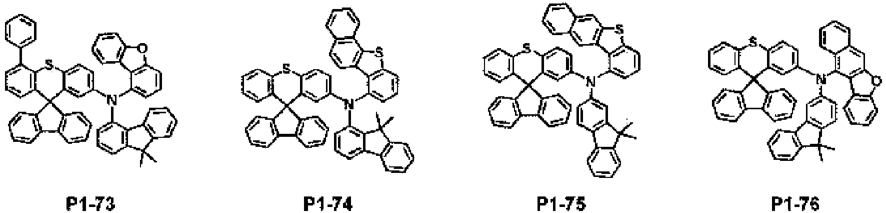

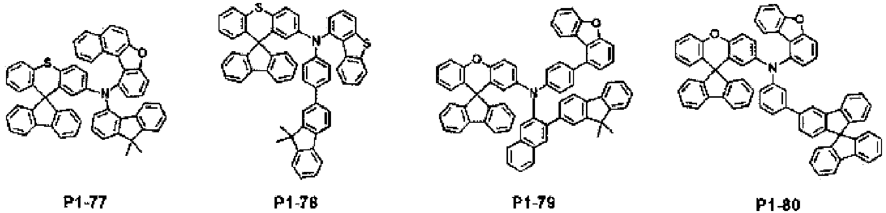

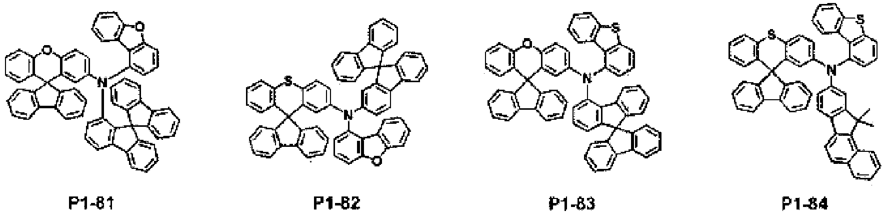

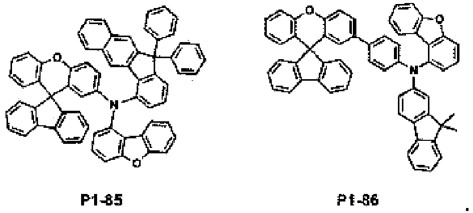

4. Organisches elektronisches Element, umfassend eine Anode, eine Kathode und eine organische Materialschicht, die zwischen der Anode und der Kathode gebildet ist, wobei die organische Materialschicht eine einzelne Verbindung oder 2 oder mehr Verbindungen umfasst, die durch Formel 4-2 nach Anspruch 1 dargestellt werden.

5. Organisches elektronisches Element nach Anspruch 4, wobei die organische Materialschicht zumindest eine von einer Lochinjektionsschicht, einer Lochtransportschicht, einer Emissions-Hilfsschicht, einer Emissionsschicht, einer Elektronentransport-Hilfsschicht, einer Elektronentransportschicht und einer Elektroneninjektionsschicht umfasst.

6. Organisches elektronisches Element nach Anspruch 4, wobei die organische Materialschicht eine Emissions-Hilfsschicht ist.

7. Organisches elektronisches Element nach Anspruch 4, ferner umfassend eine die Lichteffizienz verbessernde Schicht, die auf zumindest einer Oberfläche der Anode und der Kathode gegenüber der organischen Materialschicht ausgebildet ist.

8. Organisches elektronisches Element nach Anspruch 4, wobei die organische Materialschicht 2 oder mehr Stapel beinhaltet, die eine Lochtransportschicht, eine Emissionsschicht, und eine Elektronentransportschicht beinhalten, die der Reihe nach auf der Anode ausgebildet sind.

9. Organisches elektronisches Element nach Anspruch 8, wobei die organische Materialschicht ferner eine Ladungserzeugungsschicht umfasst, die zwischen den 2 oder mehr Stapeln gebildet ist.

10. Anzeigevorrichtung, umfassend das organische elektronische Element nach Anspruch 4; und eine Steuereinheit zum Ansteuern der Anzeigevorrichtung.

11. Anzeigevorrichtung nach Anspruch 10, wobei das organische elektronische Element zumindest eines von einer OLED, einer organischen Solarzelle, einem organischen Fotoleiter (OPC), einem organischen Transistor (organischen TFT), und einem Element für die monochrome oder weiße Beleuchtung ist.

## Revendications

1. Composé représenté par la formule 4-2 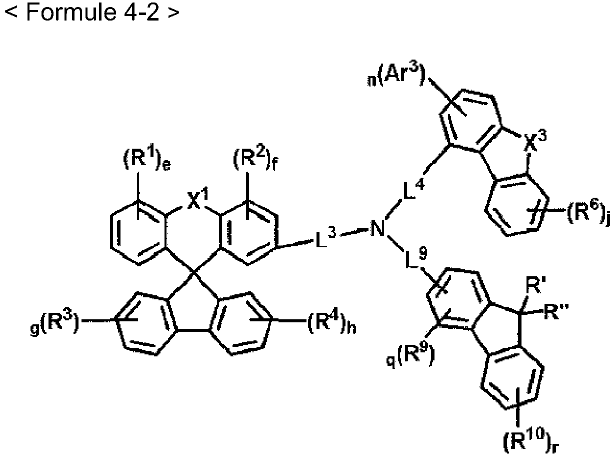 dans laquelle
1) X¹ et X³ sont chacun indépendamment O ou S,
2) e, g, h, j et r sont chacun indépendamment un nombre entier de 0 à 4, et f et q sont un nombre entier de 0 à 3, dans laquelle dans le cas où e, f, g, h, j, q et r valent 2 ou plus, R¹, R², R³, R⁴ et R⁶ sont chacun au pluriel en étant identiques ou différents, et une pluralité adjacente de R¹ ou une pluralité de R² ou une pluralité de R³ ou une pluralité de R⁴ ou une pluralité de R⁶ ou une pluralité de R⁷ ou une pluralité de R⁸ peuvent être liés les uns aux autres pour former un noyau,
3) n est un nombre entier de 0 à 3,
4) L³, L⁴ et L⁹ sont identiques ou différents les uns des autres, et sont chacun indépendamment choisis dans le groupe constitué par une liaison simple ; un groupe C₆-C₆₀ arylène ; un groupe fluorénylène ; un groupe à noyaux fusionnés d'un noyau C₃-C₆₀ aliphatique et d'un noyau C₆-C₆₀ aromatique ; un groupe C₂-C₆₀ hétérocyclique ;
5) Ar³ est choisi dans le groupe constitué par hydrogène ; deutérium ; tritium ; halogène ; un groupe cyano ; un groupe nitro ; un groupe C₆-C₆₀ aryle ; un groupe fluorényle ; un groupe C₂-C₆₀ hétérocyclique comprenant au moins un hétéroatome de O, N, S, Si ou P ; un groupe à noyaux fusionnés d'un noyau C₃-C₆₀ aliphatique et d'un noyau C₆-C₆₀ aromatique ; un groupe C₁-C₅₀ alkyle ; un groupe C₂-C₂₀ alcényle ; un groupe C₂-C₂₀ alcynyle ; un groupe C₁-C₃₀ alcoxyle ; un groupe C₆-C₃₀ aryloxy ; et L'-N(Rₐ)(R_{b}) ;
6) R¹, R², R³, R⁴, R⁶, R⁹ et R¹⁰ sont identiques ou différents les uns des autres, et sont chacun indépendamment choisis dans le groupe constitué par hydrogène ; deutérium ; tritium ; halogène ; un groupe cyano ; un groupe nitro ; un groupe C₆-C₆₀ aryle ; un groupe fluorényle ; un groupe C₂-C₆₀ hétérocyclique comprenant au moins un hétéroatome de O, N, S, Si ou P ; un groupe à noyaux fusionnés d'un noyau C₃-C₆₀ aliphatique et d'un noyau C₆-C₆₀ aromatique ; un groupe C₁-C₅₀ alkyle ; un groupe C₂-C₂₀ alcényle ; un groupe C₂-C₂₀ alcynyle ; un groupe C₁-C₃₀ alcoxy ; un groupe C₆-C₃₀ aryloxy ; et L'-N(Rₐ)(R_{b}) ;
7) dans laquelle L' est choisi dans le groupe constitué par une liaison simple ; un groupe C₆-C₆₀ arylène ; un groupe fluorénylène ; un noyau C₃-C₆₀ aliphatique ; un groupe C₂-C₆₀ hétérocyclique comprenant au moins un hétéroatome de O, N, S, Si ou P ; une combinaison de ceux-ci ; dans laquelle Rₐ et R_{b} sont chacun indépendamment choisis dans le groupe constitué par un groupe C₆-C₆₀ aryle ; un groupe fluorényle ; un noyau C₃-C₆₀ aliphatique ; un groupe C₂-C₆₀ hétérocyclique comprenant au moins un hétéroatome de O, N, S, Si ou P ;
8) R' et R" sont chacun un hydrogène ; un groupe C₆-C₆₀ aryle ; un groupe fluorényle ; un groupe C₃-C₆₀ hétérocyclique ; un groupe C₁-C₅₀ alkyle ; un groupe C₂-C₆₀ alcényle ; ou un groupe C₆-C₆₀ aryloxy ; un groupe fluorényle ; un groupe C₂-C₆₀ hétérocyclique comprenant au moins un hétéroatome de O, N, S, Si ou P ; un noyau C₃-C₆₀ aliphatique ; ou un groupe à noyaux fusionnés d'un noyau C₃-C₆₀ aliphatique et d'un noyau C₆-C₆₀ aromatique ;
9) dans laquelle, les groupe aryle, groupe arylène, groupe hétérocyclique, groupe fluorényle, groupe fluorénylène, groupe à noyaux fusionnés, groupe alkyle, groupe alcényle, groupe alcoxy et groupe aryloxy peuvent être substitués par un ou plusieurs substituants choisis dans le groupe constitué par deutérium ; halogène ; groupe silane ; groupe siloxane ; groupe bore ; groupe germanium ; groupe cyano ; groupe nitro ; groupe C₁-C₂₀ alkylthio ; groupe C₁-C₂₀ alcoxyle ; groupe C₁-C₂₀ alkyle ; groupe C₂-C₂₀ alcényle ; groupe C₂-C₂₀ alcynyle ; groupe C₆-C₂₅ aryle ; groupe C₆-C₂₅ aryle substitué avec deutérium ; un groupe fluorényle ; groupe C₂-C₂₀ hétérocyclique ; groupe C₃-C₂₀ cycloalkyle ; groupe C₇-C₂₀ arylalkyle ; groupe C₈-C₂₀ arylalcényle ; et L'-N(Rₐ)(R_{b}) ; dans laquelle les substituants peuvent être liés les uns aux autres pour former un noyau saturé ou non saturé, dans laquelle le terme « noyau » désigne un noyau C₃-C₆₀ aliphatique, un noyau C₆-C₆₀ aromatique, un groupe C₂-C₆₀ hétérocyclique, un noyau fusionné formé par une combinaison de ceux-ci.

2. Composé selon la revendication 1, dans lequel la formule 4-2 est représenté par l'une quelconque des formules 5-1 à 5-4. 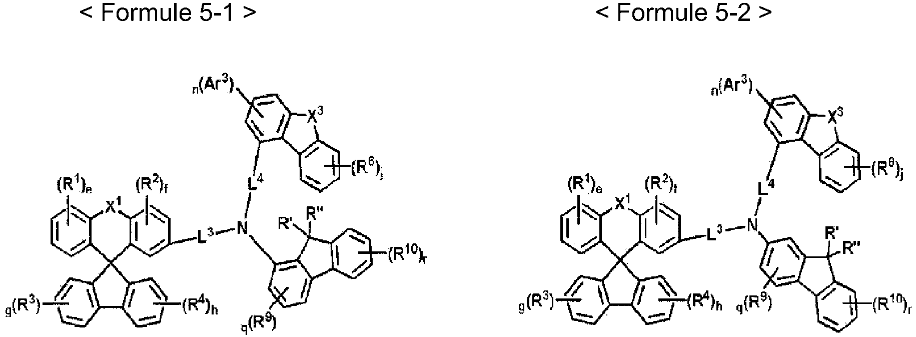 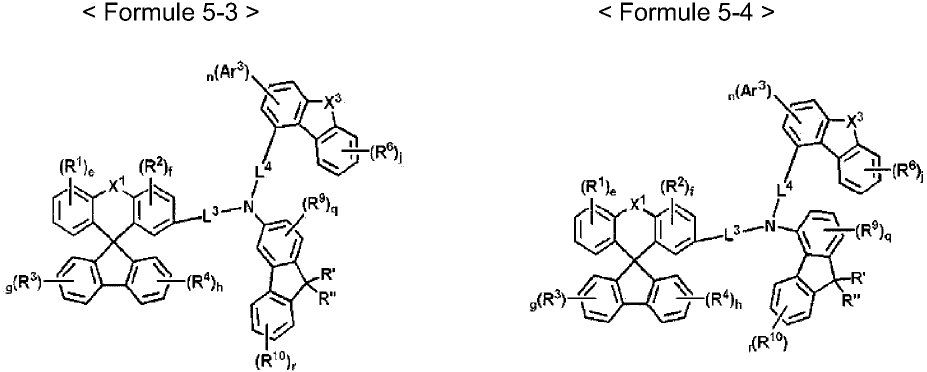 dans lesquelles,
1) X¹, X³, R¹, R², R³, R⁴, R⁶, L³ , L⁴, Ar³, e, f, g, h, j, n, R⁹, R¹⁰, R', R", q et r sont les mêmes que définis dans la revendication 1.

3. Composé selon la revendication 1, dans lequel la formule 4-2 est représentée par l'un quelconque des composés suivants :

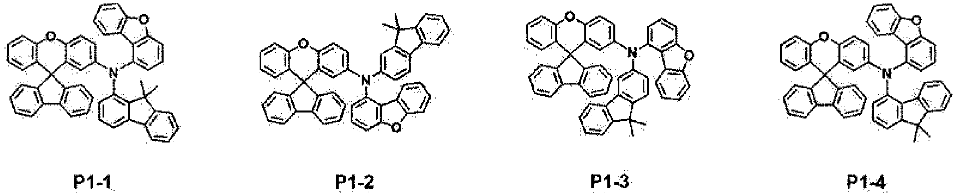

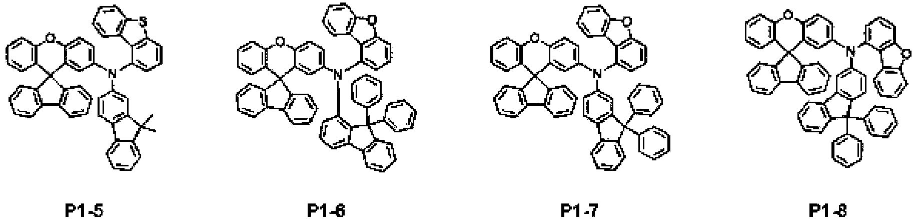

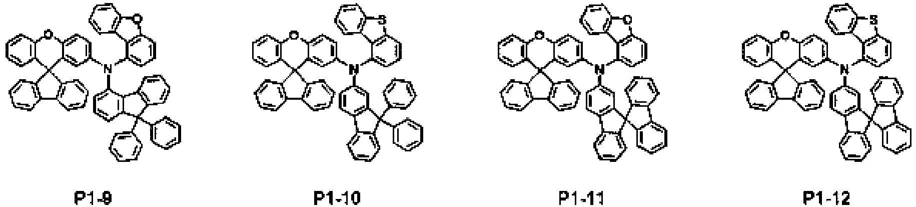

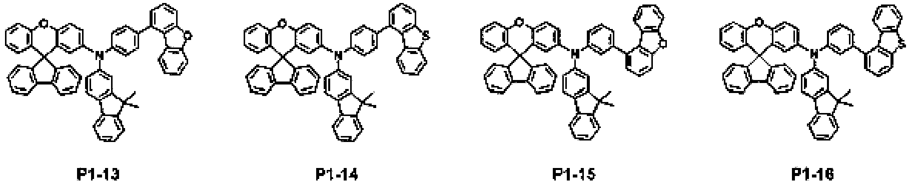

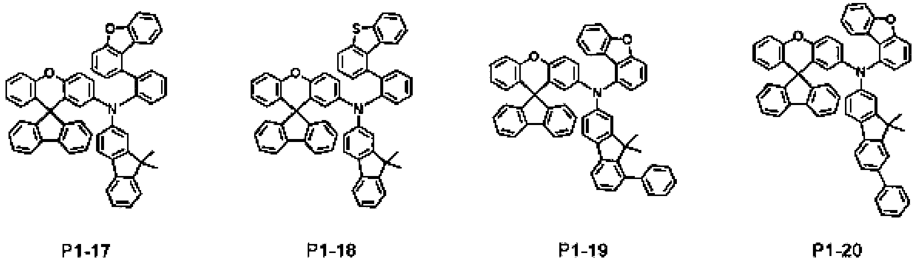

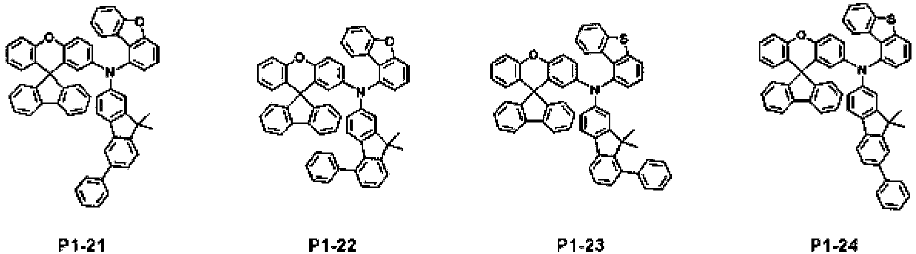

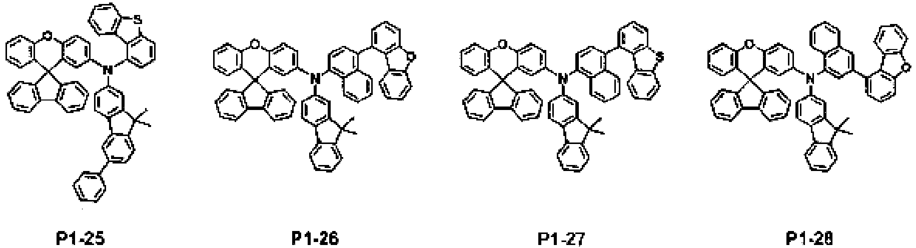

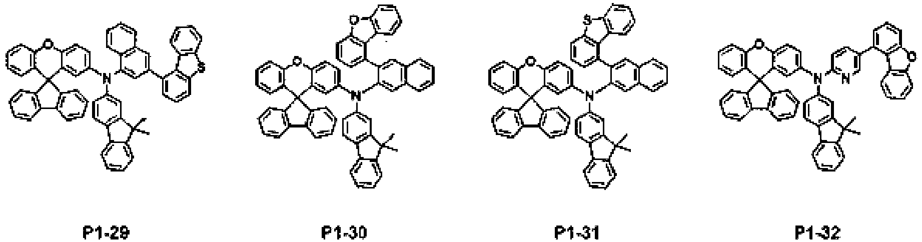

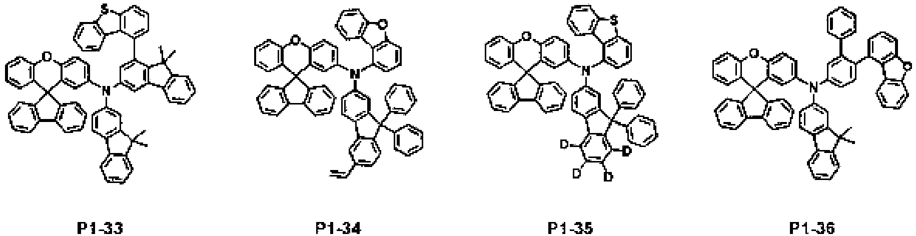

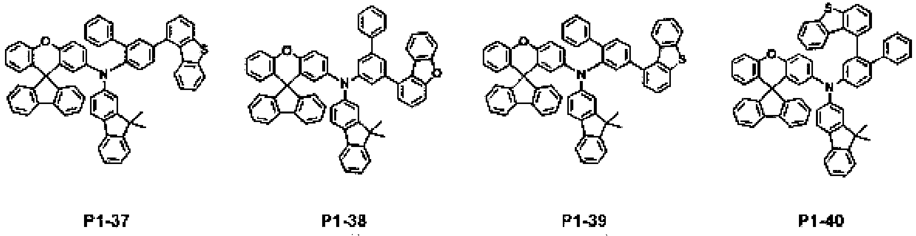

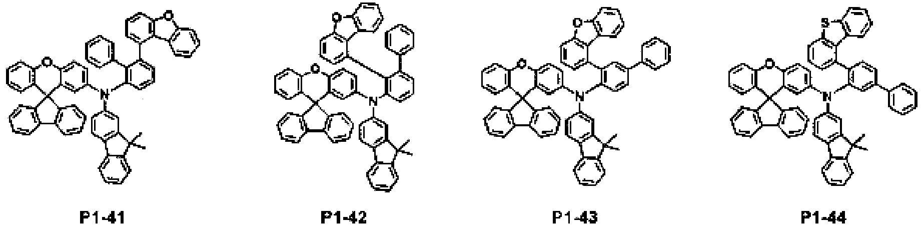

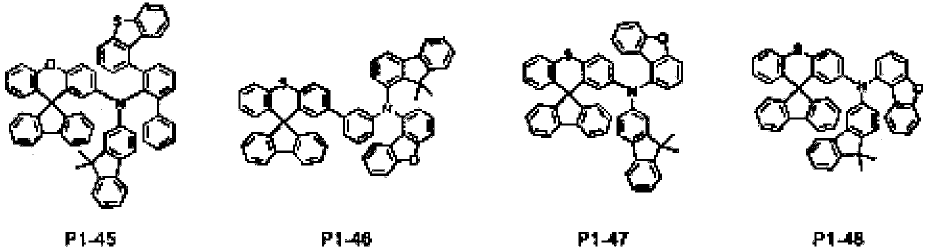

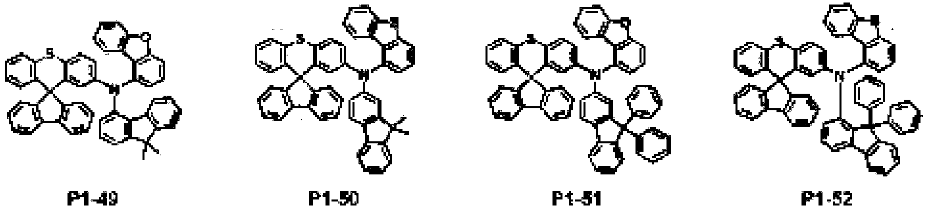

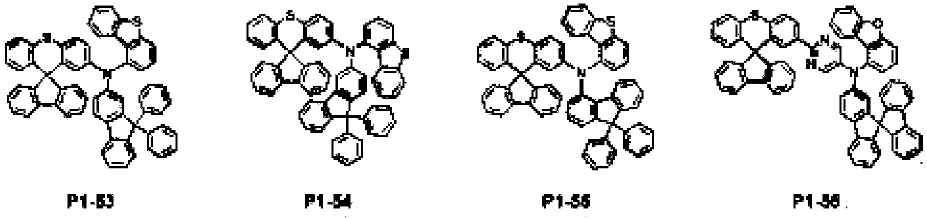

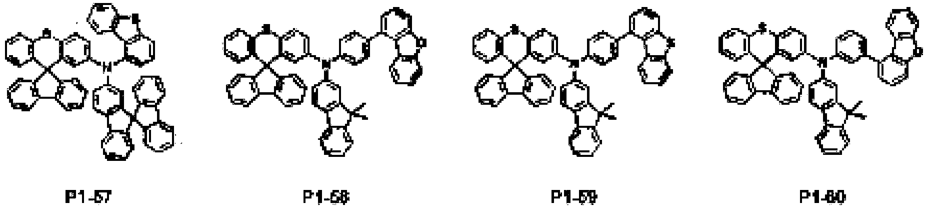

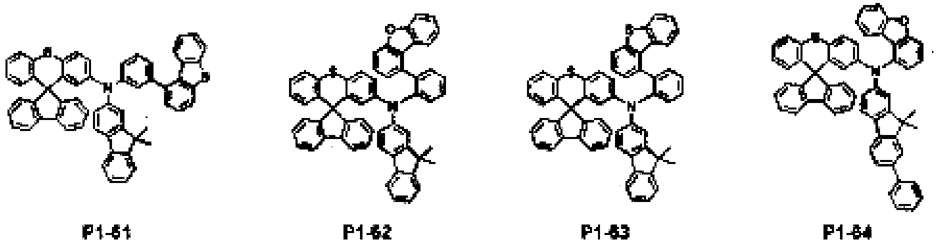

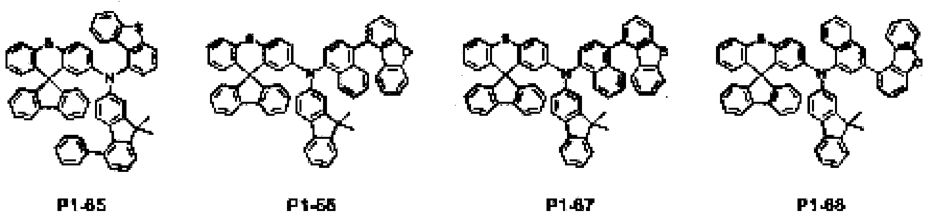

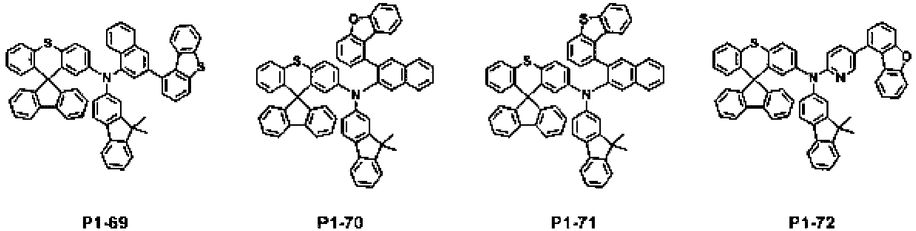

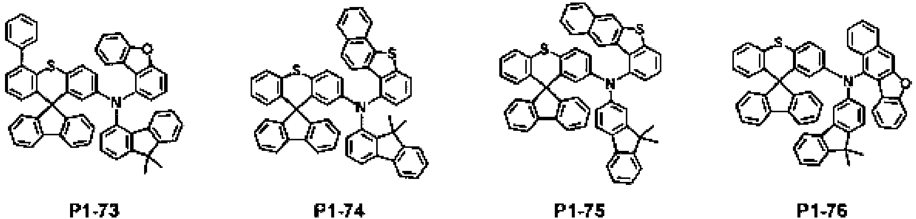

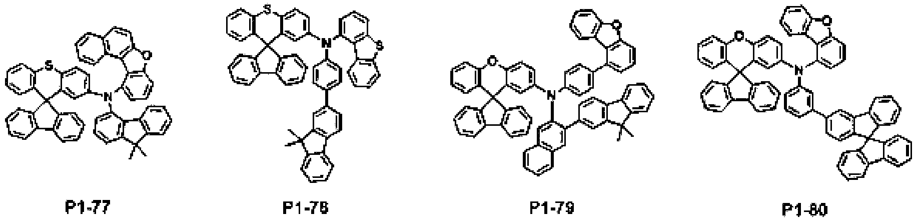

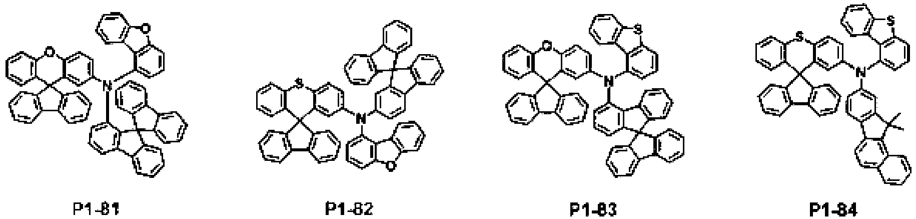

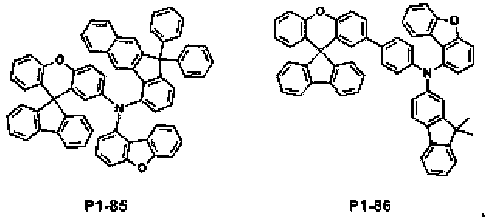

4. Elément électronique organique comprenant une anode, une cathode et une couche de matériau organique formée entre l'anode et la cathode, dans lequel la couche de matériau organique comprend un seul composé ou 2 composés ou plus représentés par la formule 4-2 de la revendication 1.

5. Elément électronique organique de la revendication 4, dans lequel la couche de matériau organique comprend au moins l'une d'une couche d'injection de trous, d'une couche de transport de trous, d'une couche auxiliaire émettrice, d'une couche émettrice, d'une couche auxiliaire de transport d'électrons, d'une couche de transport d'électrons et d'une couche d'injection d'électrons.

6. Elément électronique organique de la revendication 4, dans lequel la couche de matériau organique est une couche auxiliaire émettrice.

7. Elément électronique organique de la revendication 4, comprenant en outre une couche d'amélioration d'efficacité lumineuse formée sur au moins une surface de l'anode et de la cathode opposée à la couche de matériau organique.

8. Elément électronique organique de la revendication 4, dans lequel la couche de matériau organique comprend 2 empilements ou plus comprenant une couche de transport de trous, une couche émettrice et une couche de transport d'électrons formées de manière séquentielle sur l'anode.

9. Elément électronique organique de la revendication 8, dans lequel la couche de matériau organique comprend en outre une couche de génération de charges formée entre 2 empilements ou plus.

10. Dispositif d'affichage comprenant l'élément électronique organique de la revendication 4 ; et une unité de commande pour commander le dispositif d'affichage.

11. Dispositif d'affichage selon la revendication 10, dans lequel l'élément électronique organique est au moins un élément parmi une DELO, une cellule solaire organique, un photoconducteur organique (OPC), un transistor organique (TFT organique) et un élément pour un éclairage monochromatique ou blanc.
